# EUROPÄISCHE PATENTSCHRIFT

(11) **EP 2 038 115 B1**
(45) Veröffentlichungstag und Bekanntmachung des Hinweises auf die Patenterteilung: **04.04.2012**
(21) Anmeldenummer: 07765633.8
(22) Anmeldetag: 26.06.2007
(51) Int. Cl.: B01J 8/06, C07C 51/215, C22C 38/40, C22C 30/00, B32B 15/01, B01J 19/02, C07C 5/333

(54) **REAKTOR AUS AUFPLATTIERTEM ROSTFREIEM STAHL FÜR DIE KONTINUIERLICHE HETEROGENE KATALYSIERTE PARTIELLE DEHYDRIERUNG WENIGSTENS EINES ZU DEHYDRIERENDEN KOHLENWASSERSTOFFS UND VERFAHREN**
REACTOR FROM PLATED STAINLESS STEEL FOR THE CONTINUOUS HETEROGENEOUSLY CATALYZED PARTIAL DEHYDRATION OF AT LEAST ONE HYDROCARBON TO BE DEHYDRATED, AND CORRESPONDING METHOD
RÉACTEUR EN ACIER INOXYDABLE PLAQUÉ DESTINÉ À LA DÉSHYDRATATION CATALYTIQUE PARTIELLE HÉTÉROGÈNE CONTINUE D'AU MOINS UN HYDROCARBURE À DÉSHYDRATER, ET PROCÉDÉ

(30) Priorität: 27.06.2006 DE 102006029790; 27.06.2006 US 816592 P
(43) Veröffentlichungstag der Anmeldung: 25.03.2009
(73) Patentinhaber: BASF SE, 67056 Ludwigshafen (DE)
(72) Erfinder: HECHLER, Claus, 67063 Ludwigshafen (DE); RUPPEL, Wilhelm, 68163 Mannheim (DE); SCHINDLER, Götz-Peter, 67071 Ludwigshafen (DE); HORSTMANN, Catharina, 67056 Ludwigshafen (DE); BASSLER, Hans-Jürgen, 67433 Neustadt (DE); DIETERLE, Martin, 67063 Ludwigshafen (DE); KLAPPERT, Karl-Heinrich, 67134 Birkenheide (DE); MÜLLER-ENGEL, Klaus Joachim, 76297 Stutensee (DE)
(86) Internationale Anmeldenummer: PCT/EP2007/056369
(87) Internationale Veröffentlichungsnummer: WO 2008/000740

(56) Entgegenhaltungen:
- EP-A- 0 683 760
- DE-A1- 3 522 646
- US-A1- 2003 044 334
- US-B1- 6 241 953

## Beschreibung

Vorliegende Erfindung betrifft ein Verfahren der kontinuierlichen heterogen katalysierten partiellen Dehydrierung wenigstens eines zu dehydrierenden Kohlenwasserstoffs in der Gasphase, umfassend eine Verfahrensweise, bei der man einem Reaktionsraum, der von einer den Reaktionsraum berührenden (materiellen) Einhüllenden (E) umschlossen ist, die wenigstens eine erste Öffnung zur Zufuhr wenigstens eines Ausgangsgasstroms in den Reaktionsraum und wenigstens eine zweite Öffnung zur Entnahme wenigstens eines Produktgasstroms aus dem Reaktionsraum aufweist,
- wenigstens einen, wenigstens einen zu dehydrierenden Kohlenwasserstoff enthaltenden, Ausgangsgasstrom kontinuierlich zuführt,
- im Reaktionsraum den wenigstens einen zu dehydrierenden Kohlenwasserstoff durch wenigstens ein im Reaktionsraum befindliches Katalysatorbett führt, und unter Erzeugung eines den wenigstens einen dehydrierten Kohlenwasserstoff, nicht umgesetzten zu dehydrierenden Kohlenwasserstoff sowie molekularen Wasserstoff und/oder Wasserdampf enthaltenden Produktgases oxidativ oder nicht oxidativ zu wenigstens einem dehydrierten Kohlenwasserstoff partiell dehydriert, und
- dem Reaktionsraum wenigstens einen Produktgasstrom kontinuierlich entnimmt.

Außerdem betrifft vorliegende Erfindung eine Vorrichtung zur Durchführung des erfindungsgemäßen Verfahrens sowie Verfahren der Partialoxidation des wenigstens einen dehydrierten Kohlenwasserstoffs.

Der in dieser Anmeldung verwendete Begriff "dehydrierter Kohlenwasserstoff' soll Kohlenwasserstoffe umfassen, deren Moleküle wenigstens zwei ("zwei" sind anwendungstechnisch bevorzugt) Wasserstoffatome weniger enthalten, als die Moleküle eines zu dehydrierenden Kohlenwasserstoffs. Im übrigen soll der Begriff Kohlenwasserstoff Stoffe umfassen, deren Moleküle nur aus den Elementen Kohlenstoff und Wasserstoff aufgebaut ist.

Damit umfassen dehydrierte Kohlenwasserstoffe insbesondere acyclische und cyclische aliphatische Kohlenwasserstoffe mit einer oder mehreren C,C-Doppelbindungen im Molekül.

Beispiele für solche aliphatischen dehydrierten Kohlenwasserstoffe sind Propen, iso-Buten, Ethylen, 1-Buten, 2-Buten und Butadien. D. h., zu den dehydrierten Kohlenwasserstoffen gehören insbesondere die einfach ungesättigten linearen (n-Alkene) oder verzweigten aliphatischen Kohlenwasserstoffe (z. B. iso-Alkene), sowie die Cycloalkene.

Ferner sollen die dehydrierten Kohlenwasserstoffe auch die Alkapolyene (z. B. Diene und Triene) umfassen, die mehr als eine Kohlenstoff-Kohlenstoff-Doppelbindung im Molekül enthalten. Dehydrierte Kohlenwasserstoffe sollen aber auch Kohlenwasserstoffverbindungen umfassen, die ausgehend von Alkylaromaten wie Ethylbenzol oder Isopropylbenzol durch Dehydrierung des Alkylsubstituenten erhältlich sind. Das sind z. B. Verbindungen wie Styrol oder α-Methylstyrol.

Ganz generell bilden dehydrierte Kohlenwasserstoffe wertvolle Ausgangsverbindungen zur Synthese von z. B. funktionalisierten, radikalisch polymerisierbaren Verbindungen (z. B. Acrylsäure aus Propen oder Methacrylsäure aus iso-Buten und deren Polymerisationsprodukte). Beispielsweise können solche funktionalisierten Verbindungen durch Partialoxidation von dehydrierten Kohlenwasserstoffen erzeugt werden. Dehydrierte Kohlenwasserstoffe eignen sich aber auch zur Herstellung von Verbindungen wie Methyl-tert.-butyl-ether (Folgeprodukt von iso-Buten, das z. B. als Kraftstoffadditiv zur Einstellung der Oktanzahl geeignet ist). Dehydrierte Kohlenwasserstoffe können aber auch als solche selbst zur Polymerisation verwendet werden.

Als zu dehydrierende Kohlenwasserstoffe kommen in dieser Schrift insbesondere die acyclischen und cyclischen Alkane, aber auch Olefine (deren C,C-Doppelbindungszahl erhöht werden soll) in Betracht (als Beispiel sei die heterogen katalysierte partielle Dehydrierung von n-Butenen zu Butadien erwähnt).

D. h., der Begriff "zu dehydrierende Kohlenwasserstoffe" umfasst in dieser Patentanmeldung z. B. Kohlenwasserstoffe der Stöchiometrie CₙH₂ₙ₊₂ mit n > 1 bis n ≤ 20, sowie der Stöchiometrie CₙH₂ₙ mit n > 1 bis n ≤ 20, sowie der Stöchiometrie CₙH₂ₙ₋₂ mit n > 2 bis n ≤ 20, und n = ganzzahlig, insbesondere C₂- bis C₁₆-Alkane wie z. B. Ethan (zu Ethylen), Propan (zu Propylen), n-Butan, iso-Butan (zu iso-Buten), n-Pentan, isoPentan, n-Hexan, n-Heptan, n-Octan, n-Nonan, n-Decan, n-Undecan, n-Dodecan, n-Tridecan, n-Tetradecan, n-Pentadecan und n-Hexadecan.

Insbesondere aber gelten alle in dieser Schrift getroffenen Aussagen für C₂- bis C₆-Alkane als zu dehydrierende Kohlenwasserstoffe und ganz besonders für C₂- bis C₄-Kohlenwasserstoffe. D. h., zu dehydrierende Kohlenwasserstoffe sind in dieser Schrift vor allem Ethan, Propan, n-Butan und iso-Butan, aber auch 1-Buten und 2-Buten.

Das eingangs beschriebene Verfahren zur Herstellung von dehydrierten Kohlenwasserstoffen ist allgemein bekannt (vgl. z. B. WO 03/076370, DE-A 10 2004 032 129, EP-A 731 077, WO 01/96271, WO 01/96270, DE-A 103 16 039, WO 03/011804, WO 00/10961, EP-A 799 169, DE-A 102 45 585 sowie die deutschen Anmeldungen Nr. 10 2005 061 626, 10 2006 017 623 und Deutsches Aktenzeichen 102006024901.1).

Prinzipiell werden die Verfahren zur Herstellung von dehydrierten Kohlenwasserstoffen durch heterogen katalysierte partielle Dehydrierung wenigstens eines zu dehydrierenden Kohlenwasserstoffs in zwei Gruppen unterschieden: oxidative und nicht oxidative heterogen katalysierte partielle Dehydrierungen. Im Unterschied zur oxidativen heterogen katalysierten partiellen Dehydrierung erfolgt die nicht oxidative heterogen katalysierte partielle Dehydrierung ohne Mitwirkung von Sauerstoff. D.h., der dem zu dehydrierenden Kohlenwasserstoff zu entreißende Wasserstoff wird unmittelbar als molekularer Wasserstoff entrissen und auch nicht in einem Folgeschritt mit Sauerstoff wenigstens teilweise oxidativ zu Wasser oxidiert. Die Wärmetönung einer nicht oxidativen Dehydrierung ist somit in jedem Fall endotherm. Bei der oxidativen heterogen katalysierten partiellen Dehydrierung wird der dem zu dehydrierenden Kohlenwasserstoff zu entreißende molekulare Wasserstoff hingegen unter Mitwirkung von Sauerstoff entrissen. Dieses Entreißen kann unmittelbar als Wasser (H₂O) erfolgen (in diesem Fall spricht man verkürzt auch von einer heterogen katalysierten Oxidehydrierung; ihre Wärmetönung ist in jedem Fall exotherm). Das Entreißen kann aber auch zunächst als molekularer Wasserstoff erfolgen (d. h., nicht oxidativ bzw. konventionell), der dann in einem Folgeschritt teilweise oder vollständig mit Sauerstoff zu Wasser (H₂O) oxidiert werden kann (je nach Umfang der Wasserstofffolgeverbrennung kann die Bruttowärmetönung endotherm, exotherm oder neutral sein).

Allen vorgenannten heterogen katalysierten partiellen Dehydrierungen von zu dehydrierenden Kohlenwasserstoffen ist gemein, dass sie bei vergleichsweise hohen Reaktionstemperaturen erfolgen. Typische Reaktionstemperaturen können ≥ 250°C, häufig ≥ 300°C, oft ≥ 350°C, oder ≥ 400°C, bzw. ≥ 450°C, oder ≥ 500°C betragen.

Im Verlauf des Langzeitbetriebs einer kontinuierlich betriebenen heterogen katalysierten partiellen Dehydrierung sind darüber hinaus normalerweise mit der Betriebszeit zunehmend noch höhere Reaktionstemperaturen erforderlich, um den Dehydrierumsatz bei einmaligem Durchgang durch den Reaktionsraum aufrechtzuerhalten. Dies rührt üblicherweise daher, dass die verwendeten Katalysatoren mit zunehmender Betriebszeit in zunehmendem Umfang irreversibel deaktivieren. D. h., selbst dann, wenn man den kontinuierlichen Betrieb immer wieder temporär unterbricht (eine solche Betriebsweise soll vom Begriff "kontinuierlich" in dieser Schrift umfasst sein), um die verwendeten Katalysatoren mittels geeigneter Maßnahmen zu reaktivieren (zu regenerieren), wird mit zunehmender Gesamtbetriebszeit des Verfahrens die Ursprungsaktivität der Katalysatoren mit der Gesamtbetriebszeit zunehmend nicht mehr erreicht. Durch entsprechende Erhöhung der Reaktionstemperatur kann diesem Sachverhalt gegengesteuert werden. Eine Erhöhung der Reaktionstemperatur wird normalerweise aber auch angewendet, um der reversiblen Deaktivierung des Katalysators entgegenzuwirken.

Nachteilig an solchermaßen hohen Reaktionstemperaturen ist, dass relativ zur gewünschten Zielreaktion (zu dehydrierender Kohlenwasserstoff → dehydrierter Kohlenwasserstoff) in mit der Reaktionstemperatur in der Regel zunehmendem Umfang unerwünschte Nebenreaktionen zunehmend an Gewicht gewinnen. Eine dieser unerwünschten Nebenreaktoren ist z. B. die thermische Zersetzung des zu dehydrierenden und/oder des dehydrierten Kohlenwasserstoffs zu üblicherweise eine geringere Anzahl an Kohlenstoffatomen aufweisenden Kohlenwasserstoffen.

Herkömmlicher Werkstoff für die Einhüllende von Reaktionsräumen für die großtechnische Produktion ist Stahl. Experimentelle Untersuchungen haben jedoch ergeben, dass Stahl als Werkstoff für die den Reaktionsraum berührende Seite der Einhüllenden eines wie eingangs beschriebenen Verfahrens die thermische Zersetzung von zu dehydrierenden und/oder von dehydrierten Kohlenwasserstoffen zu katalysieren vermag. Die katalytische Wirkung mindert die für die thermische Zersetzung erforderliche Aktivierungsenergie, wodurch sich diese unerwünschte Nebenreaktion bereits bei vergleichsweise niedrigen Reaktionstemperaturen in unerwünschter Weise bemerkbar macht. In entsprechender Weise vermag Stahl im Beisein von molekularem Sauerstoff auch die Verbrennung von zu dehydrierendem und/oder von dehydriertem Kohlenwasserstoff zu katalysieren. Wesentlich im vorgenannten Zusammenhang ist, dass das Ausmaß der katalytischen Wirkung von der Elementzusammensetzung des jeweiligen Stahls abhängig ist.

Vor vorgenanntem Hintergrund werden im Stand der Technik unterschiedliche Stähle für das eingangs beschriebene Verfahren empfohlen. Die WO 03/076370 empfiehlt beispielsweise in ihren Ausführungsbeispielen als Werkstoff für die Einhüllende Stahl, der auf der den Reaktionsraum berührenden Seite der Einhüllenden alonisiert, alitiert und/oder aluminiert (d. h., mit Aluminium, bzw. mit Aluminiumoxid, bzw. mit Aluminium und Aluminiumoxid beschichtet) ist. Weitere die Zusammensetzung des Stahls betreffende Angaben macht die WO 03/076370 nicht. Die gleiche Empfehlung macht die WO 03/011804. Als mögliche Alternative empfiehlt sie zusätzlich die Mitverwendung von Sulfiden im Ausgangsgasstrom, zum Zweck der Passivierung der den Reaktionsraum berührenden Seite der Einhüllenden.

Nachteilig an einer Alonisierung bzw. Alitierung und/oder Aluminierung ist jedoch, dass sie im großtechnischen Maßstab nur mit besonderem Aufwand durchgeführt werden kann. Nachteilig an einer Mitverwendung von Sulfiden im Ausgangsgasstrom ist zum einen deren Bedarf und zum anderen, dass sich eine solche Mitverwendung in der Regel negativ auf die Standzeit der für die heterogen katalysierte partielle Dehydrierung verwendeten Katalysatoren und/oder negativ auf die Standzeit der für eine sich gegebenenfalls anschließende heterogen katalysierte partielle Oxidation des dehydrierten Kohlenwasserstoff verwendeten Katalysatoren auswirkt.

Die vorgenannten Nachteile nicht aufweisend ist die Lehre der DE-A 102004032129, die als Werkstoff für die Einhüllende in ihrem Vergleichsbeispiel unbeschichteten Stahl der DIN Werkstoffnummer 1.4841 empfiehlt. Dabei handelt es sich um einen Stahl, der nachfolgende Elementzusammensetzung aufweisen darf:

| | |
|---|---|
| 24 bis 26 Gew.-% | Cr, |
| 19 bis 22 Gew.-% | Ni, |
| 1,5 bis 2,5 Gew.-% | Si, |
| ≥ 0 bis 0,11 Gew.-% | N, |
| ≥ 0 bis 0,2 Gew.-% | C, |
| ≥ 0 bis 2 Gew.-% | Mn, |
| ≥ 0 bis 0,045 Gew.-% | P, |
| ≥ 0 bis 0,015 Gew.-% | S, und |
| im übrigen Fe und herstellungsbedingte Verunreinigungen, wobei die Prozentangaben jeweils auf das Gesamtgewicht bezogen sind. | |

Die DE-A 10 2005 051 401 empfiehlt in ähnlicher Weise Si-haltigen Stahl, z. B. solchen vom DIN-Typ 1.4841, als Werkstoff für die Einhüllende. Unter anderen Empfehlungen umfasst auch das Deutsche Aktenzeichen 102006017623.5 für die den Reaktionsraum berührende Seite die Empfehlung des Stahls vom DIN-Typ 1.4841.

Als Alternative zu Stahl der DIN-Werkstoffnummer 1.4841 empfiehlt das Deutsche Aktenzeichen 102005061626.7 für die den relevanten Reaktionsraum Einhüllende die Verwendung eines Si-haltigen Stahls, der die nachfolgende Elementzusammensetzung aufweist:

| | |
|---|---|
| 18 bis 30 Gew.-% | Cr, |
| 9 bis 37 Gew.-% | Ni, |
| 1 bis 3 Gew.-% | Si, |
| 0,1 bis 0,3 Gew.-% | N, |
| ≥ 0 bis 0,15 Gew.-% | C, |
| ≥ 0 bis 4 Gew.-% | Mn, |
| ≥ 0 bis 4 Gew.-% | Al, |
| ≥ 0 bis 0,05 Gew.-% | P, |
| ≥ 0 bis 0,05 Gew.-% | S, und |
| ≥ 0 bis 0,1 Gew.-% | eines oder mehrere seltene Erdmetalle, und |
| im übrigen Fe und herstellungsbedingte Verunreinigungen, wobei die Prozentangaben jeweils auf das Gesamtgewicht bezogen sind. | |

Dabei wird im Deutschen Aktenzeichen 102005061626.7 davon ausgegangen, dass die vorgenannten Stahlzusammensetzungen so ausgewählt sind, dass sie sich auch hinsichtlich des übrigen aus dem relevanten Produktionsprozess resultierenden Anforderungsprofils als vorteilhaft erweisen. Zu diesem Anforderungsprofil gehört beispielsweise, dass der zu verwendende Stahl auch als Druckbehälterwerkstoff geeignet sein sollte, da das eingangs beschriebene Verfahren bzw. zugehörige Regenerierzyklen vorzugsweise bei Arbeitsdrucken oberhalb von 1 atm (beispielsweise wenigstens 0,5 bar oberhalb Atmosphärendruck) durchgeführt wird. Beispielsweise fordert die Deutsche Druckbehälterverordnung diesbezüglich von einem als geeignet qualifizierten Werkstoff eine (bezogen auf Raumtemperatur (ca. 25 °C) und Normaldruck (1 atm)) Kerbschlagzähigkeit (Kerbschlagarbeit) von ≥ 40 J (bestimmt im Kerbschlagbiegeversuch gemäß DIN 50115 in Verbindung mit DIN EN 10045 Teil 1 u. 2 (im Fall von Kleinstproben); beim Versuch fällt ein Pendelhammer von einer vorgegebenen Höhe und trifft in seinem tiefsten Punkt die Rückseite einer gekerbten Probe; beim Durchschlagen der Probe wird ein Teil der Hammerenergie für die sogenannte Kerbschlagarbeit verbraucht; diese kann entweder am Schleppzeiger des Gerätes gemessen oder bei instrumentierten Pendeln direkt elektronisch weiterverarbeitet werden). Unmittelbare Konsequenz des Vorgenannten ist das Erfordernis einer geringen Langzeitversprödungsneigung des Stahls unter den für das eingangs beschriebene Verfahren anzuwendenden Prozessbedingungen, da mit der Versprödung eines Werkstoffs eine abnehmende Kerbschlagzähigkeit desselben einhergeht.

Zu diesen Prozessbedingungen zählen insbesondere die bei den vorgenannten Verfahren normalerweise anzuwendenden erhöhten Reaktions- und/oder Katalysatorregeneriertemperaturen.

Des weiteren sollte die Aufkohlungsneigung des erwählten Stahls unter den Bedingungen des relevanten Verfahrens möglichst gering sein. Beim Aufkohlen wird der Werkstoff von der den Reaktionsraum berührenden Seite her durch elementaren Kohlenstoff angegriffen, der durch Kohlenwasserstoffzersetzung entsteht und in den Stahl eindiffundiert. Im Unterschied zur vorbeschriebenen Langzeitversprödung, die von Aufgang an das gesamte Stahlgefüge erfasst, arbeitet sich das Aufkohlen von der Oberfläche der den Reaktionsraum berührenden Seite her ausgehend erst sukzessive ins Werkstoffinnere vor.

Mit zunehmender Aufkohlung verschlechtern sich die Werkstoffeigenschaften (beispielsweise ist die Sprödigkeit in Bereichen mit Aufkohlung erhöht; infolge Carbidbildung relevanter Elemente intensivieren sich die unerwünschten katalytischen Wirkungen häufig). In besonders einfacher Weise lässt sich Aufkohlung bei Anwendung von rohrförmigen Reaktionsräumen metallografisch untersuchen. Dazu wird aus dem jeweiligen Rohrreaktor nach der jeweiligen Betriebszeit quer zu seiner Längsachse ein Querschnitt entnommen. Dieser wird geschliffen und poliert und anschließend zunächst mit 65 gew.-%iger (65 g HNO₃ in 35 g H₂O) wässriger Salpetersäure behandelt und nachfolgend mit 10 gew.-%iger ethanolischer Essigsäure (10 g Essigsäure in 90 g Ethanol) nachbehandelt, so dass das Stahlgefüge unter dem Mikroskop sichtbar wird. Ein Gefüge, welches durch eine Kohlenstoffaufnahme verändert ist, erscheint dunkelgrau bis schwarz. Die Aufkohlungstiefe kann mit einem Maßstab von der Oberfläche ausgehend bestimmt werden. Häufig geht mit dem Erscheinungsbild der Aufkohlung eine Sonderform der Korrosion, das so genannte "Metal Dusting" einher. Bei diesem Metallangriff handelt es sich um eine ausgeprägte Lokalkorrosion, die in der Regel von Muldenbildung begleitet ist. Im Unterschied zur Aufkohlung, die vergleichsweise gleichmäßig in den Werkstoff vordringt, ist Metal Dusting charakterisiert durch einen eher ungleichmäßig erfolgenden, dafür aber lokal vergleichsweise rasch in die tiefe fortschreitenden Angriff, bei dem sich zuvor gebildete Metallcarbide unter Freisetzung von Kohlenstoff und Metallpartikeln zersetzen. Dieser Angriff ist an den entsprechenden Oberflächenstellen in vorbeschriebener metallografischer Untersuchung unter dem Mikroskop mit bloßem Auge deutlich und relativ voluminös sichtbar ("Blumenkohlbildung"). Metal Dusting kann zu schnellem Materialversagen führen.

Die Bildung von Metallstaub ist auch insofern nachteilig, als sich dieser z. B. über den Reaktionsgasstrom im gesamten Reaktionsraum, einschließlich des Katalysatorbetts, verteilt. In der Regel handelt es sich bei den Metallstaubpartikeln jedoch um katalytisch aktive Partikel, die die Bildung von elementarem Kohlenstoff aus zu dehydrierendem Kohlenwasserstoff und/oder dehydriertem Kohlenwasserstoff fördern. Dies ist insbesondere dann nachteilig, wenn sich die Metallstaubpartikel im Katalysatorbett festsetzen und die dort gebildeten Kohlenstoffpartikel die aktive Oberfläche des Katalysators belegen und selbigen dadurch vorzeitig deaktivieren.

Weiterhin sollte der als Werkstoff erwählte Stahl unter den Bedingungen des relevanten Verfahrens Zunderbeständigkeit aufweisen (Verzunderung = oxidativer Angriff durch in der Reaktionsatmosphäre enthaltenen molekularen Sauerstoff). Das Erfordernis der Korrosionsbeständigkeit ist insbesondere auch unter dem Gesichtspunkt von Bedeutung, dass sich der beim eingangs dieser Schrift beschriebenen Verfahren gebildete dehydrierte Kohlenwasserstoff in einem sich an ein solches Verfahren anschließenden Verfahren (besonders vorteilhaft in Begleitung des nicht dehydrierten Kohlenwasserstoffs) heterogen katalysiert partialoxidieren lässt. Trennt man nachfolgend aus dem Produktgasgemisch der Partialoxidation das gewünschte Partialoxidationsprodukt ab, verbleibt normalerweise ein noch nicht dehydrierten Kohlenwasserstoff und Oxygenate enthaltendes Restgas, das vorteilhaft zum Zweck der weiteren Umsetzung des noch nicht dehydrierten Kohlenwasserstoffs wenigstens teilweise in das eingangs dieser Schrift beschriebene Verfahren rückgeführt wird. Der überwiegenden Mehrzahl aller Oxygenate (z. B. Acrolein, Acrylsäure, Methacrolein, Methacrylsäure, H₂O, O₂, CO₂, etc.) kommt jedoch korrosive Wirkung zu.

Ein weiteres Erfordernis an den erwählten Werkstoffstahl ist, dass er in vorteilhafter Weise verarbeitbar sein muss. Dies heißt insbesondere, dass er schweißtechnisch verarbeitbar sein muss, was bei vorab der Verarbeitung aloniserten Stählen ohne Beeinträchtigung der Alonisierung nicht möglich ist. Eine Alonisierung nach der Verarbeitung ist jedoch im großtechnischen Maßstab nur sehr aufwending bzw. gar nicht durchführbar.

Eigene Untersuchungen haben nun ergeben, dass keiner der im Stand der Technik empfohlenen Stähle das beschriebene Anforderungsprofil für sich allein zu erfüllen vermag.

Vielmehr hat sich gezeigt, dass in der Regel diejenigen der im Stand der Technik empfohlenen Stähle, die ≥ 1 Gew.-% Si enthalten, hinsichtlich ihrer Langzeitversprödungsneigung unter den relevanten Prozessbedingungen nicht zu befriedigen vermögen.

Umgekehrt haben sich diejenigen Stähle, die im Stand der Technik als optionale Werkstoffe in die Überlegungen einbezogen wurden und einen Si-Gehalt von ≤ 0,8 Gew.-% aufweisen, unter den relevanten Prozessbedingungen (insbesondere den erhöhten Reaktions- und/oder Katalysatorregeneriertemperaturen) in der Regel zwar als im wesentlichen nicht langzeitversprödend erwiesen, doch zeigten sie starkes Aufkohlen sowie Metal Dusting.

Die GB-A 2066696 empfiehlt als Problemlösung, die Einhüllende aus einem Nickel enthaltenden Werkstoff zu fertigen und diesen auf seiner den Reaktionsraum berührenden Seite mit einem an Nickel-freien Material zu beschichten, da insbesondere in Stahl enthaltener Nickel sowohl Aufkohlen als auch Metall Dusting (Metallstaubbildung) fördere.

Die EP-A 683 760 schließt sich an die Lehre der GB-A 2066696 an, und empfiehlt, den für die Einhüllende als Werkstoff zu verwendenden Stahl auf der den Reaktionsraum berührenden Seite mit einer Schutzschicht zu versehen, die insbesondere gemäß der erteilten Fassung der EP-A 683 760 (vgl. DE-69417879 T2, erteilte Patentansprüche sowie Seite 5, Zeilen 20ff und Seite 6, Zeilen 25 ff) zwingend über eine carbidreiche und nickelarme Verbundzwischenschicht mit dem Stahl verankert werden muss. Übliches Plattieren sei nicht ausreichend. Schließlich müsse die Schutzschicht auch beim Verfahrensbetrieb unversehrt bleiben.

Vor diesem Hintergrund bestand die Aufgabe der vorliegenden Erfindung darin, einen Werkstoff für die Einhüllende von Reaktionsräumen zur Durchführung eines wie eingangs dieser Schrift beschriebenen Verfahrens zur Verfügung zu stellen, der das beschriebene Anforderungsprofil besser als die im Stand der Technik empfohlenen bzw. nahegelegten Werkstoffe erfüllt und dies auch unter Einbezug der zur Katalysatorregenerierung in der Regel anzuwendenden Oxidations-/Reduktionszyklen.

Als Lösung wird ein Verfahren der kontinuierlichen heterogen katalysierten partiellen Dehydrierung wenigstens eines zu dehydrierenden Kohlenwasserstoffs in der Gasphase, umfassend eine Verfahrensweise, bei der man einen Reaktionsraum, der von einer den Reaktionsraum berührenden Einhüllenden (E) umschlossen ist, die wenigstens eine erste Öffnung zur Zufuhr wenigstens eines Ausgangsgasstroms in den Reaktionsraum und wenigsten eine zweite Öffnung zur Entnahme wenigstens eines Produktgasstroms aus dem Reaktionsraum aufweist,
- wenigstens einen, wenigstens einen zu dehydrierenden Kohlenwasserstoff enthaltenden, Ausgangsgasstrom kontinuierlich zuführt,
- im Reaktionsraum den wenigstens einen zu dehydrierenden Kohlenwasserstoff durch wenigstens ein im Reaktionsraum befindliches Katalysatorbett führt, und unter Erzeugung eines den wenigstens einen dehydrierten Kohlenwasserstoff, nicht umgesetzten zu dehydrierenden Kohlenwasserstoff sowie molekularen Wasserstoff und/oder Wasserdampf enthaltenden Produktgases oxidativ oder nicht oxidativ zu wenigstens einem dehydrierenden Kohlenwasserstoff partiell dehydriert, und
- dem Reaktionsraum wenigstens einen Produktgasstrom kontinuierlich entnimmt,
zur Verfügung gestellt, das dadurch gekennzeichnet ist, dass
die Einhüllende (E) aus einem Verbundwerkstoff gefertigt ist, der auf seiner den Reaktionsraum berührenden Seite B mit der Maßgabe aus Stahl B der nachfolgenden Elementzusammensetzung:

| | |
|---|---|
| 18 bis 30 Gew.-% | Cr, |
| 9 bis 37 Gew.-% | Ni, |
| 1 bis 4 Gew.-% (bzw. 1 bis 3 Gew.-%) | Si, |
| ≥ 0 bis 4 Gew.-% | Al, |
| ≥ 0 bis 0,3 Gew.-% | N, |
| ≥ 0 bis 0,15 Gew.-% | C, |
| ≥ 0 bis 4 Gew.-% | Mn, |
| ≥ 0 bis 0,05 Gew.-% | P, |
| ≥ 0 bis 0,05 Gew.-% | S, und |
| ≥ 0 bis 0,1 Gew.-% | eines oder mehrere seltene Erdmetalle, und |
| im übrigen Fe und herstellungsbedingte Verunreinigungen, wobei die Prozentangaben jeweils auf das Gesamtgewicht bezogen sind, | |

besteht, dass der Stahl B auf seiner vom Reaktionsraum abgewandten Seite A entweder unmittelbar, oder über eine Zwischenschicht aus Kupfer, oder aus Nickel, oder aus Kupfer und Nickel, auf Stahl A der Elementzusammensetzung

| | |
|---|---|
| 15 bis 20 Gew.-% | Cr, |
| 6 bis 18 (vorzugsweise 8 bis 16 bzw. bis 14) Gew.-% | Ni, |
| ≥ 0 bis 0,8 Gew.-% | Si, |
| ≥ 0 bis 0,8 Gew.-% | Al, |
| ≥ 0 bis 0,3 Gew.-% | N, |
| ≥ 0 bis 0,15 Gew.- | C, |
| ≥ 0 bis 4 Gew.-% | Mo, |
| ≥ 0 bis 2,0 Gew.-% | Mn, |
| ≥ 0 bis 0,8 Gew.-% | Ti, |
| ≥ 0 bis 1,2 Gew.-%, bzw. bis 0,5 Gew.-% | Nb, |
| ≥ 0 bis 0,9 Gew.-% | V, |
| ≥ 0 bis 0,1 Gew.-% | B, |
| ≥ 0 bis 0,05 Gew.-% | P, |
| ≥ 0 bis 0,05 Gew.-% | S, und |
| | |
| im übrigen Fe und herstellungsbedingte Verunreinigungen, wobei die Prozentangaben jeweils auf das Gesamtgewicht bezogen sind, | |

oder der Elementzusammensetzung

| | |
|---|---|
| 19 bis 23 Gew.-% | Cr, |
| 30 bis 35 Gew.-% | Ni, |
| ≥ 0 bis 1 Gew.-% | Co, |
| ≥ 0 bis 1 Gew.-% | Si, |
| 0,15 bis 0,7 Gew.-% | Al, |
| ≥ 0 bis 0,12 Gew.-% | C, |
| ≥ 0 bis 2,0 Gew.-% | Mn, |
| ≥ 0 bis 0,75 Gew.-% | Cu, |
| 0,15 bis 0,7 Gew.-% | Ti, |
| ≥ 0 bis 0,05 Gew.-%, vorzugsweise bis 0,015 Gew.-% | P, |
| ≥ 0 bis 0,05 Gew.-%, vorzugsweise bis 0,01 Gew.-% | S, und |
| im übrigen Fe und herstellungsbedingte Verunreinigungen, wobei die Prozentangaben jeweils auf das Gesamtgewicht bezogen sind, | |

aufplattiert ist.

Beispielsweise kommt als Stahl A für das erfindungsgemäße Verfahren der DIN-Typ 1.4876 (auch Alloy 800 H genannt) in Betracht.

Angesichts der im Stand der Technik gegebenen Lehren überrascht, dass das erfindungsgemäße Verfahren die gestellte Aufgabe in befriedigender Weise zu lösen vermag, muss beim erfindungsgemäßen Verfahren der Stahl B doch wenigstens 9 Gew.-% Ni enthalten.

Unter Plattieren soll in dieser Schrift in Übereinstimmung mit Ullmanns Encyklopädie der technischen Chemie, 4. Auflage, 1973, Band 3, Verfahrenstechnik und Reaktionsapparate, Verlag Chemie, Weinheim, Seite 26 ff, das Herstellen eines bei der Warm-und Kaltverarbeitung, beim Schweißen und bei normaler Beanspruchung nicht trennbaren Verbundes zweier oder mehrerer Metallschichten, dem Grundwerkstoff und dem Auflagewerkstoff verstanden werden. Erfindungsgemäß vorteilhaft ist der Verbund so beschaffen, dass er die Anforderungen der EN 13445-2:2002 (D) Ausgabe 1 (2002 - 05) für plattierte Produkte erfüllt. Dies gilt insbesondere für die technischen Lieferbedingungen für plattierte Produkte für Druckgeräte gemäß Anhang C.

D. h., wie bereits gesagt, die Bindung zwischen Grundwerkstoff und Auflagewerkstoff muss ausreichend fest sein, so dass es weder bei der Herstellung der Einhüllenden (E) noch beim Betreiben des erfindungsgemäßen Verfahrens zu Schichtentrennungen kommt.

Anwendungstechnisch zweckmäßig wird die Scherfestigkeit der Plattierung (bestimmt nach DIN 50162, September 1978) bei einer Zugfestigkeit des Auflagewerkstoffs von weniger als 280 N/mm² (bestimmt gemäß DIN 50111) mehr als die Hälfte der Mindest-Zugfestigkeit des Auflagewerkstoffs betragen und darf bei allen anderen Auflagewerkstoffen, unabhängig von der Prüfrichtung, nicht unter 140 N/mm² liegen.

Ferner sollte bei erfindungsgemäßem Verbundwerkstoff die Bindungsfläche vorteilhaft wenigstens 95 % der gesamten Kontaktfläche umfassen, wobei einzelne umgebundene Flächen nicht mehr als 50 cm² betragen dürfen. Plattieren kann man durch gemeinsames Auswalzen der zu verbindenden Metalle (Walzplattieren; K. Born: "Plattierte Bleche für höchste Beanspruchungen" in: Werkstofftechnik, DECHEMA-Monographie Bd. 45, Verlag Chemie, Weinheim 1962), durch Auftragschweißen des Auflagewerkstoffes auf den Grundwerkstoff (Schweißplattieren; K. Born, W. Speth: Herstellung plattierter Bauteile. Vergleichende Betrachtung zwischen Walzplattieren und Beschichten durch Auftragschweißen, Schweißen Schneiden, 19, 209-214, 1967) oder in der Weise, dass die Schichten durch eine stoßartige Druckwelle beim Explodieren eines Sprengstoffs miteinander verbunden werden (Sprengplattieren; H.C.A. Burkhardt: Schockwellenpyhsik, Metall 19, 1-9, 1965; U. Richter, J.F. Roth: Grundlagen und Anwendung des Sprengplattierens, Naturwissenschaften 57, 487-493, 1970; G. Buck, E. Hornbogen: Metallkundliche Untersuchungen an Explosivschweißnähten, Metall 20, 9-21, 1966) bewirkt werden.

Weist der erfindungsgemäß zu verwendende Verbundwerkstoff eine Zwischenschicht aus Kupfer, oder aus Nickel, oder aus Kupfer und Nickel auf, so sind sowohl der Stahl A als auch der Stahl B mit dieser Zwischenschicht durch plattieren verbunden. Im Rahmen einer z. B. Sprengplattierung kann der Gesamtverbund auch in einem Arbeitsschritt erzeugt werden. Im übrigen unterscheidet die Erfindung nicht, ob der Stahl A auf den Stahl B aufplattiert wurde oder umgekehrt (ist erfindungsgemäß bevorzugt). Vielmehr steht der Wortlaut "dass der Stahl B auf seiner vom Reaktionsraum abgewandten Seite A entweder unmittelbar, oder über eine Zwischenschicht aus Kupfer, oder aus Nickel, oder aus Kupfer und Nickel, auf Stahl A aufplattiert ist" für beide Varianten. In der Regel wird man den dünneren der beiden Stähle A, B auf den dickeren der beiden Stähle A,B aufplattieren. Weitere Angaben zum Plattieren von Werkstoffen findet der Fachmann in K. Bungardt, G. Lennartz, R. Oppenheim: Einfluss von Stickstoff auf die Eigenschaften austenitischer Chrom-Nickel- und Chrom-Nickel-Molybdän-(Kupfer)-Stähle, DEW (Deut. Edelstahlwerke) - Tech. Ber. 7, 71-90, 1967; R. Oppenheim: Chemisch beständige Stähle und Legierungen. Derzeitiger Stand und neuere Entwicklungen, DEW (Deut. Edelstahlwerke) - Tech. Ber. 7, 49-64, 1967; H. Warwesik: Reaktordruckgefäß, Atomwirt., Atomtech. 10, 376-379, 1965; W. Baberg: Dampferzeuger, Atomwirt., Atomtech. 10, 379 - 381, 1965; W. Klein: Gefügeänderungen beim Explosivplattieren von Stahl mit Stahl und einigen Nichteisenmetallen, Z. Metallk. 56, 261-267, 1965 und O. Wilms: Anwendung des Sprengplattierens mit Sonderwerkstoffen als Auflage, DEW (Dent. Edelstahlwerke) - Tech. Ber. 10, 176-183, 1970.

Die Verbindung der beiden Werkstoffe geschieht beim Plattieren teils auf mechanischem, teils auf metallurgischem Wege. Insgesamt wird beim Plattieren eine stoffschlüssige Verbindung der beiden Werkstoffe erreicht.

Mit Vorteil wird der erfindungsgemäß zu verwendende Verbundwerkstoff durch Sprengplattieren erzeugt. Bevorzugt bildet dabei (bzw. generell) der Stahl A den Grundwerkstoff und der Stahl B den Auflagewerkstoff.

Erfindungsgemäß vorteilhaft ist, dass Stähle A und Stähle B im Temperaturbereich von 20 bis 700°C ähnliche Wärmeausdehnungskoeffizienten γ (auch thermischer Längenausdehnungskoeffizient genannt; er wird üblicherweise an einem Stab der Länge 1 m und des homogenen Querschnitts 1 cm² ermittelt) aufweisen. Auch sind die Temperaturabhängigkeiten der Wärmeausdehnungskoeffizienten γ für Stähle A und Stähle B im vorgenannten Temperaturbereich vergleichbar.

Erfindungsgemäß bevorzugt unterscheiden sich die Wärmeausdehnungskoeffizienten γ von im erfindungsgemäßen Verbundwerkstoff enthaltenem Stahl A und im erfindungsgemäßen Verbundwerkstoff enthaltenem Stahl B bei 500°C und 1 atm um ≤ 2 · 10⁻⁶ m/m · K.

Besonders vorteilhaft beträgt der vorgenannte Unterschied ≤ 1,75 · 10⁻⁶ m/m · K, besser ≤ 1,5 · 10⁻⁶ m/m · K, bevorzugt ≤ 1.25 · 10⁻⁶ m/m · K, besonders bevorzugt ≤ 1,0 · 10⁻⁶ m/m · K, ganz besonders vorteilhaft ≤ 0,80 · 10⁻⁶ m/m · K, noch besser ≤ 0,60 · 10⁻⁶ m/m · K oder ≤ 0,50 · 10⁻⁶ m/m · K bzw. ≤ 0,40 · 10⁻⁶ m/m · K und am besten ≤ 0,30 · 10⁻⁶ m/m · K oder ≤ 0,20 · 10⁻⁶ m/m · K, oder ≤ 0,10 · 10⁻⁶ m/m · K und am allerbesten ≤ 0,05 · 10⁻⁶ m/m · K oder 0 m/m · K.

Die Ähnlichkeit der Wärmeausdehnungskoeffizienten γ für die Stähle A und B des erfindungsgemäß zu verwendenden Verbundwerkstoffs ist deshalb von Bedeutung, weil bei der Ausübung des erfindungsgemäßen Verfahrens insbesondere aufgrund der zwischenzeitlich durchzuführenden Katalysatorregenerierung zyklisch auftretend ein breiter Temperaturbereich zu durchfahren ist. Würden die Stähle A und B des erfindungsgemäß zu verwendenden Verbundwerkstoffs in diesem Temperaturbereich voneinander signifikant verschiedene Wärmeausdehnungskoeffizienten γ aufweisen, würde die den Reaktionsraum umschließende Einhüllende bei vorgenannter Temperaturvariation wie ein Bimetallthermometer deformiert, was im Rahmen einer Ausübung des erfindungsgemäßen Verfahrens unerwünscht ist (dies trifft insbesondere dann zu, wenn der Stahl B im Verlauf der Verfahrensausübung versprödet; entsprechende Deformierungen könnten Riß- und/oder Bruchbildung im Stahl B fördert). Beispielsweise wären un-oder niedrig legierte Stähle (sie enthalten eine Gesamtmenge von < 5 Gew.-% an von Fe verschiedenen Elementen) oder warmfeste martensitische Stähle mit bis zu 13 Gew.-% Cr (die beide aufgrund ihres günstigen Preises üblicherweise bevorzugte Reaktorwerkstoffe sind) als Plattierungspartner eines Stahls B zur Herstellung eines erfindungsgemäß zu verwendenden Verbundwerkstoffs aufgrund ihres deutlich anderen γ-Wertes ungeeignet.

Die Bezeichnung Kupfer und/oder Nickel soll in dieser Schrift neben den entsprechenden Reinmetallen auch Legierungen umfassen, die, bezogen auf ihr Gesamtgewicht, bis zu 5 Gew.-%, oder bis zu 4 Gew.-%, oder bis zu 3 Gew.-%, oder bis zu 2 Gew.-%, oder bis zu 1 Gew.-%, oder bis zu 0,5 Gew.-% von Kupfer und/oder Nickel verschiedene Elemente (Metalle) enthalten. Sie bilden alle vergleichsweise weiche Zwischenschichten (die auch nicht verspröden), weshalb eine Verschiedenheit ihres thermischen Ausdehnungskoeffizienten von jenem der Stähle A, B sich im wesentlichen nicht in der vorbeschriebenen negativen Weise auswirkt (sie agieren quasi wie ein Gummiband). Im übrigen ist der thermische Ausdehnungskoeffizient von z. B. reinem Kupfer im relevanten Temperaturbereich von jenem der Stähle A, B nur unwesentlich verschieden.

Anwendungstechnisch zweckmäßig beträgt die Stärke (Dicke) von Stählen B in erfindungsgemäß zu verwendenden Verbundwerkstoffen in der Regel 0,2 bis 25 mm, häufig 1 bis 15 mm oder 1 bis 10 mm. Vorteilhaft beträgt die vorgenannte Stärke 2 bis 8 mm, bevorzugt 4 bis 6 mm oder 5 mm.

Die Stärke (Dicke) von Stählen A in erfindungsgemäß zu verwendenden Verbundwerkstoffen beträgt anwendungstechnisch zweckmäßig 10 bis 150 mm, vielfach 20 bis 100 mm bzw. 60 bis 100 mm oder 20 (30) bis 75 mm bzw. 20 (30) bis 50 mm. Üblicherweise enthält der erfindungsgemäß zu verwendende Verbundwerkstoff den Stahl A und den Stahl B sowie gegebenenfalls die Zwischenschicht in jeweils möglichst, über den Verbundwerkstoff betrachtet, konstanter (einheitlicher) Dicke.

Die zu wählende Werkstoffdicke orientiert sich im Fall eines Stahles A sowohl an der spezifischen Stahlsorte als auch am Durchmesser der Einhüllenden des Reaktionsraums. Für größere Durchmesser sind größere Stärken (Dicken) des jeweiligen Stahls A und für kleinere Durchmesser sind geringere Stärken (Dicken) des jeweiligen Stahls A empfehlenswert.

Bei Verwendung eines Stahls A vom DIN-Typ 1.4910 beträgt vorgenannte Dicke erfindungsgemäß bevorzugt 20 (30) bis 50 mm. Bei Verwendung eines Stahls A vom DIN-Typ 1.4941, oder vom DIN-Typ 1.4948 , oder vom DIN-Typ 1.4541, beträgt vorgenannte Dicke (Stärke) erfindungsgemäß bevorzugt 40 bzw. 60 bis 100 mm. Je geringer der auslegungsrelevante Festigkeitswert (Dehngrenze oder Zeitstandfestigkeit; vgl. DIN EN 10028-7) des Stahls A, desto größer wird man anwendungstechnisch zweckmäßig die Stärke des Stahls A für den erfindungsgemäß zu verwendenden Verbundwerkstoff wählen.

Erfindungsgemäß bevorzugt handelt es sich sowohl bei den Stählen A als auch bei den Stählen B um austenitische (Edel)stähle. Sie unterscheiden sich von den ferritischen (kubisch raumzentrierte Gitterzellen der Kristallite) Stählen im wesentlichen durch den andersartigen Gefügeaufbau. Im besonderen bestehen ihre Kristallite aus den dichter gepackten kubisch-flächenzentrierten Gitterzellen. Im Rahmen der Herstellung von austenitischen Stählen wird normalerweise als abschließende Wärmebehandlung ein Lösungsglühen bei Temperaturen zwischen 1000°C und 1150°C mit anschließender Abkühlung in Wasser oder Luft durchgeführt, um die Ausbildung von Ausscheidungen zu vermeiden.

Weist der erfindungsgemäß einzusetzende Verbundwerkstoff eine Zwischenschicht aus Kupfer, oder aus Nickel, oder aus Kupfer und Nickel auf, so ist deren Stärke (Dicke) normalerweise ≥ 0,1 mm, meist ≥ 0,2 mm oder ≥ 0,3 mm. In der Regel wird die Dicke dieser Zwischenschicht jedoch 3 mm nicht überragen. D.h., üblicherweise beträgt die Stärke der Zwischenschicht ≤ 3 mm, oder ≤ 2,5 mm, vielfach ≤ 2 mm, oder ≤ 1,5 mm und vorzugsweise ≤ 1 mm. Bei Anwendung einer vorgenannten Zwischenschicht sollte keine nachträgliche (nach erfolgtem Plattieren) Lösungsglühung mehr vorgenommen werden.

Ohne Zwischenschicht kann nachträgliches (nach erfolgtem Plattieren) Lösungsglühen erfolgen (vorgenommen werden).

Die Zwischenschicht vermag einerseits zur Verbesserung der Anbindung des Stahls B an den Stahl A beizutragen und auch bei Relativbewegungen auftretende Scherkräfte aufzunehmen. Sollten bei der Durchführung des erfindungsgemäßen Verfahrens über eine längere Betriebszeit im Stahl B Risse auftreten, schützt die Zwischenschicht den Stahl A vor einem Kontakt mit dem Reaktionsgas und bewahrt den Stahl A so zusätzlich vor der Gefahr des Aufkohlens und einer Metallstaubbildung. Eine weitere vorteilhafte Eigenschaft der Zwischenschicht besteht darin, dass sie auch bei Kontakt mit dem Reaktionsgas nicht versprödet.

Erfindungsgemäß vorteilhaft beträgt die Gesamtmenge der herstellungsbedingten Verunreinigungen in den Stählen A,B, ganz generell und in gleicher Weise bezogen ≤ 1 Gew.-%, vorzugsweise ≤ 0,75 Gew.-%, besonders bevorzugt ≤ 0,5 Gew.-%, und ganz besonders bevorzugt ≤ 0,25 Gew.-%. In der Regel wird die Gesamtmenge der herstellungsbedingten Verunreinigungen des jeweiligen Stahls A, B jedoch ≥ 0,1 Gew.-% betragen. Im Unterschied zu den herstellungsbedingten Verunreinigungen handelt es sich bei den anderen Bestandteilen des Stahls um dessen Eigenschaften bestimmende Legierungsbestandteile. Dies gilt insbesondere für die Elemente Cr, Ni, Si, N und C, sowie Al, Mo, Mn, Ti, V und Nb.

Die seltenen Erdmetalle umfassen in dieser Schrift die Elemente Cer (Ce), Praseodym (Pr), Neodym (Nd), Promethium (Pm), Samarium (Sm), Europium (Eu), Gadolinium (Gd), Terbium (Tb), Dysprosium (Dy), Holmium (Ho), Erbium (Er), Thulium (Tm), Ytterbium (Yb) und Lutetium (Lu). Erfindungsgemäß bevorzugtes seltenes Erdmetall ist Ce. Erfindungsgemäß bevorzugt weist ein erfindungsgemäßer Stahl B daher ≥ 0 bis 0,1 Gew.-% Ce oder Ce und eines oder mehrere von Ce verschiedene (insbesondere La, Nd und/oder Pr) seltene Erdmetalle auf.

Ein erfindungsgemäß besonders bevorzugter Stahl B ist Stahl der DIN Werkstoffnummer 1.4841.

Ein erfindungsgemäß besonders bevorzugter Stahl A ist Stahl der DIN Werkstoffnummer 1.4910, der beispielsweise als Thermon® 4910 im Handel erhältlich ist. Dieser Stahl A ist durch eine besonders hohe Hochtemperaturfestigkeit (Dehngrenze, Zeitstandfestigkeit) bei gleichzeitig hoher Gefügestabilität (hoher Kerbschlagzähigkeit), und dies insbesondere nach erfindungsgemäßem Langzeiteinsatz, ausgezeichnet, was eine Anwendung in vergleichsweise dünnen Stärken (Dicken) ermöglicht. Dabei handelt es sich um Stahl im nachfolgenden Zusammensetzungsraster:

| | |
|---|---|
| 16 bis 18 Gew.-% | Cr, |
| 12 bis 14 Gew.-% | Ni, |
| ≥ 0 bis 0,8 Gew.-% | Si, |
| ≥ 0,10 bis 0,18 Gew.-% | N, |
| ≥ 0 bis 0,1 Gew.-% | C (vorzugsweise ≥ 0 bis 0,05 Gew.-%), |
| ≥ 2 bis 3 Gew.-% | Mo, |
| ≥ 0 bis 2 Gew.-% | Mn, |
| 0,0015 bis 0,0050 Gew.-% | B, |
| ≥ 0 bis 0,05 Gew.-% | P, |
| ≥ 0 bis 0,05 Gew.-% | S, und |
| | |
| im übrigen Fe und herstellungsbedingte Verunreinigungen, wobei die Prozentangaben jeweils auf das Gesamtgewicht bezogen sind. | |

Mit Vorteil liegen die P- und S-Gehalte im vorgenannten Zusammensetzungsraster bei folgenden Werten:

| | |
|---|---|
| ≥ 0 bis 0,035 Gew.-% | P und |
| ≥ 0 bis 0,015 Gew.-% | S. |

Erfindungsgemäße Verfahren sind deshalb dann ganz besonders vorteilhaft, wenn die Einhüllende des Reaktionsraums aus einem Verbundwerkstoff gefertigt ist, dessen Stahl B ein Stahl der DIN Werkstoffnummer 1.4841 und dessen Stahl A ein Stahl der DIN Werkstoffnummer 1.4910 ist, wobei der Stahl B entweder unmittelbar, oder über eine Zwischenschicht aus Kupfer, oder aus Nickel, oder aus Kupfer und Nickel auf den Stahl A aufplattiert (vorzugsweise sprengplattiert) ist.

Mit Vorteil wird dabei die Dicke (Stärke) des DIN 1.4910-Stahls zu 20 bzw. 30 bis 50 mm und die Dicke (Stärke) des DIN 1.4841 -Stahls zu 1 bis 10 mm, bevorzugt zu 4 bis 6 mm gewählt. Bei zusätzlicher Anwendung einer Zwischenschicht aus Cu, oder aus Ni, oder aus Cu und Ni ist deren Schichtdicke mit Vorteil 0,2 bis 2 mm, vorzugsweise 0,2 bis 1 mm.

Ein anderer für das erfindungsgemäße Verfahren günstiger Stahl B ist Stahl der DIN Werkstoffnummer 1.4828, der nachfolgende Zusammensetzung aufweisen kann:

| | |
|---|---|
| 19 bis 21 Gew.-% | Cr, |
| 11 bis 13 Gew.-% | Ni, |
| 1,5 bis 2,5 Gew.-% | Si, |
| ≥ 0 bis 0,2 Gew.-% | C, |
| ≥ 0 bis 2 Gew.-% | Mn, |
| ≥ 0 bis 0,05 Gew.-% | P, |
| ≥ 0 bis 0,05 Gew.-% | S, und |
| | |
| im übrigen Fe und herstellungsbedingte Verunreinigungen, wobei die Prozentangaben jeweils auf das Gesamtgewicht bezogen sind. | |

Mit Vorteil liegen die P- und S-Gehalte im vorgenannten Zusammensetzungsraster bei folgenden Werten:

| | |
|---|---|
| ≥ 0 bis 0,035 Gew.-% | P und |
| ≥ 0 bis 0,015 Gew.-% | S. |

Vorgenannter Stahl ist beispielsweise bei der Outokumpu GmbH erhältlich (D-47877 Willich).

D.h., erfindungsgemäß besonders geeignete Stähle B sind solche, deren Zusammensetzungen im nachfolgenden Zusammensetzungsraster liegen:

| | |
|---|---|
| 19 bis 26 Gew.-% | Cr, |
| 11 bis 22 Gew.-% | Ni, |
| 1,5 bis 2,5 Gew.-% | Si, |
| ≥ 0 bis ≥ 0,15 Gew.-% | N, |
| ≥ 0 bis ≤ 0,5 Gew.-% | C, |
| ≥ 0 bis ≤ 2 Gew.-% | Mn, |
| ≥ 0 bis 0,05 Gew.-% | P, |
| ≥ 0 bis 0,05 Gew.-% (bzw. ≥ 0 bis 0,02 Gew.-%) | S, |
| ≥ 0 bis 0,1 Gew.-% | eines oder mehrere seltene Erdmetalle, und |
| | |
| im übrigen Fe und herstellungsbedingte Verunreinigungen, wobei die Prozentangaben jeweils auf das Gesamtgewicht bezogen sind, | |

In den vorgenannten Zusammensetzungsrastern kann der P-Gehalt auch ≥ 0 bis ≤ 0,035 Gew.-% betragen

Eine andere Gruppe S erfindungsgemäß geeigneter Stähle B subsumiert sich unter dem Zusammensetzungsraster

| | |
|---|---|
| 18 bis 30 Gew.-% | Cr, |
| 9 bis 36 Gew.-% (bzw. bis 37 Gew.-%) | Ni, |
| 1 bis 3 Gew.-% | Si, |
| 0,1 bis 0,3 Gew.-% | N, |
| ≥ 0 bis ≤ 0,15 Gew.-% | C, |
| ≥ 0 bis ≤ 4 Gew.-% | Mn, |
| ≥ 0 bis ≤ 4 Gew.-% | Al, |
| ≥ 0 bis 0,05 Gew.-% | P, |
| ≥ 0 bis 0,05 Gew.-% | S, |
| ≥ 0 bis 0,1 Gew.-% | eines oder mehrere seltene Erdmetalle, und |
| | |
| im übrigen Fe und herstellungsbedingte Verunreinigungen, wobei die Prozentangaben jeweils auf das Gesamtgewicht bezogen sind, | |

Vorgenannte Gruppe S von möglichen Stählen B ist ausführlich im Deutschen Aktenzeichen 102005061626.7 beschrieben. Innerhalb dieser Gruppe S sind folgende Zusammensetzungen bevorzugt:

| | |
|---|---|
| 18 bis 26 Gew.-% | Cr, |
| 9 bis 36 Gew.-% | Ni, |
| 1 bis 2,5 Gew.-% | Si, |
| 0,1 bis 0,3 Gew.-% | N, |
| ≥ 0, bevorzugt (unabhängig von den anderen Gehalten) | |
| 0,03 bis 0,15 Gew.-% | C, |
| ≥ 0 bis 3 Gew.-% | Mn, |
| ≥ 0 bis 4 Gew.-% | Al, |
| ≥ 0 bis 0,05 Gew.-% | P, |
| ≥ 0 bis 0,05 Gew.-% | S, und |
| ≥ 0 bis 0,1, vorzugsweise > 0 bis 0,1, und besonders bevorzugt 0,03 bis 0,08 (jeweils unabhängig von allen anderen Gehalten) Gew.-% eines oder mehrere seltene Erdmetalle (vorzugsweise Ce oder Ce und eines oder mehrere andere seltene Erdmetalle), und | |

im übrigen Fe und herstellungsbedingte Verunreinigungen, wobei die Prozentangaben jeweils auf das Gesamtgewicht (des Stahls) bezogen sind.

Weitere bevorzugte Zusammensetzungen innerhalb der Gruppe S von möglichen Stählen B sind die folgenden:

| | |
|---|---|
| 20 bis 25 Gew.-% | Cr, |
| 9 bis 20 bzw. (unabhängig von den anderen Gehalten) | |
| vorzugsweise bis 15 Gew.-% | Ni, |
| 1,4 bis 2,5 Gew.-% | Si, |
| 0,1 bis 0,3 Gew.-% | N, |
| ≥ 0, bevorzugt (unabhängig von den anderen Gehalten) | |
| 0,03 bis 0,15 Gew.-% | C, |
| ≥ 0 bis 3 Gew.-% | Mn, |
| ≥ 0 bis 4 Gew.-% | Al, |
| ≥ 0 bis 0,05 Gew.-% | P, |
| ≥ 0 bis 0,05 Gew.-% | S, und |
| ≥ 0 bis 0,1, vorzugsweise > 0 bis 0,1, und besonders bevorzugt 0,03 bis 0,08 (jeweils unabhängig von allen anderen Gehalten) Gew.-% eines oder mehrere seltene Erdmetalle (vorzugsweise Ce oder Ce und eines oder mehrere andere seltene Erdmetalle), und | |
| | |
| im übrigen Fe und herstellungsbedingte Verunreinigungen, wobei die Prozentangaben jeweils auf das Gesamtgewicht (des Stahls) bezogen sind. | |

Eine andere bevorzugte Untergruppe von Zusammensetzungen innerhalb der Gruppe S von möglichen Stählen B sind die folgenden:

| | |
|---|---|
| 20 bis 22 Gew.-% | Cr, |
| 10 bis 12 Gew.-% | Ni, |
| 1,4 bis 2,5 Gew.-% | Si, |
| 0,12 bis 0,2 Gew.-% | N, |
| ≥ 0, bevorzugt (unabhängig von allen anderen Gehalten) | |
| 0,05 bis 0,12 Gew.-% | C, |
| ≥ 0 bis 1 Gew.-% | Mn, |
| ≥ 0 bis 2, vorzugsweise (unabhängig von allen | |
| anderen Gehalten) 0 Gew.-% | Al, |
| ≥ 0 bis 0,045 Gew.-% | P, |
| ≥ 0 bis 0,015 Gew.-% | S, und |
| ≥ 0 bis 0,1, vorzugsweise > 0 bis 0,1, und besonders bevorzugt 0,03 bis 0,08 (jeweils unabhängig von allen anderen Gehalten) Gew.-% eines oder mehrere seltene Erdmetalle), und | |
| | |
| im übrigen Fe und herstellungsbedingte Verunreinigungen, wobei die Prozentangaben jeweils auf das Gesamtgewicht bezogen sind. | |

Ganz generell ist es im-Rahmen aller zur Gruppe S gehöriger Stähle B günstig, wenn der Gehalt an N ≥ 0,11, bessere ≥ 0,12, bevorzugt ≥ 0,13, besonders bevorzugt ≥ 0,14 und noch besser ≥ 0,15 Gew.-% beträgt.

Innerhalb der Gruppe S von Stählen B besonders günstige Stähle B sind diejenigen der EN Werkstoffnummern 1.4818, 1.4835 und 1.4854 sowie diejenigen der DIN Werkstoffnummern 1.4891 und 1.4893. Zur Gruppe S von erfindungsgemäß geeigneten Stählen B gehört auch der legierte Edelstahl THERMAX® 4835 der Firma Thyssen Krupp Nirosta GmbH, in D-47794 Krefeld, Germany.

Als erfindungsgemäß geeignete Stähle A mit 30 bis 35 Gew.-% Ni kommen insbesondere die DIN Werkstoffe 1.4958 sowie 1.4959 in Betracht.

Weitere erfindungsgemäß geeignete Stähle A sind der DIN Werkstoff 1.4941, dessen Zusammensetzung im folgenden Zusammensetzungsraster liegen kann

| | |
|---|---|
| 17 bis 19 Gew.-% | Cr, |
| 9 bis 12 Gew.-% | Ni, |
| ≥ 0 bis 0,8 Gew.-% | Si, |
| ≥ 0 bis 0,08 (bzw. ≥ 0,04 bis 0,08) Gew.-% | C, |
| ≥ 0 bis 2 Gew.-% | Mn, |
| ≥ 0 bis 0,6 Gew.-% | Mo, |
| ≥ 0 bis 0,8 Gew.-% | Ti, |
| ≥ 0 bis 0,005 Gew.-% | B, |
| ≥ 0 bis 0,05 Gew.-% | P, |
| ≥ 0 bis 0,05 Gew.-% | S, und |
| | |
| im übrigen Fe und herstellungsbedingte Verunreinigungen, wobei die Prozentangaben jeweils auf das Gesamtgewicht bezogen sind, | |

der DIN Werkstoff 1.4541 mit z. B. der Zusammensetzung

| | |
|---|---|
| 17,31 Gew.-% | Cr, |
| 10,05 Gew.-% | Ni, |
| 0,51 Gew.-% | Si, |
| 0,017 Gew.-% | N, |
| 0,042 Gew.-% | C, |
| 1,17 Gew.-% | Mn, |
| 0,025 Gew.-% | P, |
| < 0,00005 Gew.-% | S, |
| 0,29 Gew.-% | Ti, sowie |
| | |
| im übrigen Fe und herstellungsbedingte Verunreinigungen, wobei die Prozentangaben jeweils auf das Gesamtgewicht bezogen sind (Prüfzeugnis von Sterling Tubes), | |

sowie der DIN Werkstoff 1.4948, dessen Zusammensetzung im folgenden Zusammensetzungsraster liegen kann:

| | |
|---|---|
| 17 bis 19 Gew.-% | Cr, |
| 8 bis 11 Gew.-% | Ni, |
| ≥ 0 bis 0,8 Gew.-% | Si, |
| ≥ 0 bis 0,11 Gew.-% | N, |
| 0,04 bis 0,08 Gew.-% | C, |
| ≥ 0 bis ≤ 2,0 Gew.-% | Mn, |
| ≥ 0 bis 0,05 Gew.-% | P, |
| ≥ 0 bis 0,05 Gew.-% | S, und |
| | |
| im übrigen Fe und herstellungsbedingte Verunreinigungen, wobei die Prozentangaben jeweils auf das Gesamtgewicht bezogen sind. | |

Beispielsweise kann die Zusammensetzung des DIN Werkstoff 1.4948 lauten:

| | |
|---|---|
| 18,1 Gew.-% | Cr, |
| 8,3 Gew.-% | Ni, |
| ≥ 0 bis 0,8 Gew.-% | Si, |
| ≥ 0 bis 0,11 Gew.-% | N, |
| 0,05 Gew.-% | C, |
| ≥ 0 bis ≤ 2,0 Gew.-% | Mn, |
| ≥ 0 bis 0,05 Gew.-% | P, |
| ≥ 0 bis 0,05 Gew.-% | S, und |
| | |
| im übrigen Fe und herstellungsbedingte Verunreinigungen, wobei die Prozentangaben jeweils auf das Gesamtgewicht bezogen sind. | |

D.h., besonders geeignete Stähle A subsumieren unter das Zusammensetzungsraster

| | |
|---|---|
| 16 bis 19 Gew.-% | Cr, |
| 8 bis 14 Gew.-% | Ni, |
| ≥ 0 bis ≤ 0,8 Gew.-% | Gesamtmenge aus Si und Al, |
| ≥ 0 bis 0,2 Gew.-% | N, |
| ≥ 0 bis 0,1 Gew.-% | C, |
| ≥ 0 bis 3 Gew.-% | Mo, oder ≥ 0 bis 2,0 (bzw. bis 1,5) Gew.-% Mn, |
| ≥ 0 bis 0,8 Gew.-% | Ti, |
| ≥ 0 bis 0,05 Gew.-% | B, |
| ≥ 0 bis 0,05 Gew.-% | P, |
| ≥ 0 bis 0,05 Gew.-% | S, und |
| | |
| im übrigen Fe und herstellungsbedingte Verunreinigungen, wobei die Prozentangaben jeweils auf das Gesamtgewicht bezogen sind. | |

Ein weiterer erfindungsgemäß geeigneter Stahl ist der DIN Werkstoff 1.4949, der im folgenden Zusammensetzungsraster liegen kann:

| | |
|---|---|
| 17 bis 19 Gew.-% | Cr, |
| 9,5 bis 11,5 Gew.-% | Ni, |
| ≥ 0 bis 0,8 Gew.-% | Si, |
| 0,1 bis 0,18 Gew.-% | N, |
| ≥ 0 bis 0,04 Gew.-% | C, |
| ≥ 0,2 bis 0,5 Gew.-% | Mo, |
| ≥ 0 bis 2 Gew.-% | Mn, |
| ≥ 0 bis 0,035 Gew.-% | P, |
| ≥ 0 bis 0,015 Gew.-% | S, und |
| | |
| im übrigen und herstellungsbedingte Verunreinigungen, wobei die Prozentangaben jeweils auf das Gesamtgewicht bezogen sind. | |

Weitere erfindungsgemäß geeignete Stähle A können im nachfolgenden Zusammensetzungsraster liegen:

| | |
|---|---|
| 15 bis 17,5 Gew.-% | Cr, |
| 12 bis 17,5 Gew.-% | Ni, |
| 0,3 bis 0,6 Gew.-% | Si, |
| ≥ 0 bis 0,14 Gew.-% | N, |
| 0,04 bis 0,1 Gew.-% | C, |
| ≥ 0 bis 2 Gew.-% | Mo, |
| ≥ 0 bis 1,5 Gew.-% | Mn, |
| 10 mal C-Gehalt bis 1,2 Gew.-% | Nb, |
| 0,6 bis 0,85 Gew.-% | V, |
| ≥ 0 bis 0,035 Gew.-% | P, |
| ≥ 0 bis 0,015 Gew.-% | S, und |
| | |
| im übrigen Fe und herstellungsbedingte Verunreinigungen, wobei die Prozentangaben jeweils auf das Gesamtgewicht bezogen sind. | |

D. h., als erfindungsgemäße Stähle A kommen auch die DIN Werkstoffe 1.4961, 1.4981 und 1.4988 in Betracht.

Vorzugsweise ist bei allen erfindungsgemäß zu verwendenden Stählen A der Si-Gehalt erfindungsgemäß bevorzugt ≤ 0,7 Gew.-% oder ≤ 0,6 Gew.-%, besser ≤ 0,5 Gew.-% oder ≤ 0,4 Gew.-%, noch besser ≤ 0,3 Gew.-%, ganz besonders bevorzugt ≤ 0,2 Gew.-% oder ≤ 0,1 Gew.-%, und am besten enthalten erfindungsgemäß zu verwendende Stähle A kein Si.

In völliger Entsprechung dazu ist bei allen erfindungsgemäß zu verwendenden Stählen A der Al-Gehalt erfindungsgemäß bevorzugt ≤ 0,7 Gew.-% oder ≤ 0,6 Gew.-%, besser ≤ 0,5 Gew.-% oder ≤ 0,4 Gew.-%, noch besser ≤ 0,3 Gew.-%, ganz besonders bevorzugt ≤ 0,2 Gew.-% oder ≤ 0,1 Gew.-%, und am besten enthalten erfindungsgemäß zu verwendende Stähle A kein Al.

Die Kerbschlagzähigkeit der erfindungsgemäß zu verwendenden Stähle A beträgt in der Regel ≥ 50 J.

Sowohl die Stähle A als auch die Stähle B enthalten ganz allgemein häufig ≥ 0,01 Gew.-%, oder ≥ 0,02 Gew.-%, oder ≥ 0,03 Gew.-% C, insbesondere die in dieser Schrift genannten.

Sowohl die Stähle A als auch die Stähle B enthalten in der Regel möglichst geringe Gehalte an P und an S. Bei allen aufgeführten Stahl-(A, B)-Zusammensetzungen dieser Schrift gelten erfindungsgemäß zweckmäßig folgende Gehalte:

| | |
|---|---|
| ≥ 0 bis ≤ 0,035 Gew.-% | P und |
| ≥ 0 bis ≤ 0,02 Gew.-% | S. |

Erfindungsgemäß bevorzugt sind auch die den wenigstens einen Ausgangsgasstrom zum Reaktionsraum hin - sowie den wenigstens einen Produktgasstrom vom Reaktionsraum wegführenden (Rohr)Leitungen aus dem erfindungsgemäß zu verwendenden Verbundwerkstoff gefertigt. Diese Rohre (insbesondere die Produktgas-wegführenden) können aber grundsätzlich auch nur aus Stählen der DIN Werkstoffnummern 1.4891, oder 1.4893 oder 1.4910, oder 1.4941, oder 1.4541 oder 1.4841 gefertigt sein. Die Verwendung von erfindungsgemäßem Verbundwerkstoff ist insbesondere dann von erhöhter Relevanz, wenn Reaktionsgas mit Werkstoff in Berührung kommt, der eine Temperatur ≥ 450°C aufweist und der Arbeitsdruck > 1 atm beträgt.

Die vorgenannten (Rohr)Leitungen werden für einen sachgerechten und sicheren Betrieb bevorzugt mit Einrichtungen zur Kompensation von Längenausdehnungseffekten, wie sie z. B. aufgrund von Temperaturänderungen auftreten können, ausgerüstet, wobei mit Vorteil Kompensatoren eingesetzt werden, die sich durch laterale Wirkungsweise auszeichnen.

Diese in der Regel mehrschichtig ausgeführten Kompensatoren können aus dem gleichen Werkstoff wie die Rohrleitung selbst gefertigt sein. Besonders vorteilhaft sind jedoch Ausführungsformen mit (allgemein: gasdurchlässigem starrem Innenrohr und gasundurchlässiger elastischer Außenhülle (gasundurchlässigem elastischen Außenrohr)) einem das zu führende Gas berührenden inneren, vorzugsweise aus dem erfindungsgemäß zu verwendenden Verbundwerkstoff gefertigten, Rohrteil, der zweckmäßig eine gasdurchlässige Dehnfuge aufweist, und einem außenliegenden, gasundurchlässigen, elastischen, gewellten Teil, der wenigstens teilweise aus einem insbesondere mechanisch und temperaturbelastbaren Werkstoff gefertigt ist, wie z. B. dem Werkstoff 1.4876 (Bezeichnung nach VdTÜV-Wb 434) bzw. 1.4958/1.4959 (Bezeichnung nach DIN 17459) bzw. INCOLOY® 800H bzw. 800HT oder Nickelbasiswerkstoff 2.4816 (alternative Bezeichnung Alloy 600) oder 2.4851 (alternative Bezeichnung Alloy 601).

Grundsätzlich kann die Einhüllende (E) für den Reaktionsraum ebenso wie die zu- und abführenden Rohrleitungen aber auch aus Nickelbasiswerkstoff 2.4642 (alternative Bezeichnung Alloy 690 bzw. Inconel H 690) gefertigt sein.

Der erfindungsgemäß zu verwendende Verbundwerkstoff eignet sich insbesondere für solche erfindungsgemäßen Verfahren, bei denen die Reaktionstemperaturen und/oder Katalysatorregeneriertemperaturen ≥ 400 bis 900°C, bzw. ≥ 500 bis 800°C, bzw. ≥ 550 bis ≤ 750°C, bzw. ≥ 600 bis ≤ 700°C betragen.

Im übrigen sind erfindungsgemäß zu verwendende Stähle A, B im Handel erhältlich oder in an sich bekannter Weise herstellbar. Vgl. z. B. die Ausführungen in Enzyklopädie Naturwissenschaften und Technik, Verlag moderne Industrie, 1976, unter den Stichworten "Stahlbegleiter", "Eisen" sowie "Eisen und Stahl", oder Ullmanns Encyclopädie der technischen Chemie, Verlage Chemie, 4. Auflage, Band 3, 1973, Verfahrenstechnik II und Reaktionsapparate, Kapitel "Werkstoffe in der chemischen Industrie".

Selbstredend kann im erfindungsgemäß zu verwendenden Verbundwerkstoff der Stahl B auf seiner den Reaktionsraum berührenden Seite auch in alonisierter, alitierter und/oder aluminierter Form enthalten sein. Wird die Alonisierung z. B. fertigungsbedingt beschädigt, ist dies wenig nachteilig, kommt dann doch noch immer die erfindungsgemäße Vorteilhaftigkeit zur Geltung.

Das erfindungsgemäße Verfahren ist insbesondere dann vorteilhaft, wenn der wenigstens eine Ausgangsgasstrom als inertes Verdünnungsgas Wasserdampf (z. B. ≥ 1 Vol.-%) und/oder als Reaktand molekularen Sauerstoff (z. B. ≥ 0,1 oder ≥ 0,5 Vol.-%) enthält. Es ist aber auch dann vorteilhaft, wenn der wenigstens eine Ausgangsgasstrom Wasserdampf und/oder molekularen Sauerstoff als Verunreinigungen enthält. Es ist ferner dann von Vorteil, wenn im Verlauf der erfindungsgemäßen heterogen katalysierten partiellen Dehydrierung Wasserdampf als Reaktionsprodukt gebildet wird. Dies insbesondere dann, wenn für das erfindungsgemäße Verfahren die in der WO 03/076370 empfohlene Kreisgas- bzw. Schlaufenfahrweise angewendet wird. Das erfindungsgemäße Verfahren ist aber nicht zuletzt dann besonders vorteilhaft, wenn der wenigstens eine Ausgangsgasstrom aus einer dem erfindungsgemäßen Verfahren nachgeschalteten Partialoxidation von im Rahmen des erfindungsgemäßen Verfahrens gebildetem dehydriertem Kohlenwasserstoff in Begleitung von noch zu dehydrierendem Kohlenwasserstoff stammendes, nach Zielproduktabtrennung aus dem Produktgasgemisch der Partialoxidation verbliebenes, Oxygenat enthaltendes Restgas umfasst.

Ganz generell kann das wenigstens eine im Reaktionsraum befindliche Katalysatorbett sowohl ein Wirbelbett, oder ein Fließbett, oder ein Festbett sein. Selbstredend können Wirbelbett und z. B. Festbett, oder Fließbett und Festbett im Reaktionsraum auch kombiniert enthalten sein. Erfindungsgemäß bevorzugt handelt es sich bei dem wenigstens einen Katalysatorbett des erfindungsgemäßen Verfahrens ausschließlich um Katalysatorfestbetten.

Unter der Belastung eines einen Reaktionsschritt katalysierenden Katalysatorbetts mit Reaktionsgas soll in dieser Schrift ganz generell die Menge an Reaktionsgas in Normlitern (= NI; das Volumen in Litern, das die entsprechende Reaktionsgasmenge bei Normalbedingungen (0°C, 1 atm) einnehmen wurde) verstanden, die pro Stunde durch einen Liter Katalysatorbett (z. B. Katalysatorfestbett) geführt wird. Die Belastung kann aber auch nur auf einen Bestandteil des Reaktionsgases bezogen sein. Dann ist es die Menge dieses Bestandteils in NI/I•h, die pro Stunde durch einen Liter des Katalysatorbetts geführt wird (reine Inertmaterialschüttungen werden nicht zu einem Katalysatorfestbett gerechnet). Die Belastung kann auch nur auf die in einem Katalysatorbett, das den eigentlichen Katalysator mit Inertmaterial verdünnt enthält, enthaltene Menge an Katalysator bezogen sein (dies wird dann explizit vermerkt).

Unter einer vollständigen Oxidation (Verbrennung) eines dehydrierten und/oder zu dehydrierenden Kohlenwasserstoffs wird in dieser Schrift verstanden, dass der im Kohlenwasserstoff insgesamt enthaltene Kohlenstoff in Oxide des Kohlenstoffs (CO, CO₂) umgewandelt wird. Alle davon verschiedenen Umsetzungen eines dehydrierten und/oder zu dehydrierenden Kohlenwasserstoffs unter reaktiver Einwirkung von molekularem Sauerstoff werden in dieser Schrift mit dem Begriff der Partialoxidation subsummiert. Die zusätzliche reaktive Einwirkung von Ammoniak kennzeichnet die Ammoxidation, die ebenfalls unter dem Begriff der Partialoxidation subsumieren soll.

Als ein Inertgas soll in dieser Schrift generell ein Reaktionsgasbestandteil verstanden werden, der sich unter den Bedingungen der entsprechenden Reaktion im wesentlichen als chemisch inert verhält und - jeder inerte Reaktionsgasbestandteil für sich betrachtet - zu mehr als 95 mol-%, vorzugsweise zu mehr als 97 mol-% bzw. zu mehr als 99 mol-% chemisch unverändert erhalten bleibt. Beispiele für typische inerte Verdünnungsgase sind z. B. N₂, H₂O, CO₂, Edelgase wie Ne und Ar sowie Mischungen aus diesen Gasen etc..

Handelt es sich beim erfindungsgemäßen Verfahren um eine heterogen katalysierte Oxidehydrierung (z. B. um diejenige von Propan zu Propylen), können als Quelle des dafür benötigten molekularen Sauerstoff Luft, reiner molekularer Sauerstoff oder an molekularem Sauerstoff angereicherte Luft bzw. sonstige Gemische aus molekularem Sauerstoff und Inertgas eingesetzt werden. Als weitere mögliche Sauerstoffquelle kommen Stickoxide in Betracht.

Die Notwendigkeit der Durchführung des erfindungsgemäßen Verfahrens an im festen Zustand befindlichen selektiv wirkenden Dehydrierkatalysatoren ist dadurch bedingt, dass die Dehydrierung (Spaltung von C-H) gegenüber der thermischen Spaltung bzw. Crackung (Spaltung von C-C) kinetisch benachteiligt ist. Infolge der selektiv wirkenden Katalysatoren sowie in Anwendung eines erfindungsgemäßen Reaktionsraumes entstehen im Fall einer erfindungsgemäßen heterogen katalysierten Dehydrierung von Propan Nebenprodukte wie Methan, Ethylen und Ethan nur in untergeordneten Mengen.

Unter einem Dehydrierkatalysator soll daher in dieser Schrift im besonderen ein Formkörper verstanden werden, dessen Längstausdehnung L (längste direkte Verbindungslinie zweier auf der Oberfläche des Formkörpers befindlicher Punkte) 0,1 bzw. 1 bis 30 mm, vorzugsweise 1 bis 20 mm und besonders bevorzugt 1 bis 10 mm bzw. 1 bis 5 mm beträgt und der im nachfolgend beschriebenen Versuch, bezogen auf einmaligen Durchgang des Reaktionsgases durch das Reaktionsrohr, wenigstens 5 mol-% des im Reaktionsgas enthaltenen Propan zu Propylen dehydriert:
Ein Reaktionsrohr aus Stahl der EN Werkstoffnummer 1.4835 (einem Stahl B) mit einer Wanddicke von 2 mm und einem Innendurchmesser von 35 mm sowie einer Länge von 80 cm wird wie folgt befüllt. 50 ml aus einer Schüttung des entsprechenden Dehydrierkatalysators werden im Reaktionsrohr mittig platziert. Oberhalb und unterhalb der Schüttung aus Katalysatorformkörper wird das Reaktionsrohr jeweils mit einer Schüttung aus Steatitkugeln (Inertkugeln) mit einem Kugeldurchmesser von 1,5 mm bis 2,5 mm aufgefüllt. Ein Gitterrost trägt die gesamte Schüttung. Von außen wird das Reaktionsrohr auf seiner gesamten Länge auf einer Temperatur von 550°C gehalten. Das Reaktionsrohr wird mit einem Gemisch aus Propan und Wasserdampf im Volumenverhältnis 2 (Propan) zu 1 (Wasserdampf) mit einer Propanbelastung der Katalysatorschüttung von 1000 NI/I•h beschickt. Der Ausgangsgasstrom ist auf eine Temperatur von 550°C vorerwärmt. Besonders bevorzugt sind Dehydrierkatalysatoren, bei denen unter vorstehenden Rahmenbedingungen die Summenselektivität der Bildung der Nebenprodukte Ethan, Ethylen und Methan ≤ 5 mol-%, bezogen auf umgesetztes Propan, beträgt.

Eine erfindungsgemäße heterogen katalysierte Oxidehydrierung lässt sich prinzipiell so durchführen, wie zum Beispiel in den Schriften US-A 4,788,371, CN-A 1073893, Catalysis Letters 23 (1994), 103-106, W. Zhang, Gaodeng Xuexiao Huaxue Xuebao, 14 (1993) 566, Z. Huang, Shiyou Huagong, 21 (1992) 592, WO 97/36849, DE-A 197 53 817, US-A 3,862,256, US-A 3,887,631, DE-A 195 30 454, US-A 4,341,664, J. of Catalysis 167, 560-569 (1997), J. of Catalysis 167, 550-559 (1997), Topics in Catalysis 3 (1996) 265-275, US 5,086,032, Catalysis Letters 10 (1991),181-192, Ind. Eng. Chem. Res. 1996,35,14-18, US 4,255,284, Applied Catalysis A: General, 100 (1993), 111-130, J. of Catalysis 148, 56-67 (1994), V. Cortés Corberän und S. Vic Bellón (Ed.), New Developments in Selective Oxidation II, 1994, Elsevier Science B.V., S. 305-313, 3rd World Congress on Oxidation Catalysis, R.K. Grasselli, S.T. Oyama, A.M. Gaffney and J.E. Lyons (Ed.), 1997, Elsevier Science B.V., S. 375ff oder in DE-A 198 37 520, DE-A 198 37 517, DE-A 198 37 519 und DE-A 198 37 518 am Beispiel der heterogen katalysierten partiellen Oxidehydrierung von Propan beschrieben. Dabei kann, wie bereits gesagt, als Sauerstoffquelle z. B. Luft eingesetzt werden. Häufig weist die verwendete Sauerstoffquelle neben Inertgas hier jedoch zu mindestens 90 mol.-% molekularen Sauerstoff, und vielfach zu wenigstens 95 mol-% molekularen Sauerstoff auf.

Die für die heterogen katalysierte Oxidehydrierung geeigneten Katalysatoren unterliegen keinen besonderen Beschränkungen. Es eignen sich alle dem Fachmann auf diesem Gebiet bekannten Oxidehydrierungskatalysatoren, die in der Lage sind, z. B. Propan zu Propylen zu oxidieren. Insbesondere können alle in den zuvor genannten Schriften genannten Oxidehydrierungskatalysatoren eingesetzt werden. Geeignete Katalysatoren sind beispielsweise Oxidehydrierungskatalysatoren, die MoVNb-Oxide oder Vanadylpyrophosphat, gegebenenfalls mit Promotor, umfassen. Ein Beispiel für einen günstigen Oxidehydrierkatalysator ist ein Katalysator, der ein Mischmetalloxid I mit Mo, V, Te, O und X als wesentliche Bestandteile enthält, wobei X mindestens ein Element ist, ausgewählt aus Niob, Tantal, Wolfram, Titan, Aluminium, Zirkonium, Chrom, Mangan, Gallium, Eisen, Ruthenium, Kobalt, Rhodium, Nickel , Palladium, Platin, Antimon, Bismut, Bor, Indium, Silizium, Lanthan, Natrium, Lithium, Kalium, Magnesium, Silber, Gold und Cer (vgl. dazu auch EP-A 938463 und EP-A 167109). Weitere besonders geeignete Oxidehydrierungskatalysatoren sind die Multimetalloxidmassen beziehungsweise -katalysatoren A (in dieser Schrift Multimetalloxidmassen II genannt) der DE-A-197 53 817 und die Katalysatoren der DE-A 19838312, wobei die in der erstgenannten Schrift als bevorzugt genannten Multimetalloxidmassen beziehungsweise - katalysatoren A ganz besonders günstig sind. Damit kommen für eine erfindungsgemäß heterogen katalysierte Oxidehydrierung als Aktivmassen u.a. Multimetalloxidmassen der allgemeinen Formel III

M¹ₐMo_{1-b}M²_{b}Oₓ (III),

mit
M¹ = Co, Ni, Mg, Zn, Mn und/oder Cu,
M² = W, V, Te, Nb, P, Cr, Fe, Sb, Ce, Sn und/oder La,
a = 0,5-1,5,
b = 0-0,5,
sowie
x = eine Zahl, die durch die Wertigkeit und Häufigkeit der von Sauerstoff verschiedenen Elemente in (III) bestimmt wird,
in Betracht. Ihre Herstellung und Formgebung kann wie in der DE-A 102 45 585 beschrieben erfolgen.

Für eine heterogen katalysierte Oxidehydrierung von z. B. Propan liegt die Reaktionstemperatur bei Verwendung frischer Katalysatoren vorzugsweise im Bereich von 200 bis 600°C, insbesondere im Bereich von 250 bis 500°C, stärker bevorzugt im Bereich von 350 bis 440°C. Der Arbeitsdruck liegt vorzugsweise im Bereich von 0,5 bis 10 bar, insbesondere von 1 bis 10 bar, stärker bevorzugt von 1 bis 5 bar. Arbeitsdrucke oberhalb von 1 bar, zum Beispiel von 1,5 bis 10 bar, haben sich als besonders vorteilhaft erwiesen. In der Regel erfolgt die heterogen katalysierte Oxidehydrierung des Propans an einem Katalysatorfestbett. Letzteres wird zweckmäßigerweise in den Rohren (die Rohrwand bildet zusammen mit den beiden Rohröffnungen die den Reaktionsraum berührende Einhüllende; das Rohrinnere ist der Reaktionsraum; vorzugsweise ist die Rohrwand vollständig aus erfindungsgemäßem Stahl gefertigt) eines z. B. salzbadgekühlten Rohrbündelreaktors aufgeschüttet (in der Regel auf einem gasdurchlässigen Gitterrost befindlich), wie sie zum Beispiel in der EP-A 700 893 und in der EP-A 700 714 sowie der in diesen Schriften zitierten Literatur beschrieben sind. Der Ausgangsgasstrom wird dem Rohreingang zugeführt. Die mittlere Verweilzeit des Reaktionsgases in der Katalysatorschüttung liegt zweckmäßigerweise bei 0,5 bis 20 Sekunden. Das Verhältnis von Propan zu Sauerstoff variiert mit dem gewünschten Umsatz und der Selektivität des Katalysators. Zweckmäßigerweise liegt es im Bereich von 0,5:1 bis 40:1, insbesondere von 1:1 bis 6:1, stärker bevorzugt von 2:1 bis 5:1. In der Regel nimmt die Propylenselektivität mit steigendem Propanumsatz ab. Vorzugsweise wird deshalb die Propan zu Propylen-Reaktion so durchgeführt, dass relativ niedrige Umsätze mit Propan bei hohen Selektivitäten zu Propylen erreicht werden. Besonders bevorzugt liegt der Umsatz an Propan im Bereich von 5 bis 40 mol-%, häufig im Bereich von 10 bis 30 mol-%. Hierbei bedeutet der Begriff "Propanumsatz" den Anteil an zugeführtem Propan, der beim einfachen Durchgang des Reaktionsgases durch das Rohr umgesetzt wird. In der Regel beträgt die Selektivität der Propylenbildung 50 bis 98 mol-%, stärker bevorzugt 80 bis 98 mol-%, wobei der Begriff "Selektivität" die Mole an Propylen bezeichnet, die pro Mol umgesetztem Propan erzeugt werden, ausgedrückt als molarer Prozentsatz. Im Reaktionsrohr durchläuft die Reaktionstemperatur in der Regel ein Maximum.

In der Regel enthält der bei einer heterogen katalysierten Propanoxidehydrierung eingesetzte Ausgangsgasstrom 5 bis 95 mol-% Propan (bezogen auf 100 mol-% Ausgangsgas). Neben Propan und Sauerstoff kann das Ausgangsgas für die heterogen katalysierte Oxidehydrierung auch weitere, insbesondere inerte, Bestandteile wie Kohlendioxid, Kohlenmonoxid, Stickstoff, Edelgase, andere Kohlenwasserstoffe, z. B. im Roh-Propan (als Propanquelle wird für das erfindungsgemäße Verfahren normalerweise Roh-Propan verwendet, wie es z. B. in der DE-A 102 45 585 bzw. in der DE-A 10 2005 022 798 empfohlen wird) enthaltene Nebenbestandteile, und/oder Propylen umfassen. Die heterogen katalysierte Oxidehydrierung kann auch in Anwesenheit von Verdünnungsmitteln, wie zum Beispiel Wasserdampf, durchgeführt werden.

Jede beliebige Reaktorsequenz kann zur Durchführung der heterogen katalysierten Oxidehydrierung von z. B. Propan eingesetzt werden, die dem Fachmann bekannt ist. Zum Beispiel kann die heterogen katalysierte Oxidehydrierung in einem einzigen Reaktor oder in einer Kaskade aus zwei oder mehreren Reaktoren, zwischen denen gegebenenfalls Sauerstoff zugeführt wird, durchgeführt werden.

Als mögliche Bestandteile kann das Produktgas einer erfindungsgemäßen heterogen katalysierten Propandehydrierung zum Beispiel folgende Bestandteile enthalten: Propylen (als Zielprodukt, d.h., als dehydrierter Kohlenwasserstoff), Propan (als nicht umgesetzter zu dehydrierender Kohlenwasserstoff), Kohlendioxid, Kohlenmonoxid, Wasser, Stickstoff, Sauerstoff, Ethan, Ethen, Methan, Acrolein, Acrylsäure, Methacrolein, Methacrylsäure, Furfurale, Ethylenoxid, Butan (z. B. n-Butan oder iso-Butan), Essigsäure, Formaldehyd, Ameisensäure, Propylenoxid und Butene (z. B. Buten-1). Dabei bilden insbesondere Ethan, Ethen und Methan mögliche thermische Zersetzungsprodukte von Propan. In typischer Weise enthält ein bei einer erfindungsgemäßen heterogen katalysierten Propanoxidehydrierung enthaltenes Produktgas: 5 bis 10 mol-% Propylen, 0,1 bis 2 mol-% Kohlenmonoxid, 1 bis 3 mol-% Kohlendioxid, 4 bis 10 mol-% Wasser, 0 bis 1 mol-% Stickstoff, 0,1 bis 0,5 mol-% Acrolein, 0 bis 1 mol-% Acrylsäure, 0,05 bis 2 mol-% Essigsäure, 0,01 bis 0,05 mol-% Formaldehyd, 1 bis 5 mol-% Sauerstoff, 0,1 bis 10 mol-% weitere oben genannte Bestandteile, sowie als Rest im wesentlichen Propan, jeweils bezogen auf 100 % Produktgas.

Heterogen katalysierte Oxidehydrierungen von von Propan verschiedenen zu dehydrierenden Kohlenwasserstoffen können erfindungsgemäß in entsprechender Weise durchgeführt werden, wie vorstehend für die Oxidehydrierung von Propan beschrieben. Als solche zu oxidehydrierenden Kohlenwasserstoffe kommen insbesondere Butan (zu Buten (vor allem iso-Butan zu iso-Buten) und/oder Butadien) sowie Butene (zu Butadien) in Betracht.

Die Regenerierung von für eine partielle heterogen katalysierte Oxidehydrierung von z. B. Propan verwendeten Katalysatoren kann so wie für Partialoxidationskatalysatoren in den Schriften DE-A 103 51 269, DE-A 103 50 812 und DE-A 103 50 822 beschrieben vorgenommen werden.

Handelt es sich bei der erfindungsgemäßen heterogen katalysierten partiellen Dehydrierung nicht um eine Oxidehydrierung, so beinhaltet sie stets eine konventionelle heterogen katalysierte Dehydrierung, d.h. es wird wenigstens intermediär molekularer Wasserstoff gebildet, der im Fall einer oxidativen (konventionellen) heterogen katalysierten partiellen Dehydrierung in einem Folgeschritt mit molekularem Sauerstoff wenigstens teilweise zu Wasser verbrannt wird.

Als Katalysatoren für eine heterogen katalysierte konventionelle Dehydrierung von zu dehydrierenden Kohlenwasserstoffen, kommen im Rahmen des erfindungsgemäßen Verfahrens grundsätzlich alle im Stand der Technik für konventionelle heterogen katalysierte Dehydrierungen bekannten Dehydrierkatalysatoren in Betracht. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen. Unter anderem können damit alle Dehydrierkatalysatoren eingesetzt werden, die in der WO 01/96270, der EP-A 731077, der DE-A 102 11 275, der DE-A 101 31 297, der WO 99/46039, der US-A 4,788,371, der EP-A-0 705 136, der WO 99/29420, der US-A 4,220,091, der US-A 5,430,220, der US-A 5,877,369, der EP-A-0 117 146, der DE-A 199 37 196, der DE-A 199 37 105, der US-A 3,670,044, der US-A 6,566,573, der US-A 4,788,371, der WO-A 94/29021 sowie der DE-A 199 37 107 empfohlen werden. Im besonderen können sowohl der Katalysator gemäß Beispiel 1, Beispiel 2, Beispiel 3, und Beispiel 4 der DE-A 199 37 107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Generell kann es sich bei den Dehydrierkatalysatoren um Katalysatorstränge (Durchmesser typisch 0,1 bzw. 1 bis 10 mm, bevorzugt 1,5 bis 5 mm; Länge typisch 1 bis 20 mm, bevorzugt 3 bis 10 mm), Tabletten (vorzugsweise gleiche Abmessungen wie bei den Strängen) und/oder Katalysatorringe (Außendurchmesser und Länge jeweils typisch 2 bis 30 mm oder bis 10 mm, Wandstärke zweckmäßig 1 bis 10 mm, oder bis 5 mm, oder bis 3 mm) handeln. Für eine heterogen katalysierte Dehydrierung im Wirbelbett (bzw. Fließ- oder Wanderbett) wird man entsprechend feinteiligeren Katalysator einsetzen. Erfindungsgemäß bevorzugt ist das Katalysatorfestbett.

In der Regel sind die Dehydrierkatalysatoren (insbesondere die in der DE-A 199 37 107 empfohlenen (insbesondere die beispielhaften Katalysatoren dieser DE-A)) so beschaffen, dass sie sowohl die Dehydrierung des zu dehydrierenden Kohlenwasserstoffs (z. B. Propan) als auch die Verbrennung des zu dehydrierenden Kohlenwasserstoffs (z. B. Propan) und von molekularem Wasserstoff zu katalysieren vermögen. Die Wasserstoffverbrennung verläuft dabei sowohl im Vergleich zur Dehydrierung des zu dehydrierenden Kohlenwasserstoff (z. B. Propans) als auch im Vergleich zu dessen Verbrennung im Fall einer Konkurrenzsituation an den Katalysatoren sehr viel schneller.

Ferner kommen für eine erfindungsgemäße konventionelle heterogen katalysierte partielle Dehydrierung eines zu dehydrierenden Kohlenwasserstoffs (z. B. Propan) prinzipiell alle im Stand der Technik bekannten Reaktortypen und Verfahrensvarianten in Betracht, sofern der Reaktionsraum im jeweiligen Reaktor das erfindungsgemäße Anforderungsprofil erfüllt. Beschreibungen solcher Verfahrensvarianten enthalten zum Beispiel alle bezüglich der Dehydrierkatalysatoren zitierten Schriften des Standes der Technik sowie der eingangs dieser Schriften zitierte Stand der Technik und die Schriften Deutsches Aktenzeichen 10 2006 017 623.5, 10 2006 015235.2, 10 2005 061 626.7. Das gleiche gilt für die Schriften EP-A 1 109 763, US-A 3,308,181, US-A 3,670,044, US-A 4,886,928, US-A 6,566,573, US-A 4,788,371 und WO 94/29021, sowie den in diesen Schriften zitierten Stand der Technik.

Eine vergleichsweise ausführliche Beschreibung von auch für erfindungsgemäße Reaktionsräume geeigneten Verfahren der konventionellen heterogen katalysierten Dehydrierung (nicht oxidativ oder oxidativ) enthält z. B. Catalytica® Studies Division, Oxidative Dehydogenation and Alternative DehydrogenationProcesses, Study Number 4192 OD, 1993, 430 Ferguson Drive, Mountain View, California, 94043-5272 U.S.A..

Charakteristisch für die konventionelle partielle heterogen katalysierte Dehydrierung von zu dehydrierenden Kohlenwasserstoffen (z. B. Propan) ist, dass der Dehydrierschritt endotherm verläuft. Das heißt, die für die Einstellung der erforderlichen Reaktionstemperatur benötigte Wärme (Energie) muss entweder dem Reaktionsgas vorab und/oder im Verlauf der heterogen katalysierten Dehydrierung zugeführt werden.

D. h., bezogen auf den einmaligen Durchgang des wenigstens einen Ausgangsgasstroms durch das wenigstens eine Katalysatorbett im erfindungsgemäß beschaffenen Reaktionsraum, kann der Reaktionsraum durch gezielten Wärmeaustausch mit z. B. außerhalb des von der erfindungsgemäßen Einhüllenden umschlossenen Reaktionsraums geführten fluiden (d.h. flüssigen oder gasförmigen) Wärmeträgern isotherm gestaltet werden (extern gelenkter Temperaturverlauf). Entsprechende Wärmeaustauscher können aber auch im Reaktionsraum selbst untergebracht sein.

Sie kann mit gleicher Bezugsbasis aber auch adiabat, d.h. im wesentlichen ohne einen solchen gezielten Wärmeaustausch mit (extern) geführten Wärmeträgern ausgeführt werden (extern ungelenkter Temperaturverlauf). Im letzteren Fall kann die auf den einmaligen Durchgang durch den erfindungsgemäßen Reaktionsraum bezogene Bruttowärmetönung durch im folgenden noch zu beschreibenden intern gelenkten (z. B. durch Wasserstoffverbrennung in einem Folgeschritt) Temperaturverlauf sowohl endotherm (negativ), oder autotherm (im wesentlichen Null) oder exotherm (positiv) gestaltet werden.

In typischer Weise benötigt eine erfindungsgemäße konventionelle heterogen katalysierte partielle Dehydrierung wenigstens eines zu dehydrierenden Kohlenwasserstoffs (z. B. von Propan), wie bereits erwähnt, vergleichsweise hohe Reaktionstemperaturen. Der dabei erzielbare Umsatz ist normalerweise durch das thermodynamische Gleichgewicht begrenzt. Typische Reaktionstemperaturen betragen 300 bis 800°C, bzw. 400 bis 700°C. Pro Molekül an z. B. zu Propylen dehydriertem Propan wird dabei ein Molekül Wasserstoff erzeugt. Hohe Temperaturen und Entfernung des Reaktionsproduktes H₂ begünstigen die Gleichgewichtslage im Sinne des Zielprodukts ebenso wie Partialdruckerniedrigung durch inerte Verdünnung.

Ferner ist es für konventionelle heterogen katalysierte partielle Dehydrierungen wenigstens eines zu dehydrierenden Kohlenwasserstoffs (z. B. von Propan) aufgrund der hohen benötigten Reaktionstemperaturen typisch, dass in geringen Mengen schwersiedende hochmolekulare organische Verbindungen, bis hin zum Kohlenstoff, gebildet werden, die sich auf der Katalysatoroberfläche abscheiden und selbige dadurch deaktivieren. Um diese nachteilige Begleiterscheinung zu minimieren, kann das zur konventionellen heterogen katalysierten Dehydrierung bei erhöhter Temperatur über die Katalysatoroberfläche zu leitende, den zu dehydrierenden Kohlenwasserstoff (z. B. das Propan) enthaltende Ausgangsgas mit Wasserdampf verdünnt werden. Sich abscheidender Kohlenstoff wird unter den so gegebenen Bedingungen nach dem Prinzip der Kohlevergasung kontinuierlich teilweise oder vollständig eliminiert.

Eine andere Möglichkeit, abgeschiedene Kohlenstoffverbindungen zu beseitigen, besteht darin, den Dehydrierkatalysator von Zeit zu Zeit bei erhöhter Temperatur mit einem Sauerstoff enthaltenden Gas zu durchströmen und damit den abgeschiedenen Kohlenstoff quasi abzubrennen. Eine sich daran anschließende reduktive Behandlung mit molekularem Wasserstoff schließt normalerweise die Katalysatorregenerierung ab. Eine gewisse Unterdrückung der Bildung von Kohlenstoffablagerungen (und damit eine Verlängerung der Katalysatorstandzeit) ist aber auch dadurch möglich, dass man dem konventionell heterogen katalysiert zu dehydrierenden Kohlenwasserstoff (z. B. Propan), bevor er bei erhöhter Temperatur über den Dehydrierkatalysator geführt wird, molekularen Wasserstoff zusetzt.

Selbstverständlich besteht auch die Möglichkeit, dem heterogen katalysiert zu dehydrierenden Kohlenwasserstoff (z. B. Propan), insbesondere zu vorgenannten Zwecken, Wasserdampf und molekularen Wasserstoff im Gemisch zuzusetzen. Ein Zusatz von molekularem Wasserstoff zur heterogen katalysierten Dehydrierung von Propan mindert auch die unerwünschte Bildung von Allen (Propadien), Propin und Acetylen als Nebenprodukten.

Erfindungsgemäß kann es daher (wie bereits angesprochen) zweckmäßig sein, die konventionelle heterogen katalysierte Kohlenwasserstoff(z. B. Propan)dehydrierung (z. B. mit vergleichsweise geringem Propan(bzw. generell Kohlenwasserstoff)umsatz) (quasi) adiabat durchzuführen. Das heißt, man wird das Ausgangsgas in der Regel zunächst auf eine Temperatur von 400 bzw. 500 bis 700°C (beziehungsweise von 550 bis 650°C) erhitzen (zum Beispiel durch Direktbefeuerung der es umgebenden Wandung). Im Normalfall wird dann ein einziger adiabater Durchgang durch wenigstens ein im erfindungsgemäßen Reaktionsraum befindliches Katalysatorbett ausreichend sein, um den gewünschten Umsatz zu erzielen, wobei sich das Reaktionsgas um etwa 30°C bis 200°C (je nach Umsatz und Verdünnung) abkühlen wird. Ein Beisein von Wasserdampf als Wärmeträger macht sich auch unter dem Gesichtspunkt einer adiabaten Fahrweise vorteilhaft bemerkbar. Die vergleichsweise niedrige Reaktionstemperatur ermöglicht längere Standzeiten des verwendeten Katalysatorbetts.

Prinzipiell ist eine erfindungsgemäße konventionelle heterogen katalysierte Kohlenwasserstoff(z. B. Propan)dehydrierung (unabhängig ob adiabat oder isotherm gefahren) sowohl im Katalysatorfestbett, als auch in einem Wanderbett oder Wirbelbett durchführbar.

Als Katalysatorbeschickung für eine konventionelle heterogen katalysierte Dehydrierung eines zu dehydrierenden Kohlenwasserstoffs (z. B. Propan) mit einem wie beschrieben vergleichsweise geringen Umsatz bei einmaligem Durchgang durch den erfindungsgemäßen Reaktionsraum, eignen sich insbesondere die in der DE-A 199 37 107 offenbarten, vor allem beispielhaft offenbarten, Katalysatoren, sowie deren Gemische mit bezüglich der konventionellen heterogen katalysierten Dehydrierung inerten geometrischen Formkörpern.

Nach längerer Betriebsdauer sind vorgenannte Katalysatoren zum Beispiel in einfacher Weise dadurch regenerierbar, dass man bei einer Eintrittstemperatur von 300 bis 600°C (in Extremfällen gegebenenfalls auch bis 750°C), häufig bei 400 bis 550°C, zunächst in ersten oxidativen Regenerierungsstufen mit Stickstoff und/oder Wasserdampf (bevorzugt) verdünnte Luft durch das Katalysator(fest)bett leitet. Die Katalysatorbelastung mit Regeneriergas kann (bezogen auf die regenerierte Katalysatorgesamtmenge) dabei z. B. 50 bis 10000 h⁻¹ und der Sauerstoffgehalt des Regeneriergases 0,1 bis 25, bzw. 0,5 bis 20 Vol.-% betragen.

In nachfolgenden weiteren oxidativen Regenerierungsstufen kann unter ansonsten gleichen Regenerierbedingungen als Regeneriergas Luft verwendet werden. Anwendungstechnisch zweckmäßig empfiehlt es sich, den Katalysator vor seiner Regenerierung mit Inertgas (z. B. H₂O, N₂, oder deren Gemisch) zu spülen.

Anschließend ist es in der Regel empfehlenswert, noch mit reinem molekularem Wasserstoff oder mit durch Inertgas (vorzugsweise Wasserdampf und/oder Stickstoff) verdünntem molekularem Wasserstoff (der Wasserstoffgehalt sollte ≥ 1 Vol.-% betragen) im ansonsten gleichen Bedingungsraster reduktiv zu regenerieren.

Eine konventionelle heterogen katalysierte Kohlenwasserstoff(z. B. Propan)dehydrierung kann beim erfindungsgemäßen Verfahren im erfindungsgemäßen Reaktionsraum bei Katalysatorbelastungen (bezogen auf die eingesetzte Katalysatorgesamtmenge) sowohl mit Ausgangsgas als auch mit darin enthaltenem zu dehydrierendem Kohlenwasserstoff (z. B. Propan) von 100 bis 10000 h⁻¹, häufig 300 bis 5000 h⁻¹, das heißt vielfach 500 bis 3000 h⁻¹ sowohl bei geringem (≤ 30 mol-%) als auch bei hohem (≥ 30 mol-%) Umsatz an zu dehydrierendem Kohlenwasserstoff (z. B. Propan) betrieben werden.

In besonders eleganter Weise lässt sich eine konventionelle erfindungsgemäße heterogen katalysierte Dehydrierung wenigstens eines zu dehydrierenden Kohlenwasserstoffs (z. B. Propan) (sowohl bei Dehydrierumsätzen ≤ 30 mol-% als auch > 30 mol-% (z. B. 40 mol-%, oder 50 mol-%)) in einem erfindungsgemäßen Hordenreaktionsraum verwirklichen.

Ein solcher enthält räumlich aufeinanderfolgend mehr als ein die Dehydrierung katalysierendes Katalysatorbett. Die Katalysatorbettenzahl kann z. B. 1 bis 20, zweckmäßig 2 bis 8, aber auch 3 bis 6 betragen. Vorzugsweise sind die Katalysatorbetten radial oder axial hintereinander angeordnet. Anwendungstechnisch zweckmäßig wird in einem solchen Hordenreaktionsraum der Katalysatorfestbetttyp angewendet.

Im einfachsten Fall sind die Katalysatorfestbetten im Reaktionsraum axial oder in den Ringspalten von zentrisch ineinandergestellten zylindrischen Gitterrosten angeordnet. Es ist jedoch auch möglich, die Ringspalte im Reaktionsraum in Segmenten übereinander anzuordnen und das Gas nach radialem Durchtritt in einem Segment in das nächste darüber- oder darunter liegende Segment zu führen.

In zweckmäßiger Weise wird das Reaktionsgas (Ausgangsgas) auf seinem Weg von einem Katalysatorbett zum nächsten Katalysatorbett zum Beispiel durch Überleiten über mit heißen Gasen erhitzte Wärmetauscherrippen oder durch Leiten durch mit heißen Brenngasen erhitzte Rohre bzw. mit heißen Gasen erhitzte Wärmetauscherplatten, im Hordenreaktionsraum einer Zwischenerhitzung unterworfen.

Wird das erfindungsgemäße Verfahren im Hordenreaktionsraum im übrigen adiabat betrieben, ist es für Dehydrierumsätze (z. B. Propanumsätze) ≤ 30 mol-%, insbesondere bei Verwendung der in der DE-A 199 37 107 beschriebenen Katalysatoren, insbesondere der beispielhaften Ausführungsformen, ausreichend, das Ausgangsgas auf eine Temperatur von 400 bzw. 450 bis 550°C (vorzugsweise 400 bis 500°C) vorerhitzt in den Reaktionsraum zu führen und innerhalb des Hordenreaktionsraums wenigstens in diesem Temperaturbereich zu halten. Das heißt, die gesamte erfindungsgemäße Dehydrierung ist so, wenigstens mit frischen Katalysatoren, bei vergleichsweise gemäßigten Temperaturen zu verwirklichen, was sich für die Standzeit der Katalysatorfestbetten zwischen zwei Regenerierungen als besonders günstig erweist.

Noch geschickter ist es, eine konventionelle heterogen katalysierte Dehydrierung im erfindungsgemäßen Reaktionsraum (wie ebenfalls bereits angesprochen) im wesentlichen autotherm durchzuführen, d. h., die vorstehend geschilderte Zwischenerhitzung auf direktem Weg durchzuführen (autotherme Fahrweise).

Dazu kann dem Reaktionsgas auf seinem Weg durch den erfindungsgemäßen Reaktionsraum z. B. nach Durchströmung des ersten Katalysatorbettes und zwischen den nachfolgenden Katalysatorbetten vorteilhaft in begrenztem Umfang molekularer Sauerstoff zugesetzt werden. Je nach verwendetem Dehydrierkatalysator wird so eine begrenzte Verbrennung der im Reaktionsgas enthaltenen Kohlenwasserstoffe, gegebenenfalls bereits auf der Katalysatoroberfläche abgeschiedener Kohle beziehungsweise kohleähnlicher Verbindungen und/oder von im Verlauf der konventionellen heterogen katalysierten Dehydrierung (z. B. einer Propandehydrierung) gebildetem und/oder dem Reaktionsgas zugesetztem Wasserstoff bewirkt (es kann auch anwendungstechnisch zweckmäßig sein, im Hordenreaktionsraum Katalysatorbetten einzufügen, die mit Katalysator beschickt sind, der spezifisch (selektiv) die Verbrennung von Wasserstoff (und/oder von Kohlenwasserstoff) katalysiert (als solche Katalysatoren kommen zum Beispiel jene der Schriften US-A 4,788,371, US-A 4,886,928, US-A 5,430,209, US-A 5,530,171, US-A 5,527,979 und US-A 5,563,314 in Betracht; beispielsweise können solche Katalysatorbetten in alternierender Weise zu den Dehydrierkatalysator enthaltenden Betten im Hordenreaktionsraum untergebracht sein)). Die dabei freigesetzte Reaktionswärme ermöglicht so (quasi adiabate Reaktorgestaltung) auf quasi autotherme Weise eine nahezu isotherme (interne Temperaturlenkung) Betriebsweise der heterogen katalysierten (z. B. Propan)Dehydrierung. Mit zunehmender gewählter Verweilzeit des Reaktionsgases im Katalysatorbett ist so eine (z. B. Propan)Dehydrierung bei abnehmender oder im wesentlichen konstanter Temperatur möglich, was besonders lange Standzeiten zwischen zwei Regenerierungen ermöglicht (Anstelle eines Hordenreaktors kann in dazu äquivalenter Weise auch eine Hintereinanderschaltung (Reihenschaltung) von einer der vorgenannten Hordenanzahl entsprechenden Anzahl von eine erfindungsgemäße Einhüllende E aufweisenden Reaktoren angewendet und dabei die Sauerstoffeinspeisung jeweils zwischen aufeinanderfolgenden Reaktoren vorgenommen werden. Die Anzahl von so verschalteten Reaktoren kann z. B. "drei", oder "zwei", oder "vier" betragen. In der Regel wird jeder einzelne dieser Reaktoren ein bzw. höchstens zwei Katalysatorfestbetten aufweisen).

Durch eine wie beschrieben durchgeführte Folgeverbrennung von im Verlauf der Dehydrierung gebildetem molekularem Wasserstoff wird aus einer nichtoxidativen konventionellen heterogen katalysierten Dehydrierung eine im Sinne der vorliegenden Anmeldung oxidative konventionelle heterogen katalysierte Dehydrierung.

In der Regel sollte eine wie vorstehend beschriebene Sauerstoffeinspeisung so vorgenommen werden, dass der Sauerstoffgehalt des Reaktionsgases, bezogen auf die darin enthaltene Menge an zu dehydrierendem Kohlenwasserstoff und dehydriertem Kohlenwasserstoff (z. B. Propan und Propylen), 0,01 bzw. 0,5 bis 30 Vol.-% beträgt. Als Sauerstoffquelle kommen dabei sowohl reiner molekularer Sauerstoff oder mit Inertgas, zum Beispiel CO, CO₂, N₂, Edelgase, verdünnter Sauerstoff, insbesondere aber auch Luft, in Betracht. Eine alternative Sauerstoffquelle können Stickoxide sein. Die resultierenden Verbrennungsgase wirken in der Regel zusätzlich verdünnend und fördern dadurch die heterogen katalysierte (z. B. Propan) Dehydrierung.

Die Isothermie einer konventionellen heterogen katalysierten (z. B. Propan)Dehydrierung lässt sich dadurch weiter verbessern, dass man im Hordenreaktionsraum in den Räumen zwischen den Katalysatorbetten geschlossene, vor ihrer Füllung günstigerweise aber nicht notwendigerweise evakuierte, Einbauten (zum Beispiel rohrförmige), anbringt. Derartige Einbauten können auch ins jeweilige Katalysatorbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Eine Möglichkeit, das Ausgangsgas bzw. den Ausgangsgasstrom für eine konventionelle heterogen katalysierte (z. B. Propan)Dehydrierung im erfindungsgemäßen Reaktionsraum auf die benötigte Reaktionstemperatur zu erwärmen, besteht auch darin, einen Teil des darin enthaltenen zu dehydrierenden Kohlenwasserstoffs (z. B. des Propans) und/oder H₂ mittels im Ausgangsgas enthaltenem molekularem Sauerstoff beim Eintritt in den Reaktionsraum zu verbrennen (zum Beispiel an geeigneten spezifisch wirkenden Verbrennungskatalysatoren, zum Beispiel durch einfaches Überleiten und/oder Durchleiten) und mittels der so freigesetzten Verbrennungswärme die Erwärmung auf die (für die Dehydrierung) gewünschte Reaktionstemperatur zu bewirken. Die resultierenden Verbrennungsprodukte, wie CO₂, H₂O sowie das den für die Verbrennung benötigten molekularen Sauerstoff gegebenenfalls begleitende N₂ bilden vorteilhafte inerte Verdünnungsgase.

Besonders elegant lässt sich die vorgenannte Wasserstoffverbrennung wie in der WO 03/076370 bzw. DE-A 102 11 275 beschrieben realisieren. D.h., in einem Verfahren der kontinuierlichen konventionellen oxidativen heterogen katalysierten partiellen Dehydrierung von zu dehydrierendem Kohlenwasserstoff (z. B. im Propan) im erfindungsgemäßen Reaktionsraum, bei dem man
- dem Reaktionsraum wenigstens einen, wenigstens einen zu dehydrierenden Kohlenwasserstoff (z. B. Propan), molekularen Sauerstoff, molekularen Wasserstoff und gegebenenfalls Wasserdampf enthaltenden Ausgangsgasstrom kontinuierlich zuführt,
- im Reaktionsraum den wenigstens einen zu dehydrierenden Kohlenwasserstoff durch wenigstens ein im Reaktionsraum befindliches Katalysatorbett führt, an welchem durch konventionelle heterogen katalysierte Dehydrierung molekularer Wasserstoff und wenigstens partiell wenigstens ein dehydrierter Kohlenwasserstoff (z. B. Propylen) gebildet werden,
- dem Reaktionsgas nach seinem Eintritt in den erfindungsgemäßen Reaktionsraum auf seinem Weg durch den Reaktionsraum gegebenenfalls weiteres molekularen Sauerstoff enthaltendes Gas zusetzt,
- der molekulare Sauerstoff im Reaktionsraum im Reaktionsgas enthaltenen molekularen Wasserstoff wenigstens teilweise zu Wasserdampf oxidiert, und
- dem Reaktionsraum wenigstens einen Produktgasstrom kontinuierlich entnimmt, der molekularen Wasserstoff, Wasserdampf, dehydrierten Kohlenwasserstoff (z. B. Propylen) und zu dehydrierenden Kohlenwasserstoff (z. B. Propan) enthält,
das dadurch gekennzeichnet ist, dass der dem erfindungsgemäßen Reaktionsraum entnommene wenigstens eine Produktgasstrom in zwei Teilmengen identischer Zusammensetzung aufgeteilt und eine der beiden Teilmengen als Dehydrierkreisgas in den dem erfindungsgemäßen Reaktionsraum zugeführten wenigstens einen Ausgangsgasstrom rückgeführt und die andere Teilmenge anderweitig weiterverwendet (z. B. zum Zweck einer heterogen katalysierten partiellen Oxidation von im Reaktionsraum gebildetem dehydriertem Kohlenwasserstoff) wird.

Beispielsweise kann das Produktgas einer erfindungsgemäßen, oxidativen oder nicht oxidativen heterogen katalysierten Dehydrierung von Propan zu Propylen die folgenden Gehalte aufweisen

| | |
|---|---|
| Propan | 25 bis 60 Vol.-%, |
| Propylen | 8 bis 25 Vol.-%, |
| H₂ | ≥ 0 bis 25 Vol.-% und |
| CO₂ | ≥ 0 bis 30 Vol.-%. |

In den vorstehenden Ausführungen wurde in individualisierter Form als nichtoxidativ oder oxidativ in konventioneller Weise heterogen katalysiert zu dehydrierender Kohlenwasserstoff stets Propan benannt. Selbstredend sind die beschriebenen Verfahrensweisen aber auch auf alle anderen eingangs dieser Schrift als zu dehydrierende Kohlenwasserstoffe aufgeführten Verbindungen anwendbar. Im besonderen seien unter diesen nochmals Butan (zu Buten und/oder Butadien; insbesondere iso-Butan zu iso-Buten) sowie von Butenen zu Butadien genannt.

Ganz allgemein enthält der wenigstens eine Ausgangsgasstrom einer erfindungsgemäßen, oxidativen oder nicht oxidativen heterogen katalysierten Dehydrierung in der Regel ≥ 5 Vol.-% des zu dehydrierenden Kohlenwasserstoffs (z. B. Propan). Daneben kann er z. B. enthalten:
a) N₂ und H₂O;
b) N₂, O₂ und H₂O;
c) N₂, O_{2,} H₂O und H₂;
d) N₂, O₂, H₂O, H₂ und CO_{2;}
e) N₂, O₂, H₂O, H₂, CO₂ und CO.

Wie bereits erwähnt, wird sich an das erfindungsgemäße Verfahren vielfach ein Verfahren der heterogen katalysierten Partialoxidation von erzeugtem dehydriertem Kohlenwasserstoff (z. B. Propylen zu Acrolein und/oder Acrylsäure), vorzugsweise in Begleitung vom nicht umgesetzten zu dehydrierenden Kohlenwasserstoff (z. B. Propan) als Inertgas anschließen. Dabei wird man den aus dem erfindungsgemäßen Reaktionsraum (kontinuierlich) entnommenen Produktgasstrom als solchen oder nach Abtrennung wenigstens einer Teilmenge seiner vom dehydrierten Kohlenwasserstoff (z. B. Propylen) und vom (nicht umgesetzten) zu dehydrierenden Kohlenwasserstoff (z. B. Propan) verschiedenen Bestandteile (z. B. H₂, H₂O, N₂, etc.) zur Beschickung wenigstens eines Oxidationsreaktors verwenden, und den im Beschickungsgasgemisch enthaltenen dehydrierten Kohlenwasserstoff (z. B. Propylen) einer selektiven heterogen katalysierten partiellen Gasphasenoxidation mit molekularem Sauerstoff zu einem das (Partialoxidationsprodukt) Zielprodukt (z. B. Acrolein, oder Acrylsäure oder deren Gemisch), sowie in der Regel nicht umgesetzten zu dehydrierenden Kohlenwasserstoff (z. B. Propan), überschüssigen molekularen Sauerstoff und gegebenenfalls nicht umgesetzten dehydrierten Kohlenwasserstoff (z. B. Propylen) enthaltenden Produktgasgemisch B unterwerfen.

In einer nachfolgenden Trennzone B wird man im Produktgasgemisch B enthaltenes Zielprodukt (z. B. Acrolein, oder Acrylsäure, oder deren Gemisch) abtrennen, und von dem dabei verbleibenden, nicht umgesetzten zu dehydrierenden Kohlenwasserstoff (z. B. Propan), molekularen Sauerstoff sowie gegebenenfalls nicht umgesetzten dehydrierten Kohlenwasserstoff (z. B. Propylen) enthaltenden Restgas wenigstens eine nicht umgesetzten zu dehydrierenden Kohlenwasserstoff (z. B. Propan) und gegebenenfalls nicht umgesetzten molekularen Sauerstoff sowie gegebenenfalls nicht umgesetzten dehydrierten Kohlenwasserstoff (z. B. Propylen) enthaltende Teilmenge als Partialoxidationskreisgas in das erfindungsgemäße Verfahren rückführen (z. B. als Bestandteil des Ausgangsgasstroms).

Handelt es sich beim erfindungsgemäßen Verfahren (aber auch sonst) z. B. um eine oxidative konventionelle heterogen katalysierte partielle Dehydrierung von Propan zu Propylen, und bei der sich anschließenden Partialoxidation um diejenige von Propylen zu Acrolein, oder zu Acrylsäure, oder zu deren Gemisch, kann der dem erfindungsgemäßen Reaktionsraum zugeführte wenigstens eine Ausgangsgasstrom z. B. als wesentliche Gehalte enthalten:

| | |
|---|---|
| Propylen | ≥ 0 bis 20 bzw. bis 10, häufig 0 bis 6 Vol.-%, |
| Acrolein | ≥ 0 bis 1, vielfach 0 bis 0,5, häufig 0 bis 0,25 Vol.-%, |
| Acrylsäure | ≥ 0 bis 0,25 (oder bis 0,4), vielfach 0 bis 0,05, häufig 0 bis 0,03 Vol.-%, |
| COₓ | ≥ 0 bis 20 bzw. bis 5, vielfach 0 bis 3, häufig 0 bis 2 Vol.-%, |
| Propan | 5 bis 50, vorzugsweise 20 bis 40 Vol.-%, |
| Stickstoff | 20 bzw. 30 bis 80, vorzugsweise 50 bis 70 Vol.-%, |
| Sauerstoff | ≥ 0 bis 5, vorzugsweise 1,0 bis 2,0 Vol.-%, |
| H₂O | ≥ 0 bis 20, vorzugsweise 5,0 bis 10,0 Vol.-%, und |
| H₂ | ≥ 0, häufig ≥ 0,01, oft ≥ 0,05 bis 10, vorzugsweise 1 bis 5 Vol.-%. |

In geringen Menge (etwa vergleichbar den möglichen Acrylsäuregehalten) kann auch Essigsäure enthalten sein.

Üblicherweise erfolgt die Abtrennung von Zielprodukt (z. B. Acrylsäure) aus dem Produktgasgemisch B dadurch, dass man das Zielprodukt (z. B. die Acrylsäure) in die kondensierte Phase überführt. Dies kann durch absorptive und/oder kondensative (kühlen) Maßnahmen erfolgen. Als Absorptionsmittel kommen im Fall von Acrylsäure als Zielprodukt z. B. Wasser, wässrige Lösungen oder hochsiedende (T_{siede} > T_{Siede} von Acrylsäure bei 1 atm), insbesondere hydrophobe, organische Lösungsmittel in Betracht. Besonders bevorzugt erfolgt das Überführen in die kondensierte Phase im Fall der Acrylsäure durch fraktionierende Kondensation des Produktgasgemisches B. Vorzugsweise erfolgt die absorptive und/oder kondensative Überführung der Acrylsäure aus dem Produktgasgemisch B in die kondensative Phase in trennwirksame Einbauten enthaltenden Kolonnen, in denen das Produktgasgemisch normalerweise von unten nach oben aufsteigend geführt wird. Das Absorptionsmittel wird in der Regel am Kolonnenkopf aufgegeben, an dem das Restgas normalerweise aus der Kolonne entlassen wird.

Die weitere Abtrennung der Acrylsäure aus der kondensierten Phase erfolgt in der Regel in der gewünschten Reinheit unter Anwendung wenigstens eines thermischen Trennverfahrens. Darunter werden solche Verfahren verstanden, bei denen wenigstens zwei voneinander verschiedene stoffliche Phasen (z. B. flüssig/flüssig; gasförmig/flüssig; fest/flüssig; gasförmig/fest etc.) erzeugt und miteinander in Kontakt gebracht werden. Aufgrund der zwischen den Phasen bestehenden Gradienten findet zwischen denselben ein Wärme- und Stoffaustausch statt, der letztlich die gewünschte Auftrennung (Abtrennung) bedingt. Die Bezeichnung "thermische Trennverfahren" reflektiert dabei, dass es entweder des Entzuges oder der Zufuhr von Wärme bedarf, um die Ausbildung der stofflichen Phasen zu erzeugen und/oder dass der Entzug oder die Zufuhr von thermischer Energie den Stoffaustausch begünstigt bzw. aufrechterhält. Erfindungsgemäß bevorzugt umfasst das wenigstens eine thermische Trennverfahren wenigstens eine kristallisative Abtrennung aus flüssiger Phase. Erfindungsgemäß zweckmäßig ist die wenigstens eine kristallisative Abtrennung der Acrylsäure eine Suspensionskristallisation und mit Vorteil wird das Suspensionskristallisat mit geschmolzenem, zuvor abgetrenntem und gewaschenem Kristallisat in einer Waschkolonne (eine gravimetrische, oder eine mechanische, oder eine hydraulische Waschkolonne; letztere ist erfindungsgemäß bevorzugt) gewaschen. Im übrigen kommen als thermische Trennverfahren z. B. extraktive, desorptive, kristallisative, rektifikative, azeotrop-destillative, azeotrop-rektifikative, destillative und/oder Strippverfahren in Betracht. In der Regel wird man zur Gewinnung von reiner Acrylsäure Kombinationen von verschiedenen der genannten thermischen Trennverfahren anwenden.

An die beschriebene Abtrennung der Acrylsäure kann sich ein Verfahren der radikalischen Polymerisation (insbesondere zur Herstellung von Wasser superabsorbierenden Polyacrylsäuren und/oder deren teil- bzw. vollneutralisierten Alkali(vorzugsweise Na)metallsalzen) anschließen, in welchem abgetrennte Acrylsäure zur Herstellung von Polymerisaten radikalisch einpolymerisiert wird.

Auch kann sich an die beschriebene Abtrennung der Acrylsäure ein Verfahren zur Herstellung von Acrylsäureestern anschließen, in welchem abgetrennte Acrylsäure mit Alkoholen (vorzugsweise Alkanolen, besonders bevorzugt C₁- bis C₁₂-Alkanolen) (in der Regel säurekatalysiert) verestert wird.

An das Verfahren der Veresterung kann sich wiederum ein Verfahren der radikalischen Polymerisation anschließen, in welchem so hergestellter Acrylsäureester einpolymerisiert wird.

Sieht man von der erfindungsgemäßen Besonderheit des erfindungsgemäßen Reaktionsraumes ab, sind erfindungsgemäße Verfahren zur Herstellung von Propylen aus Propan als Propylenquelle für Partialoxidationen desselben zur Herstellung von Acrolein und/oder Acrylsäure vorbekannt, einschließlich einer Kreisgasführung von Oxidationskreisgas und gegebenenfalls Dehydrierkreisgas. Beispielsweise findet man solche mehrstufigen Verfahren beschrieben in den Schriften DE-A 10 2005 022 798, Deutsches Aktenzeichen 10 2006 024 901.1, DE-A 102 46 119, DE-A 102 45 585, DE-A 10 2005 049 699, DE-A 10 2004 032 129, DE-A 10 2005 013 039, DE-A 10 2005 010 111,DE-A 10 2005 009 891, DE-A 102 11 275, EP-A 117 146, US 3,161,670, DE-A 33 13 573, WO 01/96270, DE-A 103 16 039, DE-A 10 2005 009 885, DE-A 10 2005 052 923, DE-A 10 2005 057 197, WO 03/076370, DE-A 102 45 585, DE-A 22 13 573, US 3,161,670 und dem in diesen Schriften zitierten Stand der Technik. Die DE-A 102 19 686 offenbart die entsprechende Vorgehensweise im Fall einer Herstellung von Methacrolein und/oder Methacrylsäure.

Absorptive und/oder kondensative Verfahren zur Überführung von Acrylsäure aus einem Produktgasgemisch B in die kondensierte Phase finden sich ebenfalls ausführlich im Stand der Technik beschrieben. Angeführt seien die Schriften DE-A 103 36 386, DE-A 196 31 645, DE-A 195 01 325, EP-A 982 289, DE-A 198 38 845, WO 02/076917, EP-A 695 736, EP-A 778 225, EP-A 1 041 062, EP-A 982 287, EP-A 982 288, US-A 2004/0242826, EP-A 792 867, EP-A 784 046, EP-A 695 736 (insbesondere absorptive) sowie WO 04/035514, DE-A 199 24 532, DE-A 198 14 387, DE-A 197 40 253, DE-A 197 40 252 und DE-A 196 27 847 (insbesondere kondensative).

Ferner finden sich solche absorptiven und/oder kondensativen Abtrennungen von Acrylsäure aus Produktgasgemischen B auch beschrieben in den Schriften EP-A 1 338 533, EP-A 1 388 532, DE-A 102 35 847, WO 98/01415, EP-A 1 015 411, EP-A 1 015 410, WO 99/50219, WO 00/53560, WO 02/09839, DE-A 102 35 847, WO 03/041833, DE-A 102 23 058, DE-A 102 43 625, DE-A 103 36 386, EP-A 854 129, US 4,317,926, DE-A 198 37 520, DE-A 196 06 877, DE-A 195 01 325, DE-A 102 47 240, DE-A 197 40 253, EP-A 695 736, EP-A 1 041 062, EP-A 117 146, DE-A 43 08 087, DE-A 43 35 172, DE-A 44 36 243, DE-A 103 32 758 sowie DE-A 199 24 533. Grundsätzlich kann dabei aber auch wie in der DE-A 103 36 386, DE-A 101 15 277, DE-A 196 06 877, EP-A 920 408, EP-A 1 068 174, EP-A 1 066 239, EP-A 1 066 240, WO 00/53560, WO 00/53561, DE-A 100 53 086, WO 01/96271, DE-A 10 2004 032 129, WO 04/063138, WO 04/35514, DE-A 102 43 625 und DE-A 102 35 847 beschrieben vorgegangen werden.

Handelt es sich beim zu dehydrierenden Kohlenwasserstoff für das erfindungsgemäße Verfahren um Propan, wird man selbigen vorzugsweise als Bestandteil von Roh-Propan gemäß der Lehre der DE-A 102 45 585 dem wenigstens einen Ausgangsgasstrom zuführen.

Generell wird der wenigstens eine Ausgangsgasstrom wenigstens zu 5 Vol.-% an zu dehydrierendem Kohlenwasserstoff enthalten. Häufig liegt dieser Volumenanteil bei entsprechend bezogenen Werten von ≥ 10 Vol.-%, oft ≥ 15 Vol.-% und meist ≥ 20 Vol.-% bzw. ≥ 25 Vol.-%, oder ≥ 30 Vol.-%. In der Regel liegt dieser Volumenanteil jedoch bei in gleicher Weise bezogenen Werten von ≤ 90 Vol.-%, meist ≤ 80 Vol.-% und oft ≤ 70 Vol.-%. Vorstehende Angaben gelten insbesondere im Fall von Propan als zu dehydrierendem Kohlenwasserstoff und Propylen als dehydriertem Kohlenwasserstoff. Selbstredend treffen sie aber auch dann zu, wenn iso-Butan der zu dehydrierende Kohlenwasserstoff und iso-Buten der dehydrierte Kohlenwasserstoff ist.

In bemerkenswerter Weise ist zur Durchführung einer erfindungsgemäßen konventionellen (oxidativen oder nichtoxidativen) heterogen katalysierten Dehydrierung, insbesondere für eine adiabate Betriebsweise, der Innenraum eines einfachen Schachtofens ("Schachtofenreaktor") als das wenigstens eine Katalysatorbett (z. B. das wenigstens eine Katalysatorfestbett) enthaltender erfindungsgemäßer Reaktionsraum ausreichend, der vom Ausgangsgasstrom axial und/oder radial durchströmt wird. Eine besonders bevorzugte Ausführungsform eines solchen Reaktors beschreiben Deutsches Aktenzeichen 10 2006 017 623.5 sowie 10 2006 015 235.2.

Im einfachsten Fall handelt es sich dabei zum Beispiel um einen im wesentlichen zylindrischen Behälter, dessen Innendurchmesser 0,1 bis 10 m, eventuell 0,5 bis 5 m beträgt und in welchem das wenigstens eine Katalysatorfestbett auf einer Trägervorrichtung (zum Beispiel ein Gitterrost) aufgebracht ist. Der mit Katalysator beschickte Reaktionsraum, dessen erfindungsgemäße Einhüllende im adiabaten Betrieb gegenüber ihrer Umgebung durch Aufbringen von entsprechenden Isoliermaterialien (z. B. Glaswolle) zusätzlich wärmeisoliert ist, wird dabei zweckmäßig mit dem heißen, den zu dehydrierenden Kohlenwasserstoff (z. B. das Propan) enthaltenden Ausgangsgasstrom axial durchströmt. Die Katalysatorgeometrie kann dabei sowohl kugelförmig als auch ringförmig oder strangförmig sein. Da im ebenda beschriebenen Fall der Reaktionsraum durch einen sehr kostengünstigen Apparat zu realisieren ist, sind alle Katalysatorgeometrien, die einen besonders niedrigen Druckverlust aufweisen, zu bevorzugen. Das sind vor allem Katalysatorgeometrien, die zu einem großen Hohlraumvolumen führen oder strukturiert aufgebaut sind, wie zum Beispiel Monolithe bzw. Wabenkörper. Zur Verwirklichung einer radialen Strömung des den zu dehydrierenden Kohlenwasserstoff (z. B. das Propan) enthaltenden Reaktionsgases kann der erfindungsgemäße Reaktionsraum zum Beispiel zwei konzentrisch ineinander gestellte zylindrische Gitterroste enthalten und die Katalysatorschüttung in deren Ringspalt angeordnet sein. Im adiabaten Fall wäre die ihn umgebende Einhüllende (die Mantelhülle) gegebenenfalls wiederum thermisch isoliert. Im Fall eines axial durchströmten im wesentlichen zylindrischen Schachtofens ist es für das erfindungsgemäße Verfahren im Fall einer adiabaten Betriebsweise vorteilhaft, wenn die Ausdehnung A des Reaktionsraums senkrecht zur zylindrischen Achse wenigstens das 5-fache, vorzugsweise wenigstens das 10-fache und besonders bevorzugt wenigstens das 15-fache der Schütthöhe S des wenigstens einen Katalysatorbettes in axialer Richtung beträgt. In der Regel wird das vorgenannte Verhältnis von A:S jedoch ≤ 200, üblicherweise ≤ 150 und meist ≤ 100 betragen.

Vor dem Hintergrund des bisher Gesagten umfasst vorliegende Erfindung als einen erfindungsgemäßen Gegenstand insbesondere eine einen Innenraum I (den Reaktionsraum) umschließende (materielle) Einhüllende E, mit wenigstens einer ersten Öffnung O1 zur Zufuhr wenigstens eines Gasstroms (Stoffstroms) S in den Innenraum I und wenigstens einer zweiten Öffnung 02 zur Abfuhr A (Entnahme) eines dem Innenraum zuvor über die wenigstens eine erste Öffnung O1 zugeführten Gasstroms (Stoffstroms) S aus dem Innenraum I, wobei die Einhüllende E aus einem Verbundwerkstoff gefertigt ist, der auf seiner den Innenraum I berührenden Seite B mit der Maßgabe aus Stahl B der nachfolgenden Elementzusammensetzung

| | |
|---|---|
| 18 bis 30 Gew.-% | Cr, |
| 9 bis 37 Gew.-% (bzw. unabhängig von allen anderen Gehalten bis 36 Gew.-%) | Ni, |
| 1 bis 4 Gew.-% (bzw. bis 3 Gew.-%) | Si, |
| ≥ 0 bis 4 Gew.-% | Al, |
| ≥ 0 bis 0,3 Gew.-% | N, |
| ≥ 0 bis 0,15 Gew.-% | C, |
| ≥ 0 bis 4 Gew.-% | Mn, |
| ≥ 0 bis 0,05 Gew.-% | P, |
| ≥ 0 bis 0,05 Gew.-% | S, und |
| ≥ 0 bis 0,1 Gew.-% | eines oder mehrere seltene Erdmetalle, |
| | |
| und im übrigen Fe und herstellungsbedingte Verunreinigungen, wobei die Prozentangaben jeweils auf das Gesamtgewicht bezogen sind, | |

besteht, dass der Stahl B auf seiner vom Innenraum I abgewandten Seite A entweder unmittelbar, oder über eine Zwischenschicht aus Kupfer, oder aus Nickel, oder aus Kupfer und Nickel, auf Stahl A der Elementzusammensetzung

| | |
|---|---|
| 15 bis 20 Gew.-% | Cr, |
| 6 bis 18 Gew.-% (häufig bis 16 Gew.-%) | Ni, |
| ≥ 0 bis 0,8 Gew.-% | Si, |
| ≥ 0 bis 0,8 Gew.-% | Al, |
| ≥ 0 bis 0,3 Gew.-% | N, |
| ≥ 0 bis 0,15 Gew.-% | C, |
| ≥ 0 bis 4 Gew.-% | Mo, |
| ≥ 0 bis 2 (bzw. bis 1,5) Gew.-% | Mn, |
| ≥ 0 bis 0,5 Gew.-% | Ti, |
| ≥ 0 bis 1,2 Gew.-%, bzw. bis 0,5 Gew.-% | Nb, |
| ≥ 0 bis 0,9 Gew.-% | V, |
| ≥ 0 bis 0,1 Gew.-% | B, |
| ≥ 0 bis 0,05 Gew.-% | P, |
| ≥ 0 bis 0,05 Gew.-% | S, und |
| | |
| und im übrigen Fe und herstellungsbedingte Verunreinigungen, wobei die Prozentangaben jeweils auf das Gesamtgewicht bezogen sind, | |

oder der Elementzusammensetzung

| | |
|---|---|
| 19 bis 23 Gew.-% | Cr, |
| 30 bis 35 Gew.-% | Ni, |
| ≥ 0 bis 1 Gew.-% | Co, |
| ≥ 0 bis 1 Gew.-% | Si, |
| 0,15 bis 0,7 Gew.-% | Al, |
| ≥ 0 bis 0,12 Gew.-% | C, |
| ≥ 0 bis 2,0 Gew.-% | Mn, |
| ≥ 0 bis 0,75 Gew.-% | Cu, |
| 0,15 bis 0,7 Gew.-% | Ti, |
| ≥ 0 bis 0,05 Gew.-% | P, |
| ≥ 0 bis 0,05 Gew.-% | S, und |
| | |
| im übrigen Fe und herstellungsbedingte Verunreinigungen, wobei die Prozentangaben jeweils auf das Gesamtgewicht bezogen sind, | |

aufplattiert ist.

Mit Vorteil wird auch im zuletzt genannten Stahl A der Si-Gehalt ≤ 0,8 Gew.-% betragen. Ferner beträgt der Fe-Gehalt im vorstehend zuletzt genannten Stahl A vorzugsweise mindestens 39,5 Gew.-%. Ferner ist es vorteilhaft, wenn im DIN Werkstoff 1.4876 der Schwefelgehalt ≥ 0 und ≤ 0,015 Gew.% beträgt. Der C-Gehalt desselben Werkstoffs beträgt vorteilhaft ≥ 0 und ≤ 0,1 Gew.%. Ferner beträgt der P-Gehalt desselben Werkstoffs vorteilhaft ≥ 0 bis ≤ 0,03 Gew.-%. Die Summe aus Ni und Co liegt im vorgenannten Werkstoff vorteilhaft bei 30 bis 34 Gew.-%.

Alle im Zusammenhang mit dem erfindungsgemäßen Verfahren in dieser Schrift an früherer Stelle zur Einhüllenden (E), den Stählen A, B sowie der Zwischenschicht aus Cu und/oder Ni gemachten Aussagen treffen so auch auf die den Innenraum I umschließende (materielle) Einhüllende E zu.

D.h., z. B. beträgt die Gesamtmenge der herstellungsbedingten Verunreinigungen in den Stählen A, B der Einhüllenden E erfindungsgemäß vorteilhaft ganz generell und in gleicher Weise bezogen ≤ 1 Gew.-%, vorzugsweise ≤ 0,75 Gew.-%, besonders bevorzugt ≤ 0,5 Gew.-% und ganz besonders bevorzugt ≤ 0,25 Gew.-%. In der Regel wird die Gesamtmenge der herstellungsbedingten Verunreinigungen des jeweiligen Stahls A, B jedoch ≥ 0,1 Gew.-% betragen. Erfindungsgemäß bevorzugt ist das seltene Erdmetall eines Stahls B Ce. Erfindungsgemäß bevorzugt weist ein Stahl B ≥ 0 bis 0,1 Gew.-% Ce oder Ce und eines oder mehrere von Ce verschiedene (insbesondere La, Nd und/oder Pr) seltene Erdmetalle auf.

Der Innenraum I aller Einhüllenden E eignet sich nach der Aufnahme wenigstens eines Dehydrierkatalysator enthaltenden Katalysatorbetts zur Durchführung des erfindungsgemäßen Verfahrens (insbesondere solchen der adiabaten heterogen katalysierten konventionellen nicht oxidativen oder oxidatlven Dehydrierung von Propan zu Propylen).

Erfindungsgemäß vorteilhaft beträgt das Volumen des Innenraums I (leer gerechnet) 5 m³ bis 500 m³, oft 10 m³ bis 400 m³, häufig 20 m³ bis 300 m³, vielfach 30 m³ bis 200 m³, oder 50 m³ bis 150 m³. Mögliche Individualwerte sind somit z. B. 150 m³, 160 m³, 170 m³, 180 m³, 190 m³ und 200 m³.

Selbstredend kommen für die Stähle A, B der Einhüllenden E auch alle anderen in dieser Schrift bereits genannten erfindungsgemäß verwendbaren Stähle A, B in Betracht und dies in der in dieser Schrift bereits im Vorstehenden gegebenen Bevorzugungsabstufung. Mit Vorteil ist der Stahl B auf seiner den Innenraum I berührenden Seite alonisiert, alitiert und/oder aluminiert. Das früher zur Stärke (Dicke) der Stähle A, B sowie der Zwischenschicht aus Cu und/oder Ni Gesagte hat auch im Zusammenhang mit der Einhüllenden E seine Gültigkeit.

Im besonderen betrifft vorliegende Erfindung Einhüllende E, deren Innenraum I wenigstens einen für eine heterogen katalysierte partielle Dehydrierung eines zu dehydrierenden Kohlenwasserstoffs geeigneten Katalysator (Dehydrierkatalysator) enthält. Als solche Katalysatoren kommen insbesondere alle in dieser Schrift aufgeführten Dehydrierkatalysatoren in Betracht.

Die im Innenraum der Einhüllenden E enthaltene Katalysatormenge kann 100 kg bis 500 t (Tonnen (metrische)) oder 200 kg bis 400 t, oder 300 kg bis 300 t, oder 400 kg bis 200 t, bzw. 500 kg bis 150 t, oder 1 t bis 100 t, oder 10 t bis 80 t, oder 20 t bis 60 t betragen. Nicht eingerechnet sind hierbei die Katalysatoren gegebenenfalls verdünnende ausschließlich inerte Formkörper.

Ferner betrifft vorliegende Erfindung Einhüllende E, deren Innenraum I wenigstens einen Auflage(Gitter)Rost enthält (vorzugsweise wenigstens zwei zentrisch ineinander gestellte (Gitter)Roste). Mit Vorteil weist (enthält) die Einhüllende E ein ringförmiges (hohlkreiszylinderförmiges) Segment R (bzw. einen solchen Abschnitt) auf (wobei der Ringinnenraum I_{R} einen Teil des Innenraumes I bildet).

Ist D die Hälfte der Differenz zwischen dem Außendurchmesser A (ohne äußere Wärmedämmung gerechnet) und dem Innendurchmesser des ringförmigen Segments R, so beträgt das Verhältnis V₁ von D zu A erfindungsgemäß bevorzugt 1 : 10 bis 1 : 1000, vielfach 1 : 20 bis 1 : 800, oft 1 : 30 bis 1 : 600, vielfach 1 : 40 bis 1 : 500, oder 1 : 50 bis 1 : 400, bzw. 1 : 60 bis 1 : 300, in vielen Fällen aber auch 1 : 70 bis 1 : 250 (bzw. 200), oder 1 : 80 bis 1 : 150.

Das Verhältnis V₂ aus der Höhe H (der Abstand der beiden das ringförmige Segment R begrenzenden parallelen kreisförmigen Ebenen) und A (V₂=H : A) kann sowohl > 1, oder = 1, oder < 1 betragen.

Beträgt V₂>1, beträgt es in typischer Weise 2 bis 10, häufig 4 bis 8 und oft 6. Beträgt V₂<1, beträgt es in typischer Weise 0,01 bis 0,95, häufig 0,02 bis 0,9, oft 0,03 bis 0,8, vielfach 0,05 bis 0,7, oder 0,07 bis 0,5 bzw. 0,1 bis 0,4. Mögliche Werte für V₂ sind somit auch 0,2 und 0,3.

Ist V₂>1, wird A meist ≥ 0,5 m bis 5 m, häufig 1 m bis 4 m, und zweckmäßig 2 m bis 3 m betragen.

Ist V₂<1, wird A typisch ≥ 0,5 m bis 8 m, vorzugsweise 2 m bis 7 bzw. 6,50 m und oft 2,5 m bis 6 bzw. 5 m betragen.

Ist V₂=1 betragen typische Außendurchmesser A 0,5 bis 8 m, oder 2 bis 7 bzw. 6,50 m, bzw. 2,5 m bis 6 bzw. 5 m.

Ist V₂>1, eignet sich der ringförmige Innenraum (der Ringinnenraum) des ringförmigen Segments R der Einhüllenden E insbesondere zur Gestaltung eines für das erfindungsgemäße Verfahren besonders geeigneten radial durchströmten erfindungsgemäßen Hordenreaktionsraums. Dazu enthält der ringförmige Innenraum I_{R} in zweckmäßiger Weise zwischen den Ringspalten von zentrisch ineinandergestellten Gitterrosten Katalysatorfestbetten aufgeschüttet, wobei die Ringspalte mit Vorteil abschnittsweise so übereinander angeordnet sind, dass ein sie durchströmendes Reaktionsgas nach radialem Durchtritt in einem Abschnitt in den nächsten darüber - oder darunter liegenden Abschnitt geführt wird. Die Anzahl vorgenannter Katalysatorbetten kann 1 bis 20, zweckmäßig 2 bis 8 und bevorzugt 3 bis 6 betragen. D.h., erfindungsgemäß bevorzugt sind Einhüllende E mit einem Ringinnenraum I_{R}, die abschnittsweise übereinander angeordnete Ringspalte aus jeweils zentrisch ineinandergestellten Gitterrosten aufweisen und deren V₂>1 beträgt.

Ist V₂<1, eignet sich der ringförmige Innenraum (der Ringinnenraum) des ringförmigen Segments R der Einhüllenden E insbesondere zur Gestaltung eines für das erfindungsgemäße Verfahren besonders geeigneten axial durchströmten erfindungsgemäßen Hordenreaktionsraums. Dazu enthält der ringförmige Innenraum I_{R} in zweckmäßiger Weise auf axial (d.h., längs der Ringachse, bzw. Zylinderachse) hintereinander angeordneten Gitterrosten Katalysatorbetten aufgeschüttet, die vom Reaktionsgas nacheinander durchströmt werden können. Die Anzahl vorgenannter Katalysatorbetten kann 1 bis 20, zweckmäßig 2 bis 8 und bevorzugt 3 bis 6 betragen. In der Regel sind sie äquidistant angeordnet.

D. h., erfindungsgemäß bevorzugt sind Einhüllende E mit einem Ringinnenraum I_{R}, die axial hintereinander angeordnete Gitterroste enthalten und deren V₂ < 1 beträgt. Letzteres gilt insbesondere dann, wenn H 2 m bis 4 m (vorzugsweise 2,50 bis 3,50 m, z. B. 2,70 m) und der Innendurchmesser des ringförmigen Segments R (bei einer Wanddicke von 1 bis 4 cm) 5,90 m bis 6,50 m beträgt. Zweckmäßig beträgt die Anzahl der axial hintereinander angeordneten Katalysatorbetthorden im vorgenannten Fall drei. Die Schütthöhe eines Katalysatorbetts (z. B. des Katalysators gemäß Bsp. 4 der DE-A 102 19 879) wird zweckmäßig 10 bis 60 cm (z. B. 30 cm) betragen.

Anwendungstechnisch zweckmäßig werden die beiden das ringförmige Segment R begrenzenden parallelen kreisförmigen Ebenen durch je eine Haube abgeschlossen (zur Einhüllenden E ergänzt). Prinzipiell können die Hauben die Gestalt eines flachen Bodens (Deckels) aufweisen oder auch gewölbt sein. Erfindungsgemäß bevorzugt sind auf beiden Seiten des ringförmigen Segments R gewölbte Hauben. Dabei kann die Wölbung Korbbogenform gemäß DIN 28013 oder Klöpperform gemäß DIN 28011 aufweisen. Die Wölbung der unteren Haube wird normalerweise vom Innenraum I_{R} wegweisen (nach außen gewölbt, konvex, sein). Die Wölbung der oberen Haube kann relativ zum Innenraum I_{R} sowohl konvex oder konkav sein. In anwendungstechnisch einfacher Weise sind beide Hauben relativ zum Innenraum I_{R} konvex gewölbt. Die wenigstens eine erste Öffnung O1 befindet sich in diesem Fall zweckmäßig im Zentrum der oberen Haube und die wenigstens eine zweite Öffnung 02 befindet sich in diesem Fall zweckmäßig im Zentrum der unteren Haube (in der Regel weist der erfindungsgemäß zu verwendende Verbundwerkstoff zur Fertigung der Hauben den Stahl A in einer größeren Wandstärke (Dicke) auf, als der zur Fertigung des ringförmigen Segments R verwendete entsprechende erfindungsgemäße Verbundwerkstoff (bei identischer Wahl der Stähle A, B), um dem Erfordernis der erhöhten Druckaufnahme zu genügen). Grundsätzlich können für die Reaktorhauben andere Stähle A, B aufweisende erfindungsgemäße Verbundwerkstoffe verwendet werden als für das ringförmige Segment R. Im Regelfall wird jedoch für die Reaktorhauben und das ringförmige Segment derselbe erfindungsgemäße Verbundwerkstoff eingesetzt (abgesehen von gebenenfalls unterschiedlichen Materialstärken). Vorzugsweise sind die Öffnungen O1 und O2 kreisförmig. Ihre Querschnitte werden anwendungstechnisch zweckmäßig so gewählt, dass ihr Verhältnis dem Verhältnis der über diese ins Auge gefassten zu- bzw. abströmenden Volumenströme entspricht. Bei einer Höhe H von 2 m bis 4 m (vorzugsweise 2,50 bis 3,50 m, z. B. 2,70 m) und einem Innendurchmesser des ringförmigen Segments von 5,90 bis 6,50 m kann der Durchmesser der wenigstens einen ersten Öffnung O1 z. B. typisch 1200 mm und der Durchmesser der wenigstens einen zweiten Öffnung 02 z. B. typisch 1400 mm betragen. Anwendungstechnisch vorteilhaft ist auch die Wanddicke des Stahls B im erfindungsgemäß zu verwendenden Verbundwerkstoff für die untere Haube stärker als für das ringförmige Segment R. In diesem Fall kann der ins Innere über D hinausragende Teil der Haubenwand die im ringförmigen Segment R z. B. axial hintereinander angeordneten (aufeinander gestapelten) Auflage-Gitterroste (bzw. Katalysatorbetten) tragen.

Die einzelnen ringförmigen Gitterroste können aber auch aus einheitlichen (voneinander getrennten) Kreissektoren (Gitterrostsektoren) zusammengesetzt (zusammengefügt) sein (wie der Querschnitt einer Orangenscheibe). Anwendungstechnisch bevorzugt sind Zwölftel-, oder Achtel-, oder Sechstel-, oder Viertel-, oder Drittelkreissektoren. Die Gitterrostkreissektoren können dabei ihrerseits auf offenen Gerüstkreissektoren aufliegen. Die untersten Gerüstkreissektoren können der Wölbung der unteren Haube angepasst fortgeführt und in die konvexe Wölbung der unteren Haube als die Gerüstkreissektoren tragendes Element aufgelegt werden. Die Gerüstkreissektoren von darüber befindlichen Gitterrostsektoren können dann jeweils auf diejenigen der unmittelbar darunter befindlichen Katalysatorhorde aufgelegt werden.

Prinzipiell können Einhüllende E aus dem ringförmigen Segment R und den beiden dieses abschließenden Hauben nahtlos gefertigt sein. In der Regel werden das Segment R und die Hauben jedoch separat gefertigt und müssen nachfolgend weitestgehend gasdicht (Gasleckage <10⁻⁴ mbar •l/s) miteinander verbunden werden. Im Fall der unteren Haube wird diese Verbindung mit dem ringförmigen Segment R vorzugsweise durch Anschweißen erfolgen. Im Fall der oberen Haube wird das Verbinden mit dem ringförmigen Segment R jedoch mit Vorteil durch Anflanschen vorgenommen (die Abnehmbarkeit der oberen Haube erleichtert die Befüllung mit bzw. die Entnahme von Katalysator, z. B. im Fall eines Teilkatalysatorwechsels oder im Fall eines Vollkatalysatorwechsels). Besonders bevorzugt ist eine Flanschverbindung mit Schweißlippendichtung (letztere wird erfindungsgemäß bevorzugt aus Blechen oder Halbzeug aus Edelstahl oder Nickelbasislegierungen gefertigt, wobei die Blechdicke z. B. 2 mm betragen kann).

Im Ringinnenraum befindliche Elemente (Einbauten) werden erfindungsgemäß vorteilhaft aus einem Stahl B gefertigt. Handelt es sich um gewichtstragende Elemente (z. B. die Auflage-Gitterroste), so werden diese erfindungsgemäß bevorzugt aus einem Stahl B der EN Werkstoffnummer 1.4835 bzw. aus einem Stahl B der DIN Werkstoffnummer 1.4893 gefertigt. In einer weniger bevorzugten Ausführungsform kommt für diesen Zweck aber auch Stahl der DIN Werkstoffnummer 1.4541 in Betracht.

Erfindungsgemäß bevorzugt werden die Katalysatorformkörper nicht unmittelbar auf die Gitterroste aufgebracht. Dies rührt daher, dass die Katalysatorformkörper üblicherweise eine geringere Ausdehnung als die Maschenweite des Gitterrosts aufweisen. In zweckmäßiger Weise wird daher auf einen Gitterrost zunächst eine Schicht (Höhe: 1 bis 10 cm, vorzugsweise 5 bis 10 cm) aus Steatitkugeln des Durchmessers 3 bis 10 cm, häufig 4 bzw. 5 bis 8 cm aufgegeben (bevorzugt wird Steatit C220 der Fa. CeramTec verwendet). Prinzipiell kommen auch von Kugeln verschiedene Formkörper in Betracht (z. B. Zylinder oder Ringe) die eine den vorgenannten Durchmessern entsprechende Längstausdehnung (größte direkte Verbindung zweier auf ihrer Oberfläche befindlicher Punkte) aufweisen. Als alternative Inertmaterialien zum Steatit kommen insbesondere Aluminiumoxide, Siliciumdioxid, Thoriumdioxid, Zirkondioxid, Siliciumcarbid oder Silikate wie Magnesium- und Aluminiumsilikat in Betracht. Erst auf diese Inertschicht wird anschließend die Katalysatorschüttung aufgebracht. Das Reaktionsgas durchströmt den Reaktionsraum in entsprechender Weise von oben nach unten. Nach oben wird die Katalysatorschüttung nochmals durch eine entsprechende Inertschüttung abgedeckt.

Eine den wenigstens einen Ausgangsgasstrom zuführende Rohrleitung wird mit der wenigstens einen ersten Öffnung O1 normalerweise ebenso durch Anflanschen gasdicht verbunden, wie eine den wenigstens einen Produktgasstrom abführende Rohrleitung mit der wenigstens einen zweiten Öffnung 02. Alternativ kann das Anflanschen auch durch Anschweißen ersetzt werden.

Die vorgenannten (Rohr)Leitungen werden für einen sachgerechten und sicheren Betrieb bevorzugt mit Einrichtungen zur Kompensation von Längenausdehnungseffekten, wie sie z. B. aufgrund von Temperaturänderungen auftreten können, ausgerüstet, wobei mit Vorteil Kompensatoren eingesetzt werden, die sich durch laterale Wirkungsweise auszeichnen.

Diese in der Regel mehrschichtig ausgeführten Kompensatoren können aus dem gleichen Werkstoff wie die Rohrleitung selbst gefertigt sein. Besonders vorteilhaft sind jedoch Ausführungsformen mit (allgemein: gasdurchlässigem starrem Innenrohr und gasundurchlässiger elastischer Außenhülle (gasundurchlässigem elastischem Außenrohr)) einem das zu führende Gas berührenden inneren, vorzugsweise aus einem erfindungsgemäß zu verwendenden Verbundwerkstoff gefertigten, Rohrteil, der zweckmäßig eine gasdurchlässige Dehnfuge aufweist, und einem außenliegenden, gasundurchlässigen, elastischen, gewellten Teil, der wenigstens teilweise aus einem insbesondere mechanisch und temperaturbelastbaren Werkstoff gefertigt ist, wie z. B. dem Werkstoff 1.4876 (Bezeichnung nach VdTÜV-Wb 434) bzw. 1.4958/1.4959 (Bezeichnung nach DIN 17459) bzw. INCOLOY 800 H bzw. 800 HT oder Nickelbasiswerkstoff 2.4816 (alternative Bezeichnung Alloy 600) oder 2.4851 (alternative Bezeichnung Alloy 601).

Grundsätzlich kann die Einhüllende E ebenso wie die zu- und abführenden Rohrleitungen aber auch aus Nickelbasiswerkstoff 2.4642 (alternative Bezeichnung Alloy 690 bzw. Inconel H 690) gefertigt sein. Allerdings ist dieser Werkstoff von den in dieser Schrift empfohlenen Werkstoffen der mit Abstand teuerste.

Um die Verweilzeit eines zugeführten Gases im Innenraum I einer Einhüllenden E erfindungsgemäß vorteilhaft zu minimieren, ist es zweckmäßig, das Haubeninnenvolumen mittels innerhalb der Haube angebrachter Verdrängerkörper zu verkleinern.

Alternativ zu vorgenanntem Lösungsansatz empfiehlt sich die Verwendung einer konkav (ins Innere des ringförmigen Segments R) gewölbten Haube. In diesem Fall wird man die wenigstens eine erste Öffnung O1 zur Zufuhr des wenigstens einen Gasstroms in den Innenraum mit Vorteil nicht im Zentrum der oberen Haube anbringen. Vielmehr ist es in diesem Fall zweckmäßig, um den Umfang des obersten Abschnitts des ringförmigen Segments R, zweckmäßig gleichmäßig verteilt, als solche Öffnungen Fenster mit Durchtritt zum ringförmigen Innenraum I_{R} anzubringen. Von außen wird man um die Fensterleiste herum zweckmäßig einen Ringkanal anbringen, der gasdicht an die Fensterleiste anschließt (ähnlich dem Ringkanal zur Zufuhr von Salzschmelze bei einem salzbadgekühlten Rohrbündelreaktor; vgl. Ziffer 22 in DE-A 198 06 810, Fig. 1). Den Ausgangsgasstrom wird man dann dem Ringkanal zuführen, der diesen Gasstrom über alle Fenster gleichmäßig verteilt und durch die Fenster hindurch dem Innenraum I_{R} zuführt. Im Innenraum I_{R} können mit kontrolliertem Abstand zu den Fenstern Prallplatten angebracht sein, die den dem Innenraum I_{R} zugeführten Ausgangsgasstrom in seiner Verteilung über den Querschnitt des Innenraums I_{R} zusätzlich vergleichmäßigen.

Zum Zweck der adiabaten Durchführung einer heterogen katalysierten Dehydrierung wenigstens eines zu dehydrierenden Kohlenwasserstoffs im Innenraum I (insbesondere im Innenraum I_{R}) der Einhüllenden E, wird man auf die vom Innenraum I abgewandte Seite der Einhüllenden E (einschließlich Hauben und Zufuhr- bzw. Abfuhrleitungen) Wärmedämmmaterial (= Materialien, die die Wärmeübergangszahl für den Wärmeübergang von der Einhüllenden an die Umgebung verringern) anbringen (z. B. Glaswolle, Steinwolle und/oder Keramikwolle). Bei Bedarf können vorgenannte Dämmmaterialien zusätzlich auf die dem Innenraum zugewandte Seite der Einhüllenden E angebracht werden. Dabei können diese ihrerseits eine Trennumhüllende, z. B. aus erfindungsgemäßem Stahlblech, aufweisen. Insgesamt ist die Wärmedämmung bevorzugt so beschaffen, dass, bezogen auf den einfachen Durchgang des Reaktionsgases durch den Innenraum I, ≤ 10 %, bevorzugt ≤ 5 % und besonders bevorzugt ≤ 2 % des über den Innenraum I gemittelten Wärmeinhalts während der Ausführung eines erfindungsgemäßen Verfahrens im Innenraum I an die äußere Umgebung der Einhüllenden E abfließen. Für den Fall einer adiabat geführten konventionellen oxidativen heterogen katalysierten Dehydrierung wenigstens eines zu dehydrierenden Kohlenwasserstoffs (z. B. Propan zu Propylen) wird man durch die Einhüllende E Leitungen in den Innenraum I führen, durch die z. B. zwischen den Katalysatorhorden molekularen Sauerstoff enthaltendes Gas (z. B. Luft) zugedüst werden kann, das der exothermen katalytischen Verbrennung von molekularem Wasserstoff sowie gegebenenfalls Kohlenwasserstoff dient.

Diese Leitungen werden erfindungsgemäß vorteilhaft ebenfalls aus einem Stahl B gefertigt. Bevorzugt wird dazu ein Stahl B der EN Werkstoffnummer 1.4835 bzw. ein Stahl B der DIN Werkstoffnummer 1.4893 verwendet. Alternativ können diese Leitungen auch aus Nickelbasiswerkstoff 2.4642 (alternative Bezeichnung Alloy 690 bzw. Inconel H 690) gefertigt sein.

Als alternative Ausführungsform kommen für das erfindungsgemäße Verfahren aber auch Einhüllende E in Betracht, die eine Hohlkugel umschreiben. In diesen Fällen weist die Einhüllende E ein hohlkugelzonenförmiges Segment K auf, das dadurch erdacht werden kann, dass eine Hohlkugel von zwei parallelen Ebenen geschnitten wird. Zwischen den beiden Ebenen entsteht dann das hohlkugelzonenförmige Segment K und oberhalb und unterhalb der beiden Ebenen wird je eine Hohlkugelkappe abgeschnitten, die die Funktion der das Segment K nach außen abschließenden Hauben übernehmen können. Der Innenraum I_{K} des Segments K bildet wiederum den besonders relevanten (I_{R} entsprechenden) Teil des Innenraums I. Im übrigen gilt auch im Fall einer kugelförmigen Einhüllenden E das bei der zylindrischen Einhüllenden E hinsichtlich der Unterbringung von Katalysatorhorden sowie Zufuhr- und Abfuhröffnungen O1 und O2 betreffend Gesagte alles in analoger Weise. Das gleiche gilt für die Aussagen zur Wärmedämmung. Um die Einhüllende E selbst wird man generell mit Vorteil einen Tragring anbringen, der zweckmäßig auf vier Stützen gestellt die Einhüllende E hält.

Enthält der von der Einhüllenden E gebildete Innenraum I Einbauten wie (Auflage)Gitterroste etc., so können diese für das erfindungsgemäße Verfahren grundsätzlich aber auch z. B. aus gebrannten Tonen (Aluminiumsilicaten) oder Steatit (z. B. C 220 der Fa. CeramTec), oder sonstigen Hochtemperaturkeramikmaterialien wie Aluminiumoxiden, Siliciumdioxid, Thoriumdioxid, Zirkondioxid, Siliciumcarbid oder sonstigen Silicaten wie Magnesiumsilicat gefertigt sein.

Im übrigen kann die Einhüllende E einschließlich des von ihr gebildeten Innenraums I und dessen Einbauten wie in den Schriften Deutsches Aktenzeichen 10 2006 017 623.5 sowie 10 2006 015 235.2 gestaltet werden. Die Höhe der Katalysatorschüttung auf der einzelnen Horde kann dabei z. B. 40 cm betragen.

Wird zur Fertigung einer Einhüllenden E erfindungsgemäß zu verwendender Verbundwerkstoff verschweißt, so erfolgt eine solche Verschweißung erfindungsgemäß bevorzugt artgleich, d.h., Basiswerkstoff und Schweißzusatzwerkstoff sind weitgehend identisch. Prinzipiell kann zu diesem Zweck aber auch tiefer schmelzender Stahl verwendet werden, der in seiner Elementzusammensetzung erfindungsgemäß zu verwendendem Stahl möglichst nahe kommt. Im Regelfall wird man im Fall des erfindungsgemäßen Verbundwerkstoffs mit der Verschweißung des Stahls A beginnen (der Druck tragende Teil). Nachfolgend wird man den Stahl B verschweißen.

Bei Anwendung besonderer Sorgfalt kann abschließend auf der den Reaktionsraum berührenden Seite auf die zuvor erzeugte Schweißnaht zusätzlich ein Abdeckstreifen aus Stahl B aufgeschweißt werden. Grundsätzlich kann die Verschweißung wie in der DIN EN 1011-5, Ausgabe 2003-10 (insbesondere Teil 5) oder wie in Böhler Thyssen Schweisstechnik Deutschland GmbH-09-2005 (insbesondere Seite 464 folgende) beschrieben vorgenommen werden. Vorzugsweise erfolgt das Schweißen unter Inertgasatmosphäre (besonders bevorzugt unter Argon und/oder He).

Geeignete Schmelzschweißverfahren sind das Lichtbogenschweißen mit Stabelektroden sowie das Schutzgasschweißen (vor allem nach dem WIG-Verfahren). Als Schutzgas wird bevorzugt ein Argon, Helium und/oder Stickstoff haltiges Schutzgas verwendet. Als Schweißelektroden können die Schweißstäbe Thermanit D (W 22 12 H) oder Thermanit C (W 25 20 Mn) oder Sandvik 22 12 HT verwendet werden.

Abschließend sei nochmals festgehalten, dass die Stähle A dem erfindungsgemäßen Verbundwerkstoff nach außen die geringe Langzeitversprödung und die Stähle B nach innen minimales Aufkohlen und minimales Metal Dusting sowie minimale unerwünschte katalytische Wirkung verleihen.

### Beispiele und Vergleichsbeispiele

### Versuchsbeschreibung

1. Gestaltung der Reaktionsrohre
   Die Geometrie der Reaktionsrohre ist:
   0,55 m lang
   21,34 bis 22 mm Außendurchmesser (A)
   2 bis 2,77 mm Wanddicke (W)

   Das jeweilige Reaktionsrohr ist auf seiner gesamten Länge mit inerten Kugeln aus Steatit C 220 der Fa. CeramTec gefüllt. Der Kugeldurchmesser beträgt im wesentlichen gleichförmig verteilt 1,5 mm bis 2,5 mm.
2. Als Werkstoff für das Reaktionsrohr werden 7 unterschiedliche Materialien verwendet.
   Material 1 (M1): Edelstahl der DIN Werkstoffnummer 1.4841 (A = 22 mm, W = 2 mm),
   Material 2 (M2): Edelstahl der DIN Werkstoffnummer 1.4541 (A = 22 mm, W = 2 mm),
   Material 3 (M3): Edelstahl der DIN Werkstoffnummer 1.4910 (A = 22 mm, W = 2 mm),
   Material 4 (M4): Edelstahl der DIN Werkstoffnummer 1.4893 mit nachfolgender Zusammensetzung (A = 21,34 mm, W = 2,77 mm):
      20,87 Gew.-% Cr,
      10,78 Gew.-% Ni,
      1,54 Gew.-% Si,
      0,161 Gew.-% N,
      0,082 Gew.-% C,
      0,75 Gew.-% Mn,
      0,02 Gew.-% P,
      0,0026 Gew.-% S,
      0,05 Gew.-% Ce, und
      im übrigen Fe und herstellungsbedingte Verunreinigungen, wobei die Prozentangaben jeweils auf das Gesamtgewicht bezogen sind.
   Material 5 (M5): Edelstahl der DIN Werkstoffnummer 1.4841 (Wanddicke: 1 mm), unmittelbar auf Edelstahl der DIN Werkstoffnummer 1.4910 (Wanddicke: 1 mm) aufgebracht (A = 22 mm).
   Material 6 (M6): Nickelbasiswerkstoff 2.4642 (A = 22 mm, W = 2 mm) mit der Elementzusammensetzung:
      8,70 Gew.-% Fe,
      27,85 Gew.-% Cr,
      0,02 Gew.-% C,
      0,15 Gew.-% Si,
      0,2 Gew.-% Mn,
      sowie im übrigen Ni und herstellungsbedingte Verunreinigungen, wobei die Prozentangaben jeweils auf das Gesamtgewicht bezogen sind.
   Material 7 (M7): Edelstahl der DIN Werkstoffnummer 1.4876 (A = 22 mm, W = 2 mm)
3. Die verschiedenen Reaktionsrohre werden jeweils mit nachfolgendem Ausgangsgasstrom beschickt, wie er in seiner Zusammensetzung für eine erfindungsgemäße heterogen katalysierte Dehydrierung von Propan zu Propylen typisch ist:
   34,4 Vol.-% Propan,
   55,6 Vol.-% Stickstoff,
   3,2 Vol.-% Sauerstoff, und
   6,8 Vol.-% Wasserdampf.
4. Das Reaktionsrohr war jeweils in einem Strahlungsofen untergebracht (elektrisch beheizter keramischer Körper, mit hohlzylindrischer Führung zur Aufnahme des Reaktionsrohres mit einem Spaltabstand von 0,13 bis 0,15 cm zur Reaktionsrohraußenwand).
5. Das jeweilige Reaktionsrohr wird wie beschrieben (P (Reaktorausgangsdruck) = 1 atm) vom Ausgangsgasstrom (dieser weist jeweils eine Eingangstemperatur von 200°C auf) durchströmt. Gleichzeitig wird die Temperatur T^{A} der Außenwand des Reaktionsrohres so erhöht, dass sich die maximale Temperatur T^{M} im Reaktionsrohr im wesentlichen linear mit einem Gradienten von 10°C/h von 400°C auf 700°C erhöht (dies simuliert die Kompensation eines im kontinuierlichen Betrieb deaktivierenden Katalysatorbettes durch Erhöhung der Reaktionstemperatur).

Anschließend wird die Regenerierung eines Dehydrierkatalysatorbetts simuliert. Dazu wird das Reaktionsrohr zunächst mit 420 Nml/min N₂ der Eingangstemperatur 200°C durchströmt und dabei die Temperatur T^{M} auf 700°C gehalten.

Unter Beibehalt der Temperatur T^{M} = 700°C wird folgendes Gasdurchströmprogramm durchlaufen:
- während 60 min Magerluft (Gemisch aus Luft (85,4 Nml/min) und N₂ (341,6 Nml/min));

| | |
|---|---|
| dann - | während 60 min 417 Nml/min Luft; |
| dann - | während 15 min 417 Nml/min N₂; |
| dann - | während 60 min 168 Nml/min H₂. |

Danach wird das jeweilige Reaktionsrohr, vom jeweiligen Feed durchströmt, mit einem T^{M}- Gradienten von 10°C/h im wesentlichen linear von T^{M} = 700°C auf T^{M} = 400°C gebracht.

Ab Erreichen der Temperatur T^{M} = 400°C wird die Temperatur T^{A} der Außenwand des Reaktionsrohres wiederum so erhöht, dass sich die maximale Temperatur T^{M} im Reaktionsrohr im wesentlichen linear mit einem Gradienten von 10°C/h von 400°C auf 700°C erhöht. Anschließend wird wieder, wie vorstehend beschrieben, die Regenerierung eines Dehydrierkatalysatorbetts simuliert usw.

Nach einer Gesamtbetriebsdauer von 1000 h wird das jeweilige Reaktionsrohr auf Aufkohlen, Metal Dusting, Langzeitversprödung (Vergleich der Kerbschlagzähigkeit KZ vor Beginn des jeweiligen Versuchs (KZ_{V}) und am Ende (KZ_{E}) der 1000stündigen Versuchsdurchführung) untersucht (die Probe befindet sich dazu jeweils bei Raumtemperatur).

Nachfolgende Tabelle zeigt die resultierenden Ergebnisse.

| | Aufkohlen | Metal Dusting | Langzeitversprödung | KZ_{V} [J] | KZ_{E} [J] |
|---|---|---|---|---|---|
| M1 | - | - | ++ | > 40 | <5 |
| M2 | ++ | + | - | >> 40 | >> 40 |
| M3 | ++ | + | - | >> 40 | >> 40 |
| M4 | 0 | - | + | > 40 | <10 |
| M5 | - | - | - | >> 40 | >>40 |
| M6 | - | - | - | >> 40 | >> 40 |
| M7 | ++ | + | - | >> 40 | >> 40 |

Dabei gelten folgende Bedeutungen:
-: nicht auftretend
0: mäßig auftretend
+: stark auftretend
++: sehr stark auftretend

Bei den Werkstoffen M2, M3, M5, M6 und M7 sind die Werte für KZ_{V} und KZ_{E} innerhalb der Maßgenauigkeit im wesentlichen ununterscheidbar.

Zusätzlich wird am Ausgang des jeweiligen Reaktionsrohres die Menge des beim Durchgang durch das Reaktionsrohr umgesetzten Propans ermittelt (und zwar jeweils bei den Temperaturen T^{A} = 500°C, 600°C, 650°C und 700°C.

Die niedrigsten Umsatzwerte werden dabei in den Fällen M1, M5 und M6 ermittelt. Mit T^{A} nehmen auch die Umsätze zu.

Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hier in spezifisch beschrieben, ausgeführt werden kann.

## Patentansprüche

1. Verfahren der kontinuierlichen heterogen katalysierten partiellen Dehydrierung wenigstens eines zu dehydrierenden Kohlenwasserstoffs in der Gasphase, umfassend eine Verfahrensweise, bei der man einem Reaktionsraum, der von einer den Reaktionsraum berührenden Einhüllenden umschlossen ist, die wenigstens eine erste Öffnung zur Zufuhr wenigstens eines Ausgangsgasstroms in den Reaktionsraum und wenigstens eine zweite Öffnung zur Entnahme wenigstens eines Produktgasstroms aus dem Reaktionsraum aufweist,
- wenigstens einen, wenigstens einen zu dehydrierenden Kohlenwasserstoff enthaltenden, Ausgangsgasstrom kontinuierlich zuführt,
- im Reaktionsraum den wenigstens einen zu dehydrierenden Kohlenwasserstoff durch wenigstens ein im Reaktionsraum befindliches Katalysatorbett führt, und unter Erzeugung eines den wenigstens einen dehydrierten Kohlenwasserstoff, nicht umgesetzten zu dehydrierenden Kohlenwasserstoff sowie molekularen Wasserstoff und/oder Wasserdampf enthaltenden Produktgases oxidativ oder nicht oxidativ zu wenigstens einem dehydrierten Kohlenwasserstoff partiell dehydriert, und
- dem Reaktionsraum wenigstens einen Produktgasstrom kontinuierlich entnimmt,
**dadurch gekennzeichnet, dass**
die Einhüllende aus einem Verbundwerkstoff gefertigt ist, der auf seiner den Reaktionsraum berührenden Seite B mit der Maßgabe aus Stahl B der nachfolgenden Elementzusammensetzung
| | |
|---|---|
| 18 bis 30 Gew.-% | Cr, |
| 9 bis 37 Gew.-% | Ni, |
| 1 bis 4 Gew.-% | Si, |
| ≥ 0 bis 4 Gew.-% | Al, |
| ≥ 0 bis 0,3 Gew.-% | N, |
| ≥ 0 bis 0,15 Gew.-% | C, |
| ≥ 0 bis 4 Gew.-% | Mn, |
| ≥ 0 bis 0,05 Gew.-% | P, |
| ≥ 0 bis 0,05 Gew.-% | S, und |
| ≥ 0 bis 0,1 Gew.-% | eines oder mehrere seltene Erdmetalle, und |
| im übrigen Fe und herstellungsbedingte Verunreinigungen, wobei die Prozentangaben jeweils auf das Gesamtgewicht bezogen sind, | |
besteht, dass der Stahl B auf seiner vom Reaktionsraum abgewandten Seite A entweder unmittelbar, oder über eine Zwischenschicht aus Kupfer, oder aus Nickel, oder aus Kupfer und Nickel, auf Stahl A der Elementzusammensetzung
| | |
|---|---|
| 15 bis 20 Gew.-% | Cr, |
| 6 bis 18 Gew.-% | Ni, |
| ≥ 0 bis 0,8 Gew.-% | Si, |
| ≥ 0 bis 0,8 Gew.-% | Al, |
| ≥ 0 bis 0,3 Gew.-% | N, |
| ≥ 0 bis 0,15 Gew.-% | C, |
| ≥ 0 bis 4 Gew.-% | Mo, |
| ≥ 0 bis 2 Gew.-% | Mn, |
| ≥ 0 bis 0,8 Gew.-% | Ti, |
| ≥ 0 bis 1,2 Gew.-% | Nb, |
| ≥ 0 bis 0,9 Gew.-% | V, |
| ≥ 0 bis 0,1 Gew.-% | B, |
| ≥ 0 bis 0,05 Gew.-% | P, |
| ≥ 0 bis 0,05 Gew.-% | S, und |
| im übrigen Fe und herstellungsbedingte Verunreinigungen, wobei die Prozentangaben jeweils auf das Gesamtgewicht bezogen sind, | |
oder der Elementzusammensetzung
| | |
|---|---|
| 19 bis 23 Gew.-% | Cr, |
| 30 bis 35 Gew.-% | Ni, |
| ≥ 0 bis 1 Gew.-% | Co, |
| ≥ 0 bis 1 Gew.-% | Si, |
| 0,15 bis 0,7 Gew.-% | Al, |
| ≥ 0 bis 0,12 Gew.-% | C, |
| ≥ 0 bis 2,0 Gew.-% | Mn, |
| ≥ 0 bis 0,75 Gew.-% | Cu, |
| 0,15 bis 0,7 Gew.-% | Ti, |
| ≥ 0 bis ≤ 0,1 Gew.-% | Nb, |
| ≥ 0 bis 0,05 Gew.-% | P, |
| ≥ 0 bis 0,05 Gew.-% | S, und |
| | |
| im übrigen Fe und herstellungsbedingte Verunreinigungen, wobei die Prozentangaben jeweils auf das Gesamtgewicht bezogen sind, | |
aufplattiert ist.

2. Verfahren nach Anspruch 1, **dadurch gekennzeichnet, dass** sich die Wärmeausdehnungskoeffizienten von Stahl A und Stahl B des Verbundwerkstoffs bei 500°C und 1 atm um ≤ 2 · 10⁻⁶ m/m·K unterscheiden.

3. Verfahren nach Anspruch 1, **dadurch gekennzeichnet, dass** sich die Wärmeausdehnungskoeffizienten von Stahl A und Stahl B des Verbundwerkstoffs bei 500°C und 1 atm um ≤ 1,75 · 10⁻⁶ m/m·K unterscheiden.

4. Verfahren nach Anspruch 1, **dadurch gekennzeichnet, dass** sich die Wärmeausdehnungskoeffizienten von Stahl A und Stahl B des Verbundwerkstoffs bei 500°C und 1 atm um ≤ 1,50 · 10⁻⁶ m/m·K unterscheiden.

5. Verfahren nach Anspruch 1, **dadurch gekennzeichnet, dass** sich die Wärmeausdehnungskoeffizienten von Stahl A und Stahl B des Verbundwerkstoffs bei 500°C und 1 atm um ≤ 1,25 · 10⁻⁶ m/m·K unterscheiden.

6. Verfahren nach Anspruch 1, **dadurch gekennzeichnet, dass** sich die Wärmeausdehnungskoeffizienten von Stahl A und Stahl B des Verbundwerkstoffs bei 500°C und 1 atm um ≤ 1,0 · 10⁻⁶ m/m·K unterscheiden.

7. Verfahren nach einem der Ansprüche 1 bis 6, **dadurch gekennzeichnet, dass** die Dicke von Stahl B des Verbundwerkstoffs 0,2 bis 25 mm beträgt.

8. Verfahren nach einem der Ansprüche 1 bis 6, **dadurch gekennzeichnet, dass** die Dicke von Stahl B des Verbundwerkstoffs 1 bis 10 mm beträgt.

9. Verfahren nach einem der Ansprüche 1 bis 6, **dadurch gekennzeichnet, dass** die Dicke von Stahl B des Verbundwerkstoffs 2 bis 8 mm beträgt.

10. Verfahren nach einem der Ansprüche 1 bis 9, **dadurch gekennzeichnet, dass** die Dicke von Stahl A des Verbundwerkstoffs 10 bis 150 mm beträgt.

11. Verfahren nach einem der Ansprüche 1 bis 9, **dadurch gekennzeichnet, dass** die Dicke von Stahl A des Verbundwerkstoffs 20 bis 100 mm beträgt.

12. Verfahren nach einem der Ansprüche 1 bis 9, **dadurch gekennzeichnet, dass** die Dicke von Stahl A des Verbundwerkstoffs 60 bis 100 mm beträgt.

13. Verfahren nach einem der Ansprüche 1 bis 9, **dadurch gekennzeichnet, dass** die Dicke von Stahl A des Verbundwerkstoffs 20 bis 50 mm beträgt.

14. Verfahren nach einem der Ansprüche 1 bis 13, **dadurch gekennzeichnet, dass** der Stahl B des Verbundwerkstoffs unmittelbar auf den Stahl A aufplattiert ist.

15. Verfahren nach einem der Ansprüche 1 bis 13, **dadurch gekennzeichnet, dass** der Stahl B des Verbundwerkstoffs über eine Zwischenschicht aus Kupfer, oder aus Nickel, oder aus Kupfer und Nickel auf den Stahl A aufplattiert ist und die Dicke der Zwischenschicht ≥ 0,1 mm und ≤ 3 mm beträgt.

16. Verfahren nach Anspruch 15, **dadurch gekennzeichnet, dass** die Dicke der Zwischenschicht ≥ 0,2 mm und ≤ 2 mm beträgt.

17. Verfahren nach Anspruch 15, **dadurch gekennzeichnet, dass** die Dicke der Zwischenschicht ≥ 0,3 mm und ≤ 1 mm beträgt.

18. Verfahren nach einem der Ansprüche 1 bis 17, **dadurch gekennzeichnet, dass** der Gehalt des Stahls A vom Verbundwerkstoff an Si ≤ 0,6 Gew.-% beträgt.

19. Verfahren nach einem der Ansprüche 1 bis 17, **dadurch gekennzeichnet, dass** der Gehalt des Stahls A vom Verbundwerkstoff an Si ≤ 0,4 Gew.-% beträgt.

20. Verfahren nach einem der Ansprüche 1 bis 17, **dadurch gekennzeichnet, dass** der Gehalt des Stahls A vom Verbundwerkstoff an Si ≤ 0,1 Gew.-% beträgt.

21. Verfahren nach einem der Ansprüche 1 bis 20, **dadurch gekennzeichnet, dass** der Stahl B des Verbundwerkstoffs die Elementzusammensetzung
| | |
|---|---|
| 24 bis 26 Gew.-% | Cr, |
| 19 bis 22 Gew.-% | Ni, |
| 1,5 bis 2,5 Gew.-% | Si, |
| ≥ 0 bis 0,11 Gew.-% | N, |
| ≥ 0 bis 0,2 Gew.-% | C, |
| ≥ 0 bis 2 Gew.-% | Mn, |
| ≥ 0 bis 0,045 Gew.-% | P, |
| ≥ 0 bis 0,015 Gew.-% | S, und |
| im übrigen Fe und herstellungsbedingte Verunreinigungen, wobei die Prozentangaben jeweils auf das Gesamtgewicht bezogen sind, | |
aufweist.

22. Verfahren nach einem der Ansprüche 1 bis 21, **dadurch gekennzeichnet, dass** der Stahl A des Verbundwerkstoffs die Elementzusammensetzung
| | |
|---|---|
| 16 bis 18 Gew.-% | Cr, |
| 12 bis 14 Gew.-% | Ni, |
| ≥ 0 bis 0,8 Gew.-% | Si, |
| 0,10 bis 0,18 Gew.-% | N, |
| ≥ 0 bis 0,1 Gew.-% | C, |
| ≥ 2 bis 3 Gew.-% | Mo, |
| ≥ 0 bis 2 Gew.-% | Mn, |
| 0,0015 bis 0,0050 Gew.-% | B, |
| ≥ 0 bis 0,05 Gew.-% | P, |
| ≥ 0 bis 0,05 Gew.-% | S, und |
| | |
| im übrigen Fe und herstellungsbedingte Verunreinigungen, wobei die Prozentangaben jeweils auf das Gesamtgewicht bezogen sind, | |
aufweist.

23. Verfahren nach einem der Ansprüche 1 bis 22, **dadurch gekennzeichnet, dass** das Aufplattieren durch Sprengplattieren erfolgt ist.

24. Verfahren nach einem der Ansprüche 1 bis 23, **dadurch gekennzeichnet, dass** der zu dehydrierende Kohlenwasserstoff ein C₂- bis C₁₆-Alkan ist.

25. Verfahren nach einem der Ansprüche 1 bis 24, **dadurch gekennzeichnet, dass** der zu dehydrierende Kohlenwasserstoff wenigstens ein Kohlenwasserstoff aus der Gruppe umfassend Ethan, Propan, n-Butan, iso-Butan, n-Pentan, isoPentan, n-Hexan, n-Heptan, n-Octan, n-Nonan, n-Decan, n-Undecan, n-Dodecan, n-Tridecan, n-Tetradecan, n-Pentadecan und n-Hexadecan ist.

26. Verfahren nach einem der Ansprüche 1 bis 25, **dadurch gekennzeichnet, dass** der zu dehydrierende Kohlenwasserstoff Ethan, Propan, n-Butan und/oder iso-Butan ist.

27. Verfahren nach einem der Ansprüche 1 bis 26, **dadurch gekennzeichnet, dass** der zu dehydrierende Kohlenwasserstoff Propan und der dehydrierte Kohlenwasserstoff Propylen ist.

28. Verfahren nach einem der Ansprüche 1 bis 27, **dadurch gekennzeichnet, dass** der Ausgangsgasstrom Wasserdampf enthält.

29. Verfahren nach einem der Ansprüche 1 bis 28, **dadurch gekennzeichnet, dass** der Ausgangsgasstrom molekularen Sauerstoff enthält.

30. Verfahren nach einem der Ansprüche 1 bis 29, **dadurch gekennzeichnet, dass** das Katalysatorbett ein Katalysatorfestbett ist.

31. Verfahren nach einem der Ansprüche 1 bis 30, **dadurch gekennzeichnet, dass** die heterogen katalysierte partielle Dehydrierung eine nicht oxidative Dehydrierung ist.

32. Verfahren nach einem der Ansprüche 1 bis 30, **dadurch gekennzeichnet, dass** die heterogen katalysierte partielle Dehydrierung eine oxidative Dehydrierung ist.

33. Verfahren nach einem der Ansprüche 1 bis 30, **dadurch gekennzeichnet, dass** die heterogen katalysierte partielle Dehydrierung eine heterogen katalysierte Oxidehydrierung ist.

34. Verfahren nach einem der Ansprüche 1 bis 30, **dadurch gekennzeichnet, dass** die heterogen katalysierte partielle Dehydrierung eine adiabat ausgeführte konventionelle heterogen katalysierte Dehydrierung ist.

35. Verfahren nach einem der Ansprüche 1 bis 30, **dadurch gekennzeichnet, dass** die heterogen katalysierte partielle Dehydrierung eine konventionelle heterogen katalysierte partielle Dehydrierung und der Reaktionsraum ein Hordenreaktionsraum ist.

36. Verfahren nach Anspruch 35, **dadurch gekennzeichnet, dass** die konventionelle heterogen katalysierte partielle Dehydrierung eine oxidative konventionelle heterogen katalysierte partielle Dehydrierung ist.

37. Verfahren nach Anspruch 36, **dadurch gekennzeichnet, dass** es in einem adiabat ausgestalteten Reaktionsraum ausgeführt wird.

38. Verfahren nach einem der Ansprüche 1 bis 37, **dadurch gekennzeichnet, dass** der dem Reaktionsraum zugeführte Ausgangsgasstrom enthält:
Propylen ≥ 0 bis 20 Vol.-%,
Acrolein ≥ 0 bis 1 Vol.-%,
Acrylsäure ≥ 0 bis 0,25 Vol.-%,
COₓ ≥ 0 bis 20 Vol.-%,
Propan 5 bis 50 Vol.-%,
Stickstoff 20 bis 80 Vol.-%,
Sauerstoff ≥ 0 bis 5 Vol.-%,
H₂O ≥ 0 bis 20 Vol.-% und
H₂ ≥ 0 bis 10 Vol.-%.

39. Verfahren nach einem der Ansprüche 1 bis 38, **dadurch gekennzeichnet, dass** man den aus dem Reaktionsraum entnommenen Produktgasstrom als solchen oder nach Abtrennung wenigstens einer Teilmenge seiner vom dehydrierten Kohlenwasserstoff und vom zu dehydrierenden Kohlenwasserstoff verschiedenen Bestandteile zur Beschickung wenigstens eines Oxidationsreaktors verwendet und in diesem den darin enthaltenen dehydrierten Kohlenwasserstoff einer selektiven heterogen katalysierten partiellen Gasphasenoxidation mit molekularem Sauerstoff zu einem das Partialoxidationsprodukt enthaltenden Produktgasgemisch B unterwirft.

40. Verfahren nach Anspruch 39, **dadurch gekennzeichnet, dass** der zu dehydrierende Kohlenwasserstoff Propan, der dehydrierte Kohlenwasserstoff Propylen und das Partialoxidationsprodukt Acrolein, Acrylsäure oder deren Gemisch ist.

41. Verfahren nach Anspruch 39, **dadurch gekennzeichnet, dass** man in einer Trennzone B der selektiven heterogen katalysierten partiellen Gasphasenoxidation nachfolgend aus dem Produktgasgemisch B Partialoxidationsprodukt abtrennt und von dem dabei verbleibenden, nicht umgesetzten zu dehydrierenden Kohlenwasserstoff, molekularen Sauerstoff sowie gegebenenfalls nicht umgesetzten dehydrierten Kohlenwasserstoff enthaltenden Restgas wenigstens eine nicht umgesetzten zu dehydrierenden Kohlenwasserstoff enthaltende Teilmenge als Partialoxidationskreisgas in das Verfahren der heterogen katalysierten partiellen Dehydrierung des zu dehydrierenden Kohlenwasserstoffs rückführt.

42. Verfahren nach Anspruch 41, **dadurch gekennzeichnet, dass** man das Partialoxidationsprodukt in der Trennzone B vom Produktgasgemisch B durch Überführen in die kondensierte Phase abtrennt.

43. Verfahren nach Anspruch 42, **dadurch gekennzeichnet, dass** das Partialoxidationsprodukt Acrylsäure ist und die Überführung in die kondensierte Phase durch absorptive und/oder kondensative Maßnahmen erfolgt.

44. Verfahren nach Anspruch 43, **dadurch gekennzeichnet, dass** unter Anwendung wenigstens eines thermischen Trennverfahrens eine Abtrennung der Acrylsäure aus der kondensierten Phase vorgenommen wird.

45. Verfahren nach Anspruch 44, **dadurch gekennzeichnet, dass** das wenigstens eine thermische Trennverfahren eine kristallisative Abtrennung von Acrylsäure aus flüssiger Phase umfasst.

46. Verfahren nach Anspruch 45, **dadurch gekennzeichnet, dass** die kristallisative Abtrennung eine Suspensionskristallisation ist.

47. Verfahren nach Anspruch 44, **dadurch gekennzeichnet, dass** sich an die Abtrennung der Acrylsäure ein Verfahren der radikalischen Polymerisation anschließt, in welchem abgetrennte Acrylsäure zur Herstellung von Polymerisaten radikalisch einpolymerisiert wird.

48. Verfahren nach Anspruch 44, **dadurch gekennzeichnet, dass** sich an die Abtrennung der Acrylsäure ein Verfahren zur Herstellung von Acrylsäureestern anschließt, in welchem abgetrennte Acrylsäure mit einem Alkohol verestert wird.

49. Verfahren nach Anspruch 48, **dadurch gekennzeichnet, dass** sich an das Verfahren zur Herstellung eines Acrylsäureesters ein Verfahren der radikalischen Polymerisation anschließt, in welchem so hergestellter Acrylsäureester einpolymerisiert wird.

50. Eine einen Innenraum I umschließende Einhüllende E mit wenigstens einer ersten Öffnung O1 zur Zufuhr wenigstens eines Gasstroms S in den Innenraum I und wenigstens einer zweiten Öffnung 02 zur Entnahme eines dem Innenraum I zuvor über die wenigstens eine erste Öffnung O1 zugeführten Gasstroms S aus dem Innenraum I, wobei die Einhüllende E aus einem Verbundwerkstoff gefertigt ist, der auf seiner den Reaktionsraum berührenden Seite B mit der Maßgabe aus Stahl B der nachfolgenden Elementzusammensetzung
| | |
|---|---|
| 18 bis 30 Gew.-% | Cr, |
| 9 bis 37 Gew.-% | Ni, |
| 1 bis 4 Gew.-% | Si, |
| ≥ 0 bis 4 Gew.-% | Al, |
| ≥ 0 bis 0,3 Gew.-% | N, |
| ≥ 0 bis 0,15 Gew.-% | C, |
| ≥ 0 bis 4 Gew.-% | Mn, |
| ≥ 0 bis 0,05 Gew.-% | P, |
| ≥ 0 bis 0,05 Gew.-% | S, und |
| ≥ 0 bis 0,1 Gew.-% | eines oder mehrere seltene Erdmetalle, und |
| | |
| im übrigen Fe und herstellungsbedingte Verunreinigungen, wobei die Prozentangaben jeweils auf das Gesamtgewicht bezogen sind, | |
besteht, dass der Stahl B auf seiner vom Reaktionsraum abgewandten Seite A entweder unmittelbar, oder über eine Zwischenschicht aus Kupfer, oder aus Nickel, oder aus Kupfer und Nickel, auf Stahl A der Elementzusammensetzung
| | |
|---|---|
| 15 bis 20 Gew.-% | Cr, |
| 6 bis 18 Gew.-% | Ni, |
| ≥ 0 bis 0,8 Gew.-% | Si, |
| ≥ 0 bis 0,8 Gew.-% | Al, |
| ≥ 0 bis 0,3 Gew.-% | N, |
| ≥ 0 bis 0,15 Gew.-% | C, |
| ≥ 0 bis 4 Gew.-% | Mo, |
| ≥ 0 bis 2 Gew.-% | Mn, |
| ≥ 0 bis 0,8 Gew.-% | Ti, |
| ≥ 0 bis 1,2 Gew.-% | Nb, |
| ≥ 0 bis 0,9 Gew.-% | V, |
| ≥ 0 bis 0,1 Gew.-% | B, |
| ≥ 0 bis 0,05 Gew.-% | P, |
| ≥ 0 bis 0,05 Gew.-% | S, und |
| im übrigen Fe und herstellungsbedingte Verunreinigungen, wobei die Prozentangaben jeweils auf das Gesamtgewicht bezogen sind, | |
oder der Elementzusammensetzung
| | |
|---|---|
| 19 bis 23 Gew.-% | Cr, |
| 30 bis 35 Gew.-% | Ni, |
| ≥ 0 bis 1 Gew.-% | Co, |
| ≥ 0 bis 1 Gew.-% | Si, |
| 0,15 bis 0,7 Gew.-% | Al, |
| ≥ 0 bis 0,12 Gew.-% | C, |
| ≥ 0 bis 2,0 Gew.-% | Mn, |
| ≥ 0 bis 0,75 Gew.-% | Cu, |
| 0,15 bis 0,7 Gew.-% | Ti, |
| ≥ 0 bis 0,05 Gew.-% | P, |
| ≥ 0 bis 0,05 Gew.-% | S, und |
| | |
| im übrigen Fe und herstellungsbedingte Verunreinigungen, wobei die Prozentangaben jeweils auf das Gesamtgewicht bezogen sind, | |
aufplattiert ist.

51. Eine Einhüllende E nach Anspruch 50, deren Innenraum I wenigstens einen Dehydrierkatalysator enthält.

52. Eine Einhüllende E nach Anspruch 50 oder 51, deren Innenraum I wenigstens einen Auflagerost enthält.

53. Eine Einhüllende E nach einem der Ansprüche 50 bis 52, die ein ringförmiges Segment R aufweist.

54. Eine Einhüllende E nach Anspruch 53, wobei das Verhältnis V₁=D : A, gebildet mit der Hälfte D der Differenz zwischen dem Außendurchmesser A und dem Innendurchmesser des ringförmigen Segments R, 1 : 10 bis 1 : 1000 beträgt.

55. Eine Einhüllende E nach Anspruch 54, wobei V₁ 1 : 40 bis 1 : 500 beträgt.

56. Eine Einhüllende E nach einem der Ansprüche 53 bis 55, wobei das Verhältnis V₂=H : A, gebildet aus dem Abstand H der beiden das ringförmige Segment R begrenzenden parallelen kreisförmigen Ebenen und dem Außendurchmesser A des ringförmigen Segments > 1 beträgt.

57. Eine Einhüllende E nach einem der Ansprüche 53 bis 55, wobei das Verhältnis V₂=H : A, gebildet aus dem Abstand H der beiden das ringförmige Segment R begrenzenden parallelen kreisförmigen Ebenen und dem Außendurchmesser des ringförmigen Segments ≤ 1 beträgt.

58. Eine Einhüllende E nach einem der Ansprüche 50 bis 52, die ein hohlkugelzonenförmiges Segment K aufweist.

59. Eine Einhüllende E nach einem der Ansprüche 50 bis 58, die auf ihrer vom Innenraum I abgewandten Seite Wärmedämmmaterial aufgebracht aufweist.

60. Verfahren der heterogen katalysierten partiellen Dehydrierung eines Kohlenwasserstoffs, **dadurch gekennzeichnet, dass** man es im Innenraum I einer Einhüllenden E gemäß einem der Ansprüche 50 bis 59 durchführt.

61. Verwendung einer Einhüllenden E gemäß einem der Ansprüche 50 bis 59 oder gemäß einem der Ansprüche 63, 65, 66 zur Durchführung einer heterogen katalysierten partiellen Dehydrierung eines Kohlenwasserstoffs.

62. Verfahren nach einem der Ansprüche 1 bis 49, **dadurch gekennzeichnet, dass** der Stahl B auf seiner den Reaktionsraum berührenden Seite alonisiert, alitiert und/oder aluminiert ist.

63. Eine Einhüllende E nach einem der Ansprüche 50 bis 59, wobei der Stahl B auf der den Innenraum I berührenden Seite alonisiert, alitiert und/oder aluminiert ist.

64. Verfahren nach einem der Ansprüche 1 bis 49 oder nach Anspruch 62, **dadurch gekennzeichnet, dass** der Stahl A und der Stahl B des Verbundwerkstoffs austenitische Stähle sind.

65. Eine Einhüllende E, nach einem der Ansprüche 50 bis 59 oder nach Anspruch 63, da- **dadurch gekennzeichnet, dass** der Stahl A und der Stahl B des Verbundwerkstoffs austenitische Stähle sind.

66. Eine Einhüllende E nach einem der Ansprüche 50 bis 59 oder nach einem der Ansprüche 63, 65, die auf ihrer zum Innenraum I zugewandten Seite Wärmedämmmaterial aufgebracht aufweist.

## Claims

1. A process for continuous heterogeneously catalyzed partial dehydrogenation of at least one hydrocarbon to be dehydrogenated in the gas phase, comprising a procedure in which a reaction chamber which is enclosed by a shell which is in contact with the reaction chamber and has at least one first orifice for feeding at least one starting gas stream into the reaction chamber and at least one second orifice for withdrawing at least one product gas stream from the reaction chamber,
- at least one starting gas stream comprising at least one hydrocarbon to be dehydrogenated is fed continuously,
- in the reaction chamber, the at least one hydrocarbon to be dehydrogenated is conducted through at least one catalyst bed disposed in the reaction chamber and, with generation of a product gas comprising the at least one dehydrogenated hydrocarbon, unconverted hydrocarbon to be dehydrogenated and molecular hydrogen and/or steam, is dehydrogenated partially in an oxidative or nonoxidative manner to at least one dehydrogenated hydrocarbon, and
- at least one product gas stream is withdrawn continuously from the reaction chamber,
the shell is manufactured from a composite material which, on its side B in contact with the reaction chamber, consists of steel B of the following elemental composition:
| | |
|---|---|
| from 18 to 30% by weight of | Cr, |
| from 9 to 37% by weight of | Ni, |
| from 1 to 4% by weight of | Si, |
| from ≥ 0 to 4% by weight of | Al, |
| from ≥ 0 to 0.3% by weight of | N, |
| from ≥ 0 to 0.15% by weight of | C, |
| from ≥ 0 to 4% by weight of | Mn, |
| from ≥ 0 to 0.05% by weight of | P, |
| from ≥ 0 to 0.05% by weight of | S, and |
| from ≥ 0 to 0.1% by weight of one or more rare earth metals, and | |
| apart from these, Fe and impurities resulting from production, the percentages each being based on the total weight, | |
with the proviso that the steel B, on its side A facing away from the reaction chamber, is plated either directly or via an intermediate layer of copper, or of nickel, or of copper and nickel, onto steel A of the elemental composition
| | |
|---|---|
| from 15 to 20% by weight of | Cr, |
| from 6 to 18% by weight of | Ni, |
| from ≥ 0 to 0.8% by weight of | Si, |
| from ≥ 0 to 0.8% by weight of | Al, |
| from ≥ Q to 0.3% by weight of | N, |
| from ≥ 0 to 0.15% by weight of | C, |
| from ≥ 0 to 4% by weight of | Mo, |
| from ≥ 0 to 2% by weight of | Mn, |
| from ≥ to 0.8% by weight of | Ti, |
| from ≥ 0 to 1.2% by weight of | Nb, |
| from ≥ 0 to 0.9% by weight of | V, |
| from ≥ 0 to 0.1% by weight of | B, |
| from ≥ 0 to 0.05% by weight of | P, |
| from ≥ 0 to 0.05% by weight of | S, and |
| | |
| apart from these, Fe and impurities resulting from production, the percentages each being based on the total weight, | |
or of the elemental composition
| | |
|---|---|
| from 19 to 23% by weight of | Cr, |
| from 30 to 35% by weight of | Ni, |
| from ≥ 0 to 1% by weight of | Co, |
| from ≥ 0 to 1% by weight of | Si, |
| from 0.15 to 0.7% by weight of | Al, |
| from ≥ 0 to 0.12% by weight of | C, |
| from ≥ 0 to 2.0% by weight of | Mn, |
| from ≥ 0 to 0.75% by weight of | Cu, |
| from 0.15 to 0.7% by weight of | Ti, |
| from ≥ 0 to ≤ 0.1% by weight of | Nb, |
| from ≥ 0 to 0.05% by weight of | P, |
| from ≥ 0 to 0.05% by weight of | S, and |
| | |
| apart from these, Fe and impurities resulting from production, the percentages each being based on the total weight. | |

2. The process according to claim 1, wherein the coefficients of thermal expansion of steel A and steel B of the composite material at 500°C and 1 atm differ by ≤ 2 · 10⁻⁶ m/m·K.

3. The process according to claim 1, wherein the coefficients of thermal expansion of steel A and steel B of the composite material at 500°C and 1 atm differ by ≤ 1.75 · 10⁻⁶ m/m ·K.

4. The process according to claim 1, wherein the coefficients of thermal expansion of steel A and steel B of the composite material at 500°C and 1 atm differ by ≤ 1.50 · 10⁻⁶ m/m ·K.

5. The process according to claim 1, wherein the coefficients of thermal, expansion of steel A and steel B of the composite material at 500°C and 1 atm differ by ≤ 1.25 · 10⁻⁶ m/m ·K.

6. The process according to claim 1, wherein the coefficients of thermal expansion of steel A and steel B of the composite material at 500°C and 1 atm differ by ≤ 1.0 · 10⁻⁶ m/m ·K.

7. The process according to any of claims 1 to 6, wherein the thickness of steel B of the composite material is from 0.2 to 25 mm.

8. The process according to any of claims 1 to 6, wherein the thickness of steel B of the composite material is from 1 to 10 mm.

9. The process according to any of claims 1 to 6, wherein the thickness of steel B of the composite material is from 2 to 8 mm.

10. The process according to any of claims 1 to 9, wherein the thickness of steel A of the composite material is from 10 to 150 mm.

11. The process according to any of claims 1 to 9, wherein the thickness of steel A of the composite material is from 20 to 100 mm.

12. The process according to any of claims 1 to 9, wherein the thickness of steel A of the composite material is from 60 to 100 mm.

13. The process according to any of claims 1 to 9, wherein the thickness of steel A of the composite material is from 20 to 50 mm.

14. The process according to any of claims 1 to 13, wherein steel B of the composite material is plated directly onto steel A.

15. The process according to any of claims 1 to 13, wherein steel B of the composite material is plated onto steel A via an intermediate layer of copper, or of nickel, or of copper and nickel, and the thickness of the intermediate layer is ≥ 0.1 mm and ≤ 3 mm.

16. The process according to claim 15, wherein the thickness of the intermediate layer is ≥ 0.2 mm and ≤ 2 mm.

17. The process according to claim 15, wherein the thickness of the intermediate layer is ≥ 0.3 mm and ≤ 1 mm.

18. The process according to any of claims 1 to 17, wherein the content of Si in steel A of the composite material is ≤ 0.6% by weight.

19. The process according to any of claims 1 to 17, wherein the content of Si in steel A of the composite material is ≤ 0.4% by weight.

20. The process according to any of claims 1 to 17, wherein the content of Si in steel A of the composite material is ≤ 0.1% by weight.

21. The process according to any of claims 1 to 20, wherein steel B of the composite material has the elemental composition
| | |
|---|---|
| from 24 to 26% by weight of | Cr, |
| from 19 to 22% by weight of | Ni, |
| from 1.5 to 2.5% by weight of | Si, |
| from ≥ 0 to 0.11% by weight of | N, |
| from ≥ 0 to 0.2% by weight of | C, |
| from ≥ 0 to 2% by weight of | Mn, |
| from ≥ 0 to 0.045% by weight of | P, |
| from ≥ 0 to 0.015% by weight of | S, and |
| apart from these, Fe and impurities resulting from production, the percentages each being based on the total weight. | |

22. The process according to any of claims 1 to 21, wherein steel A of the composite material has the elemental composition
| | |
|---|---|
| from 16 to 18% by weight of | Cr, |
| from 12 to 14% by weight of | Ni, |
| from ≥ 0 to 0.8% by weight of | Si, |
| from 0.10 to 0.18% by weight of | N, |
| from ≥ 0 to 0.1% by weight of | C, |
| from ≥ 2 to 3% by weight of | Mo, |
| from ≥ 0 to 2% by weight of | Mn, |
| from 0.0015 to 0.0050% by weight of | B, |
| from ≥ 0 to 0.05% by weight of | P, |
| from ≥ 0 to 0.05% by weight of | S, and |
| | |
| apart from these, Fe and impurities resulting from production, the percentages each being based on the total weight | |

23. The process according to any of claims 1 to 22, wherein the plating-on is effected by explosive plating.

24. The process according to any of claims 1 to 23, wherein the hydrocarbon to be dehydrogenated is a C₂-to C₁₆-alkane.

25. The process according to any of claims 1 to 24, wherein the hydrocarbon to be dehydrogenated is at least one hydrocarbon from the group comprising ethane, propane, n-butane, isobutane, n-pentane, isopentane, n-hexane, n-heptane, n-octane, n-nonane, n-decane, n-undecane, n-dodecane, n-tridecane, n-tetradecane, n-pentadecane and n-hexadecane.

26. The process according to any of claims 1 to 25, wherein the hydrocarbon to be dehydrogenated is ethane, propane, n-butane and/or isobutane.

27. The process according to any of claims 1 to 26, wherein the hydrocarbon to be dehydrogenated is propane and the dehydrogenated hydrocarbon is propylene.

28. The process according to any of claims 1 to 27, wherein the starting gas stream comprises steam.

29. The process according to any of claims 1 to 28, wherein the starting gas stream comprises molecular oxygen.

30. The process according to any of claims 1 to 29, wherein the catalyst bed is a fixed catalyst bed.

31. The process according to any of claims 1 to 30, wherein the heterogeneously catalyzed partial dehydrogenation is a nonoxidative dehydrogenation.

32. The process according to any of claims 1 to 30, wherein the heterogeneously catalyzed partial dehydrogenation is an oxidative dehydrogenation.

33. The process according to any of claims 1 to 30, wherein the heterogeneously catalyzed partial dehydrogenation is a heterogeneously catalyzed oxydehydrogenation.

34. The process according to any of claims 1 to 30, wherein the heterogeneously catalyzed partial dehydrogenation is an adiabatic conventional heterogeneously catalyzed dehydrogenation.

35. The process according to any of claims 1 to 30, wherein the heterogeneously catalyzed partial dehydrogenation is a conventional heterogeneously catalyzed partial dehydrogenation and the reaction chamber is a tray reaction chamber.

36. The process according to claim 35, wherein the conventional heterogeneously catalyzed partial dehydrogenation is an oxidative conventional heterogeneously catalyzed partial dehydrogenation.

37. The process according to claim 36, which is performed in an adiabatic reaction chamber.

38. The process according to any of claims 1 to 37, wherein the starting gas stream fed to the reaction chamber comprises:
| |
|---|
| from ≥ 0 to 20% by volume of propylene, |
| from ≥ 0 to 1% by volume of acrolein, |
| from ≥ 0 to 0.25% by volume of acrylic acid, |
| from ≥ 0 to 20% by volume of COx, |
| from 5 to 50% by volume of propane, |
| from 20 to 80% by volume of nitrogen, |
| from ≥ 0 to 5% by volume of oxygen, |
| from ≥ 0 to 20% by volume of H2O and |
| from ≥ 0 to 10% by volume of H2. |

39. The process according to any of claims 1 to 38, wherein the product gas stream withdrawn from the reaction chamber is used as such or after removal of at least a portion of its constituents other than the dehydrogenated hydrocarbon and the hydrocarbon to be dehydrogenated to charge at least one oxidation reactor, and the dehydrogenated hydrocarbon present therein is subjected in this oxidation reactor to a selective heterogeneously catalyzed partial gas phase oxidation with molecular oxygen to give a product gas mixture B comprising the partial oxidation product.

40. The process according to claim 39, wherein the hydrocarbon to be dehydrogenated is propane, the dehydrogenated hydrocarbon is propylene and the partial oxidation product is acrolein, acrylic acid or a mixture thereof.

41. The process according to claim 39, wherein, in a separation zone B of the selective heterogeneously catalyzed partial gas phase oxidation, partial oxidation product is subsequently removed from the product gas mixture B and, from the remaining residual gas comprising unconverted hydrocarbon to be dehydrogenated, molecular oxygen and any unconverted dehydrogenated hydrocarbon, at least a portion comprising unconverted hydrocarbon to be dehydrogenated is recycled as partial oxidation cycle gas into the process for heterogeneously catalyzed partial dehydrogenation of the hydrocarbon to be dehydrogenated.

42. The process according to claim 41, wherein the partial oxidation product, in separation zone B, is removed from product gas mixture B by conversion to the condensed phase.

43. The process according to claim 42, wherein the partial oxidation product is acrylic acid and the conversion to the condensed phase is effected by absorptive and/or condensative measures.

44. The process according to claim 43, wherein a removal of acrylic acid from the condensed phase is undertaken using at least one thermal separation process.

45. The process according to claim 44, wherein the at least one thermal separation process comprises a crystallizative removal of acrylic acid from the liquid phase.

46. The process according to claim 45, wherein the crystallizative removal is a suspension crystallization.

47. The process according to claim 44, wherein the removal of acrylic acid is followed by a process for free-radical polymerization in which acrylic acid removed is free-radically polymerized to prepare polymers.

48. The process according to claim 44, wherein the removal of acrylic acid is followed by a process for preparing acrylic esters in which acrylic acid removed is esterified with an alcohol.

49. The process according to claim 48, wherein the process for preparing an acrylic ester is followed by a process for free-radical polymerization in which acrylic ester thus prepared is polymerized.

50. A shell E which encloses an interior I and has at least one first orifice 01 for feeding at least one gas stream S into the interior I and at least one second orifice 02 for withdrawing a gas stream S fed to the interior I beforehand via the at least one first orifice 01 from the interior I, the shell E being manufactured from a composite material which, on its side B in contact with the reaction chamber, consists of steel B of the following elemental composition:
| | |
|---|---|
| from 18 to 30% by weight of | Cr, |
| from 9 to 37% by weight of | Ni, |
| from 1 to 4% by weight of | Si, |
| from ≥ 0 to 4% by weight of | Al, |
| from ≥ 0 to 0.3% by weight of | N, |
| from ≥ 0 to 0.15% by weight of | C, |
| from ≥ 0 to 4% by weight of | Mn, |
| from ≥ 0 to 0.05% by weight of | P, |
| from ≥ 0 to 0.05% by weight of | S, and |
| from ≥ 0 to 0.1% by weight of one or more rare earth metals, and | |
| apart from these, Fe and impurities resulting from production, the percentages each being based on the total weight, | |
with the proviso that the steel B, on its side A facing away from the reaction chamber, is plated either directly or via an intermediate layer of copper, or of nickel, or of copper and nickel, onto steel A of the elemental composition
| | |
|---|---|
| from 15 to 20% by weight of | Cr, |
| from 6 to 18% by weight of | Ni, |
| from ≥ 0 to 0.8% by weight of | Si, |
| from ≥ 0 to 0.8% by weight of | Al, |
| from ≥ 0 to 0.3% by weight of | N, |
| from ≥ 0 to 0.15% by weight of | C, |
| from ≥ 0 to 4% by weight of | Mo, |
| from ≥ 0 to 2% by weight of | Mn, |
| from ≥ 0 to 0.8% by weight of | Ti, |
| from ≥ 0 to 1.2% by weight of | Nb, |
| from ≥ 0 to 0.9% by weight of | V, |
| from ≥ 0 to 0.1% by weight of | B, |
| from ≥ 0 to 0.05% by weight of | P, |
| from ≥ 0 to 0.05% by weight of | S, and |
| | |
| apart from these, Fe and impurities resulting from production, the percentages each being based on the total weight, | |
or of the elemental composition
| | |
|---|---|
| from 19 to 23% by weight of | Cr, |
| from 30 to 35% by weight of | Ni, |
| from ≥ 0 to 1% by weight of | Co, |
| from ≥ 0 to 1% by weight of | Si, |
| from 0.15 to 0.7% by weight of | Al, |
| from ≥ 0 to 0.12% by weight of | C, |
| from ≥ 0 to 2.0% by weight of | Mn, |
| from ≥ 0 to 0.75% by weight of | Cu, |
| from 0.15 to 0.7% by weight of | Ti, |
| from ≥ 0 to 0.05% by weight of | P, |
| from ≥ 0 to 0.05% by weight of | S, and |
apart from these, Fe and impurities resulting from production, the percentages each being based on the total weight.

51. A shell E according to claim 50 whose interior I comprises at least one dehydrogenation catalyst.

52. A shell E according to claim 50 or 51 whose interior I comprises at least one support grid.

53. A shell E according to any of claims 50 to 52 which has an annular segment R.

54. A shell E according to claim 53 where the ratio of V₁=D : A, formed from half D of the difference between the external diameter A and the internal diameter of the annular segment R, is from 1 : 10 to 1 : 1000.

55. A shell E according to claim 54 where V₁ is from 1 : 40 to 1 : 500.

56. A shell E according to any of claims 53 to 55 where the ratio V₂=H : A, formed from the separation H of the two parallel circular planes delimiting the annular segment R and the external diameter A of the annular segment is > 1.

57. A shell E according to any of claims 53 to 55 where the ratio V₂=H : A, formed from the separation H of the two parallel circular planes delimiting the annular segment R and the external diameter of the annular segment is ≤ 1.

58. A shell E according to any of claims 50 to 52 which has a hollow spherical zone segment K.

59. A shell E according to any of claims 50 to 58 which has thermal insulation material on its side facing away from the interior I.

60. A process for heterogeneously catalyzed partial dehydrogenation of a hydrocarbon, which is performed in the interior I of a shell E according to any of claims 50 to 59.

61. The use of a shell E according to any of claims 50 to 59 or according to any of claims 63, 65, 66 for carrying out a heterogeneously catalyzed partial dehydrogenation of a hydrocarbon.

62. The process according to any of claims 1 to 49, wherein the steel B has been alonized, alitized and/or aluminized on its side in contact with the reaction chamber.

63. A shell E according to any of claims 50 to 59 where the steel B has been alonized, alitized and/or aluminized on the side in contact with the interior I.

64. The process according to any of claims 1 to 49 or according to claim 62, wherein steel A and steel B of the composite material are austenitic steels.

65. A shell E according to any of claims 50 to 59 or according to claim 63, wherein steel A and steel B of the composite material are austenitic steels.

66. A shell E according to any of claims 50 to 59 or according to either of claims 63, 65 which has thermal insulation material mounted on its side facing toward the interior I.

## Revendications

1. Procédé de déshydrogénation partielle continue sous catalyse hétérogène d'au moins un hydrocarbure à déshydrogéner en phase gazeuse, comprenant un procédé selon lequel une chambre de réaction, qui est entourée par une enveloppe en contact avec la chambre de réaction, qui comprend au moins une première ouverture pour l'introduction d'au moins un courant gazeux initial dans la chambre de réaction et au moins une seconde ouverture pour le déchargement d'au moins un courant gazeux de produits de la chambre de réaction,
- est alimentée en continu avec au moins un courant gazeux initial contenant au moins un hydrocarbure à déshydrogéner,
- dans la chambre de réaction, le ou les hydrocarbures à déshydrogéner traversent au moins un lit catalytique se trouvant dans la chambre de réaction, et sont partiellement déshydrogénés oxydativement ou non oxydativement en au moins un hydrocarbure déshydrogéné en formant un gaz produit qui contient le ou les hydrocarbures déshydrogénés, le ou les hydrocarbures à déshydrogéner non réagis et de l'hydrogène moléculaire et/ou de la vapeur d'eau, et
- au moins un courant gazeux de produits est déchargé en continu de la chambre de réaction,
**caractérisé en ce que**
l'enveloppe est fabriquée en un matériau composite qui est constitué sur son côté B en contact avec la chambre de réaction d'un acier B de la composition élémentaire suivante
| | |
|---|---|
| 18 à 30 % en poids | Cr, |
| 9 à 37 % en poids | Ni, |
| 1 à 4 % en poids | Si, |
| ≥ 0 à 4 % en poids | Al, |
| ≥ 0 à 0,3 % en poids | N, |
| ≥ 0 à 0,15 % en poids | C, |
| ≥ 0 à 4 % en poids | Mn, |
| ≥ 0 à 0,05 % en poids | P, |
| ≥ 0 à 0,05 % en poids | S et |
| ≥ 0 à 0,1 % en poids | un ou plusieurs métaux de terres rares, |
| le reste étant du Fe et des impuretés liées à la fabrication, les données de pourcentage étant à chaque fois relatives au poids total, | |
à condition que l'acier B soit plaqué sur son côté A détourné de la chambre de réaction soit directement, soit par le biais d'une couche intermédiaire en cuivre ou en nickel ou en cuivre et nickel, sur un acier A de composition élémentaire
| | |
|---|---|
| 15 à 20 % en poids | Cr, |
| 6 à 18 % en poids | Ni, |
| ≥ 0 à 0,8 % en poids | Si, |
| ≥ 0 à 0,8 % en poids | Al, |
| ≥ 0 à 0,3 % en poids | N, |
| ≥ 0 à 0,15 % en poids | C, |
| ≥ 0 à 4 % en poids | Mo, |
| ≥ 0 à 2 % en poids | Mn, |
| ≥ 0 à 0,8 % en poids | Ti, |
| ≥ 0 à 1,2 % en poids | Nb, |
| ≥ 0 à 0,9 % en poids | V, |
| ≥ 0 à 0,1 % en poids | B, |
| ≥ 0 à 0,05 % en poids | P, |
| ≥ 0 à 0,05 % en poids | S, |
| le reste étant du Fe et des impuretés liées à la fabrication, les données de pourcentage étant à chaque fois relatives au poids total, | |
ou de composition élémentaire
| | |
|---|---|
| 19 à 23 % en poids | Cr, |
| 30 à 35 % en poids | Ni, |
| ≥ 0 à 1 % en poids | Co, |
| ≥ 0 à 1 % en poids | Si, |
| 0,15 à 0,7 % en poids | Al, |
| ≥ 0 à 0,12 % en poids | C, |
| ≥ 0 à 2,0 % en poids | Mn, |
| ≥ 0 à 0,75 % en poids | Cu, |
| 0,15 à 0,7 % en poids | Ti, |
| ≥ 0 à ≤ 0,1 % en poids | Nb, |
| ≥ 0 à 0,05 % en poids | P, |
| ≥ 0 à 0,05 % en poids | S, |
| le reste étant du Fe et des impuretés liées à la fabrication, les données de pourcentage étant à chaque fois relatives au poids total. | |

2. Procédé selon la revendication 1, **caractérisé en ce que** les coefficients d'expansion thermique de l'acier A et de l'acier B du matériau composite diffèrent de ≤ 2 · 10⁻⁶ m/m·K à 500 °C et 1 atm.

3. Procédé selon la revendication 1, **caractérisé en ce que** les coefficients d'expansion thermique de l'acier A et de l'acier B du matériau composite diffèrent de ≤ 1,75 · 10⁻⁶ m/m·K à 500 °C et 1 atm.

4. Procédé selon la revendication 1, **caractérisé en ce que** les coefficients d'expansion thermique de l'acier A et de l'acier B du matériau composite diffèrent de ≤ 1,50 · 10⁻⁶ m/m·K à 500 °C et 1 atm.

5. Procédé selon la revendication 1, **caractérisé en ce que** les coefficients d'expansion thermique de l'acier A et de l'acier B du matériau composite diffèrent de ≤ 1,25 · 10⁻⁶ m/m·K à 500 °C et 1 atm.

6. Procédé selon la revendication 1, **caractérisé en ce que** les coefficients d'expansion thermique de l'acier A et de l'acier B du matériau composite diffèrent de ≤ 1,0 · 10⁻⁶ m/m·K à 500 °C et 1 atm.

7. Procédé selon l'une quelconque des revendications 1 à 6, **caractérisé en ce que** l'épaisseur de l'acier B du matériau composite est de 0,2 à 25 mm.

8. Procédé selon l'une quelconque des revendications 1 à 6, **caractérisé en ce que** l'épaisseur de l'acier B du matériau composite est de 1 à 10 mm.

9. Procédé selon l'une quelconque des revendications 1 à 6, **caractérisé en ce que** l'épaisseur de l'acier B du matériau composite est de 2 à 8 mm.

10. Procédé selon l'une quelconque des revendications 1 à 9, **caractérisé en ce que** l'épaisseur de l'acier A du matériau composite est de 10 à 150 mm.

11. Procédé selon l'une quelconque des revendications 1 à 9, **caractérisé en ce que** l'épaisseur de l'acier A du matériau composite est de 20 à 100 mm.

12. Procédé selon l'une quelconque des revendications 1 à 9, **caractérisé en ce que** l'épaisseur de l'acier A du matériau composite est de 60 à 100 mm.

13. Procédé selon l'une quelconque des revendications 1 à 9, **caractérisé en ce que** l'épaisseur de l'acier A du matériau composite est de 20 à 50 mm.

14. Procédé selon l'une quelconque des revendications 1 à 13, **caractérisé en ce que** l'acier B du matériau composite est plaqué directement sur l'acier A.

15. Procédé selon l'une quelconque des revendications 1 à 13, **caractérisé en ce que** l'acier B du matériau composite est plaqué sur l'acier A par le biais d'une couche intermédiaire en cuivre ou en nickel ou en cuivre et en nickel, et l'épaisseur de la couche intermédiaire est ≥ 0,1 mm et ≤ 3 mm.

16. Procédé selon la revendication 15, **caractérisé en ce que** l'épaisseur de la couche intermédiaire est ≥ 0,2 mm et ≤ 2 mm.

17. Procédé selon la revendication 15, **caractérisé en ce que** l'épaisseur de la couche intermédiaire est ≥ 0,3 mm et ≤ 1 mm.

18. Procédé selon l'une quelconque des revendications 1 à 17, **caractérisé en ce que** la teneur de l'acier A du matériau composite en Si est ≤ 0,6 % en poids.

19. Procédé selon l'une quelconque des revendications 1 à 17, **caractérisé en ce que** la teneur de l'acier A du matériau composite en Si est ≤ 0,4 % en poids.

20. Procédé selon l'une quelconque des revendications 1 à 17, **caractérisé en ce que** la teneur de l'acier A du matériau composite en Si est ≤ 0,1 % en poids.

21. Procédé selon l'une quelconque des revendications 1 à 20, **caractérisé en ce que** l'acier B du matériau composite présente la composition élémentaire
| | |
|---|---|
| 24 à 26 % en poids | Cr, |
| 19 à 22 % en poids | Ni, |
| 1,5 à 2,5 % en poids | Si, |
| ≥ 0 à 0,11 % en poids | N, |
| ≥ 0 à 0,2 % en poids | C, |
| ≥ 0 à 2 % en poids | Mn, |
| ≥ 0 à 0,045 % en poids | P, |
| ≥ 0 à 0,015 % en poids | S, |
| le reste étant du Fe et des impuretés liées à la fabrication, les données de pourcentage étant à chaque fois relatives au poids total. | |

22. Procédé selon l'une quelconque des revendications 1 à 21, **caractérisé en ce que** l'acier A du matériau composite présente la composition élémentaire
| | |
|---|---|
| 16 à 18 % en poids | Cr, |
| 12 à 14 % en poids | Ni, |
| ≥ 0 à 0,8 % en poids | Si, |
| 0,10 à 0,18 % en poids | N, |
| ≥ 0 à 0,1 % en poids | C, |
| ≥ 2 à 3 % en poids | Mo, |
| ≥ 0 à 2 % en poids | Mn, |
| 0,0015 à 0,0050 % en poids | B, |
| ≥ 0 à 0,05 % en poids | P, |
| ≥ 0 à 0,05 % en poids | S, |
| le reste étant du Fe et des impuretés liés à la fabrication, les données de pourcentage étant à chaque fois relatives au poids total. | |

23. Procédé selon l'une quelconque des revendications 1 à 22, **caractérisé en ce que** le plaquage a lieu par plaquage par explosion.

24. Procédé selon l'une quelconque des revendications 1 à 23, **caractérisé en ce que** l'hydrocarbure à déshydrogéner est un alcane en C₂ à C₁₆.

25. Procédé selon l'une quelconque des revendications 1 à 24, **caractérisé en ce que** l'hydrocarbure à déshydrogéner est au moins un hydrocarbure du groupe comprenant l'éthane, le propane, le n-butane, l'iso-butane, le n-pentane, l'iso-pentane, le n-hexane, le n-heptane, le n-octane, le n-nonane, le n-décane, le n-undécane, le n-dodécane, le n-tridécane, le n-tétradécane, le n-pentadécane et le n-hexadécane.

26. Procédé selon l'une quelconque des revendications 1 à 25, **caractérisé en ce que** l'hydrocarbure à déshydrogéner est l'éthane, le propane, le n-butane et/ou l'iso-butane.

27. Procédé selon l'une quelconque des revendications 1 à 26, **caractérisé en ce que** l'hydrocarbure à déshydrogéner est le propane et l'hydrocarbure déshydrogéné est le propylène.

28. Procédé selon l'une quelconque des revendications 1 à 27, **caractérisé en ce que** le courant gazeux initial contient de la vapeur d'eau.

29. Procédé selon l'une quelconque des revendications 1 à 28, **caractérisé en ce que** le courant gazeux initial contient de l'oxygène moléculaire.

30. Procédé selon l'une quelconque des revendications 1 à 29, **caractérisé en ce que** le lit catalytique est un lit catalytique fixe.

31. Procédé selon l'une quelconque des revendications 1 à 30, **caractérisé en ce que** la déshydrogénation partielle sous catalyse hétérogène est une déshydrogénation non oxydative.

32. Procédé selon l'une quelconque des revendications 1 à 30, **caractérisé en ce que** la déshydrogénation partielle sous catalyse hétérogène est une déshydrogénation oxydative.

33. Procédé selon l'une quelconque des revendications 1 à 30, **caractérisé en ce que** la déshydrogénation partielle sous catalyse hétérogène est une oxydéshydrogénation sous catalyse hétérogène.

34. Procédé selon l'une quelconque des revendications 1 à 30, **caractérisé en ce que** la déshydrogénation partielle sous catalyse hétérogène est une déshydrogénation sous catalyse hétérogène classique réalisée de manière adiabatique.

35. Procédé selon l'une quelconque des revendications 1 à 30, **caractérisé en ce que** la déshydrogénation partielle sous catalyse hétérogène est une déshydrogénation partielle sous catalyse hétérogène classique et la chambre de réaction est une chambre de réaction à plateaux.

36. Procédé selon la revendication 35, **caractérisé en ce que** la déshydrogénation partielle sous catalyse hétérogène classique est une déshydrogénation partielle sous catalyse hétérogène classique oxydative.

37. Procédé selon la revendication 36, **caractérisé en ce qu'**il est réalisé dans une chambre de réaction configurée de manière adiabatique.

38. Procédé selon l'une quelconque des revendications 1 à 37, **caractérisé en ce que** le courant gazeux initial introduit dans la chambre de réaction contient :
| | |
|---|---|
| propylène | ≥ 0 à 20 % en volume, |
| acroléine | ≥ 0 à 1 % en volume, |
| acide acrylique | ≥ 0 à 0,25 % en volume, |
| COₓ | ≥ 0 à 20 % en volume, |
| propane | 5 à 50 % en volume, |
| azote | 20 à 80 % en volume, |
| oxygène | ≥ 0 à 5 % en volume, |
| H₂O | ≥ 0 à 20 % en volume, et |
| H₂ | ≥ 0 à 10 % en volume. |

39. Procédé selon l'une quelconque des revendications 1 à 38, **caractérisé en ce que** le courant gazeux de produits déchargé de la chambre de réaction est utilisé en tant que tel ou après séparation d'au moins une partie de ses constituants différents de l'hydrocarbure déshydrogéné et de l'hydrocarbure à déshydrogéner pour l'alimentation d'au moins un réacteur d'oxydation et, dans celui-ci, l'hydrocarbure déshydrogéné qu'il contient est soumis à une oxydation partielle sélective en phase gazeuse sous catalyse hétérogène avec de l'oxygène moléculaire pour former un mélange gazeux de produits B contenant le produit de l'oxydation partielle.

40. Procédé selon la revendication 39, **caractérisé en ce que** l'hydrocarbure à déshydrogéner est le propane, l'hydrocarbure déshydrogéné est le propylène et le produit de l'oxydation partielle est l'acroléine, l'acide acrylique ou leur mélange.

41. Procédé selon la revendication 39, **caractérisé en ce que** le produit de l'oxydation partielle est séparé du mélange gazeux de produits B dans une zone de séparation B suivant l'oxydation partielle sélective en phase gazeuse sous catalyse hétérogène et, à partir du gaz résiduel restant contenant de l'hydrocarbure à déshydrogéner non réagi, de l'oxygène moléculaire et éventuellement de l'hydrocarbure déshydrogéné non réagi, au moins une partie contenant de l'hydrocarbure à déshydrogéner non réagi est recyclée en tant que gaz circulaire de l'oxydation partielle dans le procédé de déshydrogénation partielle sous catalyse hétérogène de l'hydrocarbure à déshydrogéner.

42. Procédé selon la revendication 41, **caractérisé en ce que** le produit de l'oxydation partielle est séparé du mélange gazeux de produits B dans la zone de séparation B par transfert dans la phase condensée.

43. Procédé selon la revendication 42, **caractérisé en ce que** le produit de l'oxydation partielle est l'acide acrylique et le transfert dans la phase condensée a lieu par des mesures d'absorption et/ou de condensation.

44. Procédé selon la revendication 43, **caractérisé en ce qu'**une séparation de l'acide acrylique de la phase condensée est réalisée en utilisant au moins un procédé de séparation thermique.

45. Procédé selon la revendication 44, **caractérisé en ce que** le ou les procédés de séparation thermiques comprennent une séparation par cristallisation de l'acide acrylique de la phase liquide.

46. Procédé selon la revendication 45, **caractérisé en ce que** la séparation par cristallisation est une cristallisation en suspension.

47. Procédé selon la revendication 44, **caractérisé en ce qu'**un procédé de polymérisation radicalaire suit la séparation de l'acide acrylique, lors duquel l'acide acrylique séparé est polymérisé par voie radicalaire pour la fabrication de polymères.

48. Procédé selon la revendication 44, **caractérisé en ce qu'**un procédé de fabrication d'esters de l'acide acrylique suit la séparation de l'acide acrylique, lors duquel l'acide acrylique séparé est estérifié avec un alcool.

49. Procédé selon la revendication 48, **caractérisé en ce qu'**un procédé de polymérisation radicalaire suit le procédé de fabrication d'un ester de l'acide acrylique, lors duquel l'ester de l'acide acrylique ainsi fabriqué est polymérisé.

50. Enveloppe E entourant une chambre intérieure I, comprenant au moins une première ouverture O1 pour l'introduction d'au moins un courant gazeux S dans la chambre intérieure I et au moins une seconde ouverture 02 pour le déchargement d'un courant gazeux S auparavant introduit dans la chambre intérieure I par la ou les premières ouvertures O1 de la chambre intérieure I, l'enveloppe E étant fabriquée en un matériau composite qui est constitué sur son côté B en contact avec la chambre de réaction d'un acier B de la composition élémentaire suivante
| | |
|---|---|
| 18 à 30 % en poids | Cr, |
| 9 à 37 % en poids | Ni, |
| 1 à 4 % en poids | Si, |
| ≥ 0 à 4 % en poids | Al, |
| ≥ 0 à 0,3 % en poids | N, |
| ≥ 0 à 0,15 % en poids | C, |
| ≥ 0 à 4 % en poids | Mn, |
| ≥ 0 à 0,05 % en poids | P, |
| ≥ 0 à 0,05 % en poids | S et |
| ≥ 0 à 0,1 % en poids | un ou plusieurs métaux de terres rares, |
| le reste étant du Fe et des impuretés liées à la fabrication, les données de pourcentage étant à chaque fois relatives au poids total, | |
à condition que l'acier B soit plaqué sur son côté A détourné de la chambre de réaction soit directement, soit par le biais d'une couche intermédiaire en cuivre ou en nickel ou en cuivre et nickel, sur un acier A de composition élémentaire
| | |
|---|---|
| 15 à 20 % en poids | Cr, |
| 6 à 18 % en poids | Ni, |
| ≥ 0 à 0,8 % en poids | Si, |
| ≥ 0 à 0,8 % en poids | Al, |
| ≥ 0 à 0,3 % en poids | N, |
| ≥ 0 à 0,15 % en poids | C, |
| ≥ 0 à 4 % en poids | Mo, |
| ≥ 0 à 2 % en poids | Mn, |
| ≥ 0 à 0,8 % en poids | Ti, |
| ≥ 0 à 1,2 % en poids | Nb, |
| ≥ 0 à 0,9 % en poids | V, |
| ≥ 0 à 0,1 % en poids | B, |
| ≥ 0 à 0,05 % en poids | P, |
| ≥ 0 à 0,05 % en poids | S, |
| le reste étant du Fe et des impuretés liées à la fabrication, les données de pourcentage étant à chaque fois relatives au poids total, | |
ou de composition élémentaire
| | |
|---|---|
| 19 à 23 % en poids | Cr, |
| 30 à 35 % en poids | Ni, |
| ≥ 0 à 1 % en poids | Co, |
| ≥ 0 à 1 % en poids | Si, |
| 0,15 à 0,7 % en poids | Al, |
| ≥ 0 à 0,12 % en poids | C, |
| ≥ 0 à 2,0 % en poids | Mn, |
| ≥ 0 à 0,75 % en poids | Cu, |
| 0,15 à 0,7 % en poids | Ti, |
| ≥ 0 à 0,05 % en poids | P, |
| ≥ 0 à 0,05 % en poids | S, |
| le reste étant du Fe et des impuretés liées à la fabrication, les données de pourcentage étant à chaque fois relatives au poids total. | |

51. Enveloppe E selon la revendication 50, dont la chambre intérieure I contient au moins un catalyseur de déshydrogénation.

52. Enveloppe E selon la revendication 50 ou 51, dont la chambre intérieure I contient au moins une grille support.

53. Enveloppe E selon l'une quelconque des revendications 50 à 52, qui comprend un segment annulaire R.

54. Enveloppe E selon la revendication 53, dans laquelle le rapport V₁ = D:A, formé par la moitié D de la différence entre le diamètre extérieur A et le diamètre intérieur du segment annulaire R, est de 1:10 à 1:1 000.

55. Enveloppe E selon la revendication 54, dans laquelle V₁ est de 1:40 à 1:500.

56. Enveloppe E selon l'une quelconque des revendications 53 à 55, dans laquelle le rapport V₂ = H:A, formé par la distance H entre les deux plans circulaires parallèles délimitant le segment annulaire R et le diamètre extérieur A du segment annulaire, est > 1.

57. Enveloppe E selon l'une quelconque des revendications 53 à 55, dans laquelle le rapport V₂ = H:A, formé par la distance H entre les deux plans circulaires parallèles délimitant le segment annulaire R et le diamètre extérieur du segment annulaire, est ≤ 1.

58. Enveloppe E selon l'une quelconque des revendications 50 à 52, qui comprend un segment K en forme d'une zone sphérique creuse.

59. Enveloppe E selon l'une quelconque des revendications 50 à 58, qui comprend appliqué sur son côté détourné de la chambre intérieure I un matériau isolant thermique.

60. Procédé de déshydrogénation partielle sous catalyse hétérogène d'un hydrocarbure, **caractérisé en ce qu'**il est réalisé dans la chambre intérieure I d'une enveloppe E selon l'une quelconque des revendications 50 à 59.

61. Utilisation d'une enveloppe E selon l'une quelconque des revendications 50 à 59 ou selon l'une quelconque des revendications 63, 65, 66 pour la réalisation d'une déshydrogénation partielle sous catalyse hétérogène d'un hydrocarbure.

62. Procédé selon l'une quelconque des revendications 1 à 49, **caractérisé en ce que** l'acier B est alonisé, alité et/ou aluminé sur son côté en contact avec la chambre de réaction.

63. Enveloppe E selon l'une quelconque des revendications 50 à 59, dans laquelle l'acier B est alonisé, alité et/ou aluminé sur le côté en contact avec la chambre intérieure I.

64. Procédé selon l'une quelconque des revendications 1 à 49 ou selon la revendication 62, **caractérisé en ce que** l'acier A et l'acier B du matériau composite sont des aciers austénitiques.

65. Enveloppe E selon l'une quelconque des revendications 50 à 59 ou selon la revendication 63, **caractérisée en ce que** l'acier A et l'acier B du matériau composite sont des aciers austénitiques.

66. Enveloppe E selon l'une quelconque des revendications 50 à 59 ou selon l'une quelconque des revendications 63, 65, qui comprend appliqué sur son côté détourné de la chambre intérieure I un matériau isolant thermique.
